(19) Europäisches Patentamt
European Patent Office
Office européen des brevets

(11) Veröffentlichungsnummer: **0 352 613 B1**

# (12) EUROPÄISCHE PATENTSCHRIFT

(45) Veröffentlichungstag der Patentschrift: **20.04.94**

(21) Anmeldenummer: **89113220.1**

(22) Anmeldetag: **19.07.89**

Die Akte enthält technische Angaben, die nach dem Eingang der Anmeldung eingereicht wurden und die nicht in dieser Patentschrift enthalten sind.

(51) Int. Cl.5: **C07D 417/12**, C07D 405/12, C07D 417/14, C07D 311/58, C07D 311/64, C07D 213/75, C07C 311/18, C07C 217/58, C07C 255/24, C07C 271/20, C07C 233/78

## (54) Substituierte Aminomethyltetraline sowie ihre heterocyclischen Analoga.

(30) Priorität: **28.07.88 DE 3825609**
**23.01.89 DE 3901814**

(43) Veröffentlichungstag der Anmeldung:
**31.01.90 Patentblatt 90/05**

(45) Bekanntmachung des Hinweises auf die Patenterteilung:
**20.04.94 Patentblatt 94/16**

(84) Benannte Vertragsstaaten:
**AT BE CH DE ES FR GB GR IT LI LU NL SE**

(56) Entgegenhaltungen:
**EP-A- 0 157 267**
**EP-A- 0 236 930**
**EP-A- 0 252 005**
**EP-A- 0 270 947**

(73) Patentinhaber: **BAYER AG**

**D-51368 Leverkusen(DE)**

(72) Erfinder: **Junge, Bodo, Dr.**
**Spiekern 23**
**D-5600 Wuppertal 23(DE)**
Erfinder: **Schohe, Rudolf, Dr.**
**Pahlkestrasse 5**
**D-5600 Wuppertal 1(DE)**
Erfinder: **Seidel, Peter-Rudolf, Dr.**
**Alte Heide 5d**
**D-5000 Köln(DE)**
Erfinder: **Glaser, Thomas, Dr.**
**Koesliner Strasse 21a**
**D-5064 Roesrath(DE)**
Erfinder: **Traber, Jörg, Dr.**
**Loewenburgstrasse 12**
**D-5204 Lohmar 21(DE)**
Erfinder: **Benz, Ulrich, Dr.**
**Fauthstrasse 73**
**D-5060 Bergisch-Gladbach 2(DE)**
Erfinder: **Schuurman, Teunis, Dr.**
**Rohrbergstrasse 20**
**D-5204 Lohmar 21(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **de Vry, Jean-Marie-Viktor, Dr.**
**Adelenhof 36**
**D-5064 Roesrath(DE)**

## Beschreibung

Die Erfindung betrifft substituierte Aminomethyltetraline sowie ihre heterocyclische Analoga, Verfahren zu ihrer Herstellung und ihre Verwendung in Arzneimitteln.

Es ist bekannt, daß 2-N-Dipropyl-amino-8-hydroxy-tetralin mit hoher Affinität und Selektivität an zentrale Rezeptoren des $5\text{-}HT_1$-Typs bindet. Ferner ist bekannt, daß substituierte Aminomethylbenzdioxane hohe Affinität zu Rezeptoren vom $5\text{-}HT_1$-Typ besitzen und sowohl Wirkung auf das kardiovaskuläre als auch auf das zentralnervöse System zeigen (EP-A 0 236 930).

Außerdem ist bekannt, daß 3-Aminochromane und 3-Aminomethylchromane eine stimulierende Wirkung auf das zentrale Nervensystem haben (Ind. J. Chem., Sect. B. 21B (4), 344-347).

Es wurden substituierte Aminomethyltetraline und ihre heterocyclischen Analoga der allgemeinen Formel

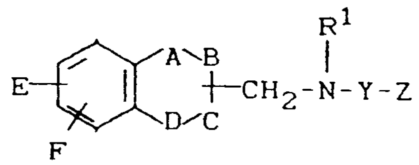

(I),

in welcher

Y
- eine geradkettige Alkylenkette mit 2-5 Kohlenstoffatomen bedeutet,

Z
- eine Gruppe der Formel

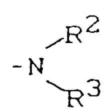

bedeutet,
wobei

$R^2$ und $R^3$ gleich oder verschieden sind und
- für Wasserstoff, Methyl, Ethyl, Propyl, oder
- für Phenyl, Benzyl oder Pyridyl stehen, das gegebenenfalls substituiert ist durch Fluor, Chlor, Methyl oder Methoxy
- für eine Gruppe der Formel

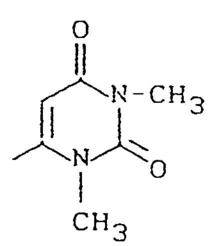

stehen oder
- für eine Gruppe der Formel $-COR^9$ oder $-SO_2R^{10}$ stehen,

worin

$R^9$
- für Methyl, Ethyl oder Ethoxy steht oder
- für Phenyl oder Benzyl steht, gegebenenfalls substituiert durch Methyl, Methoxy, Fluor oder Chlor

$R^{10}$

- für Methyl, Ethyl oder Propyl steht,
- für Phenyl, Naphthyl oder Benzyl steht, das gegebenenfalls durch Fluor oder Chlor substitutiert ist oder
- für eine Gruppe der Formel

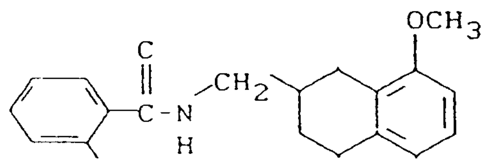

steht

$R^2$ und $R^3$ gemeinsam mit dem Stickstoffatom einen Heterocyclus aus der Reihe

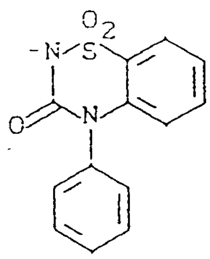

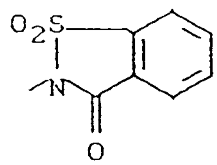

,

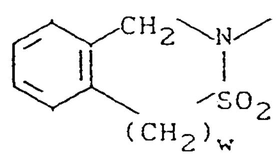

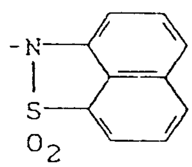

,

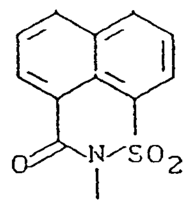

,

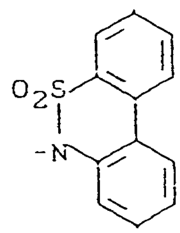

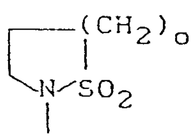

oder

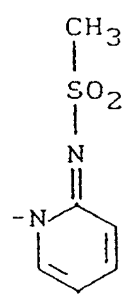

bilden, wobei

w
- eine Zahl 0, 1 oder 2 bedeutet und

o
- eine Zahl 1 oder 2 bedeutet

$R^1$
- Wasserstoff, Ethyl, Methyl, Propyl oder Benzyl bedeutet, oder
- die Gruppe -($Y^1$-$Z^1$) bedeutet, wobei $Y^1$ und $Z^1$ gleich oder verschieden zu Y und Z sein können und die oben angegebene Bedeutung von Y und Z haben,

A und D für eine Gruppe der Formel $-CH_2-$, für Sauerstoff: oder für den CH- oder N-Teil einer C=C-C=N-Doppelbindung stehen, mit der Maßgabe, daß entweder nur A oder nur D für Sauerstoff stehen darf,

B

- für eine Gruppe der Formel
$>CH_2$ oder $>CH$ oder für den CH-Teil einer C = C- oder C = N-Doppelbindung steht,

C

- für eine Gruppe der Formel $>CH$ oder den C-Teil einer C = C oder C = N Doppelbindung steht,

E und F gleich oder verschieden sind und für Wasserstoff, Ethoxy, Methoxy, Fluor, Cyano oder eine Gruppe der Formel $-CONR^2R^3$ stehen, worin $R^2$ und $R^3$ Wasserstoff bedeuten,
E und F gemeinsam einen Phenyl oder Cyclohexanring bilden,
und deren Salze gefunden.

Im Rahmen der vorliegenden Erfindung können die erfindungsgemäßen Verbindungen mehrere asymmetrische Kohlenstoffatome haben und daher in verschiedenen stereochemischen Formen vorliegen. Darüber hinaus können Verbindungen mit einer Sulfoxidgruppe ebenfalls in unterschiedlichen stereochemischen Formen existieren. Beispielsweise seien folgende isomere Formen der substituierten Aminomethyltetraline und ihrer heterocyclischen Analoga genannt.

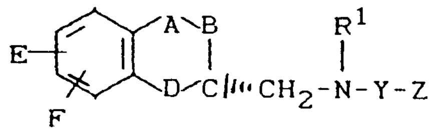

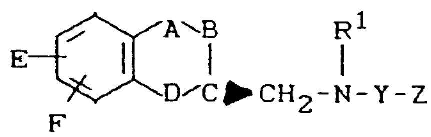

Die erfindungsgemäßen Verbindungen können auch in Form ihrer Salze vorliegen. Im allgemeinen seien hier Salze mit anorganischen oder organischen Säuren genannt.

Im Rahmen der vorliegenden Erfindung werden physiologisch unbedenkliche Salze bevorzugt.

Physiologisch unbedenkliche Salze der substituierten Aminomethyltetraline und ihre heterocyclischen Analoga können Salze der erfindungsgemäßen Stoffe mit Mineralsäuren, Carbonsäuren oder Sulfonsäuren sein. Besonders bevorzugt sind beispielsweise Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Oxalsäure, Malonsäure, Bernsteinsäure, Maleinsäure, Fumarsäure, Äpfelsäure, Weinsäure, Zitronensäure oder Benzoesäure.

Die erfindungsgemäßen Stoffe zeigen überraschenderweise eine vorteilhafte Wirkung auf das Zentralnervensystem und können zur therapeutischen Behandlung von Menschen und Tieren verwendet werden. Gegenüber den bereits bekannten strukturverwandten Verbindungen zeichnen sie sich durch eine größere Selektivität für den $5\text{-}HT_{1A}$-Rezeptor durch zum Teil serotoninantagonistische Wirkung und geringere Nebenwirkungen aus.

Bevorzugt sind die Verbindungen der Formel I,
in welcher

Y

- eine geradkettige Alkylenkette mit 2-5 Kohlenstoffatomen bedeutet,

Z

- eine Gruppe der Formel

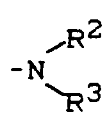

bedeutet,
worin

$R^2$ und $R^3$ gleich oder verschieden sind und
- für Wasserstoff, Methyl, Benzyl oder Pyridyl stehen

- für eine Gruppe der Formel

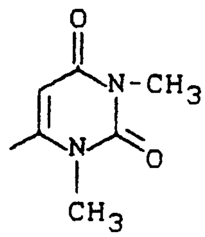

stehen oder

- für eine Gruppe der Formel $-COR^9$ oder $SO_2R^{10}$ stehen,

worin

$R^9$

- für Phenyl oder Ethoxy steht

$R^{10}$

- für Phenyl oder Naphthyl steht, das durch Fluor substituiert sein kann oder
- für Methyl steht
- für eine Gruppe der Formel

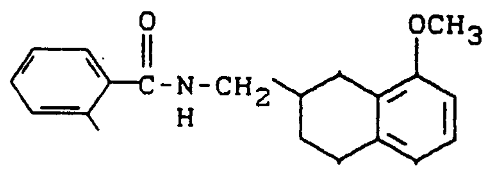

oder

$R^2$ und $R^3$ gemeinsam mit dem Stickstoffatom einen Heterocyclus der Formel

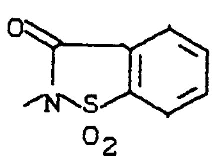 , 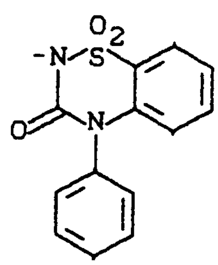 , 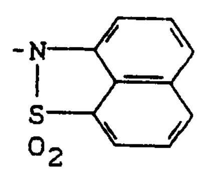 ,

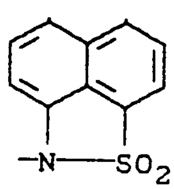 , 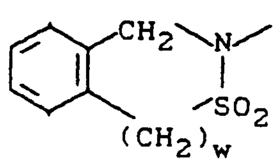 , 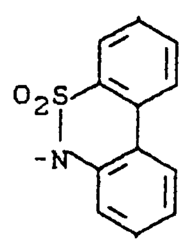 oder

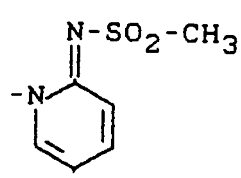 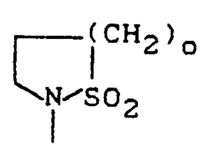

worin

w
- eine Zahl 0, 1 oder 2 bedeutet und

o
- eine Zahl 1 oder 2 bedeutet

bilden,

$R^1$
- Wasserstoff, Methyl, Propyl oder Benzyl bedeutet oder
- eine Gruppe -($Y^1$-$Z^1$) bedeutet, wobei $Y^1$ und $Z^1$ gleich oder verschieden zu Y und Z sein können und die oben angegebene Bedeutung von Y und Z haben

A und D für eine Gruppe der Formel $-CH_2-$, für Sauerstoff oder für den CH- oder N-Teil einer C=C-C=N-Doppelbindung stehen, mit der Maßgabe, daß entweder nur A oder nur D für Sauerstoff stehen darf,

B
- für eine Gruppe der Formel $>CH_2$ oder $\geqslant CH$ oder für den CH-Teil einer C=C- oder C=N-Doppelbindung steht,

C
- für eine Gruppe der Formel $\geqslant CH$ oder den C-Teil einer C=C oder C=N Doppelbindung steht,

E und F gleich oder verschieden sind und für Wasserstoff, Ethoxy, Methoxy, Fluor, Cyano oder eine Gruppe der Formel $-CONR^2R^3$ stehen, worin $R^2$ und $R^3$ Wasserstoff bedeuten,

E und F gemeinsam einen Cyclohexan oder Phenylring bilden,

und deren Salze.

Weiterhin wurden Verfahren zur Herstellung der erfindungsgemäßen Verbindungen der allgemeinen Formel (I) gefunden, dadurch gekennzeichnet, daß man

[A] Verbindungen der allgemeinen Formel (II)

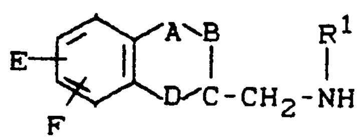

(II),

in welcher

A, B, C, D, E, F und $R^1$ die oben angegebene Bedeutung haben,

(A1) mit Alkylierungsmitteln der Formel (III)

L-Y-Z (III),

in der

Y und Z die oben angegebenen Bedeutungen haben, mit den Maßgaben, daß

Z nicht für Amino steht und

$R^2$ nicht für Wasserstoff steht, wenn

$R^3$ für Alkyl oder Aryl steht,

und

L eine leaving group für Alkylierungsmittel bedeutet,

umsetzt,

oder

(A2) mit Aldehyden der Formel (IV)

OCH-$Y^2$-Z (IV),

in welcher

Z die oben angegebene Bedeutung hat und $Y^2$ eine um eine Methylengruppe verkürzte Alkylenkette Y ist,

reduktiv alkyliert,
oder
(A3) mit reaktiven Säurederivaten der allgemeinen Formel (V)

$M\text{-}CO\text{-}Y^2\text{-}Z$ (V),

in welcher
$Y^2$ und Z die unter den Verfahrensvarianten (A1) angegebenen Bedeutungen haben und M eine leaving group für Acylierungsmittel bedeutet,
umsetzt,
und die entsprechenden Säureamide mit Wasserstoff in Gegenwart eines Katalysators, mit Boranen oder mit komplexen Metallhydriden reduziert,
oder
(A4) mit Nitrilen der Formel

G-CN

in welcher
G für monochloriertes Niederalkyl($C_1$ bis $C_3$), Vinyl oder Niederalkyl($C_1$ bis $C_3$)-substituiertes Vinyl steht,
zu Verbindungen der Formel (VI) und (VII)

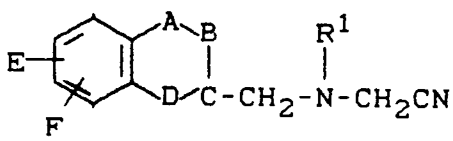

(VI)

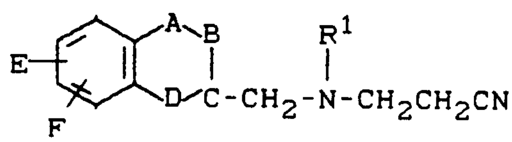

(VII),

worin
A, B, C, D, E, F und $R^1$ die oben angegebene Bedeutung haben,
alkyliert,
die erhaltenen Nitrile zu den Aminen (VIII) und (IX)

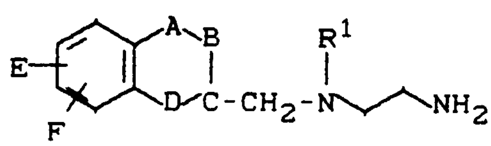

(VIII)

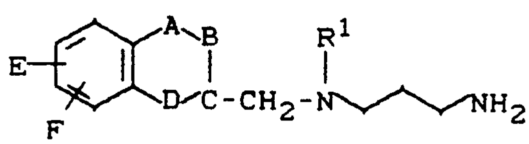

(IX),

worin
A, B, C, D, E, F und $R^1$ die oben genannte Bedeutung haben,
hydriert,
und diese in an sich bekannter Weise durch Alkylierung, reduktive Alkylierung, Acylierung, Reaktion mit Isocyanaten oder Sulfonylierungen umsetzt,
oder indem man

[B] Verbindungen der allgemeinen Formel (X)

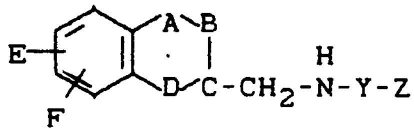 (X),

in welcher
in welcher
A, B, C, D, E, F, Y und Z die oben angegebenen Bedeutungen haben,
mit den Maßgaben, daß
Z nicht für Amino steht und
$R^2$ nicht für Wasserstoff steht, wenn
$R^3$ für Alkyl oder Aryl steht,
(B1) mit Alkylierungsmitteln der Formel (XI)

$R^1$-L (XI),

worin
- $R^1$ die oben angegebene Bedeutung hat und
- L eine leaving group für Alkylierungsmittel bedeutet,

alkyliert,
oder
(B2) mit Aldehyden der Formel (XII)

$R^{14}$-CHO (XII),

in der $R^{14}$ eine um eine Methylengruppe verkürzter Rest $R^1$ bedeutet,
reduktiv alkyliert
oder
(B3) mit reaktiven Säurederivaten der allgemeinen Formel (XIII)

M-CO-$R^{14}$ (XIII)

in welcher
- $R^{14}$ die oben angegebene Bedeutung hat und
- M eine leaving group für Acylierungsmittel bedeutet,

umsetzt
und die entsprechenden Säureamide mit Wasserstoff in Gegenwart eines Katalysators oder mit komplexen Metallhydriden reduziert,
oder indem man
[C] Aldehyde der Formel (XIV)

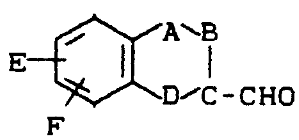 (XIV),

in der
A, B, C, D, E und F die oben angegebene Bedeutung haben,
mit Aminen der Formel (XV), (XVI) oder (XVII)

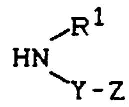

(XV)

$H_2N-R^1$ (XVI)

$H_2N-Y-Z$ (XVII),

worin
$R^1$, Y und Z die oben angegebene Bedeutung haben
mit den Maßgaben, daß
Z nicht für Amino steht und
$R^2$ nicht für Wasserstoff steht, wenn
$R^3$ für Alkyl oder Aryl steht,
in an sich bekannter Weise reduktiv aminiert,
oder indem man
[D] Verbindungen der allgemeinen Formel (XVIII)

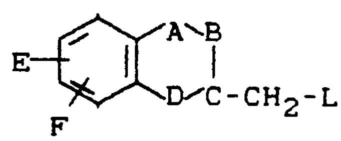

(XVIII),

in welcher
A, B, C, D, E und F und X die oben angegebene Bedeutung haben und
L eine leaving group für Alkylierungsmittel bedeutet,
mit Aminen der Formeln

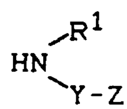

(XV)

$H_2N-R^1$ (XVI)

$H_2N-Y-Z$ (XVII),

worin
$R^1$, Y und Z die oben genannte Bedeutung haben,
mit den Maßgaben, daß
Z nicht für Amino steht und
$R^2$ nicht für Wasserstoff steht, wenn
$R^3$ für Alkyl oder Aryl steht,
umsetzt,
oder mit einem Alkaliazid umsetzt und anschließend die Azidofunktion zu einer Aminofunktion reduziert,
oder indem man

[E] reaktive Carbonsäurederivate der Formel (XIX)

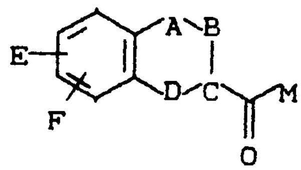

(XIX),

worin
A, B, C, D, E und F die oben angegebene Bedeutung haben, und
M eine leaving group für Acylierungsmittel bedeutet,
mit Aminen der Formeln

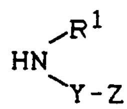

(XV)

$H_2N-R^1$ (XVI)

$H_2N-Y-Z$ (XVII),

worin
$R^1$, Y und Z die oben genannte Bedeutung haben,
mit den Maßgaben, daß
Z nicht für Amino steht, und
$R^2$ nicht für Wasserstoff steht, wenn
$R^3$ für Alkyl oder Aryl steht,
umsetzt,
und die so erhaltenen Amide der Formeln (XX) und (XXI)

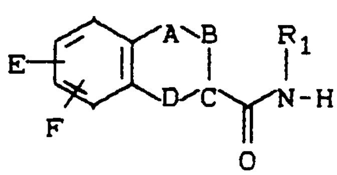

(XX)

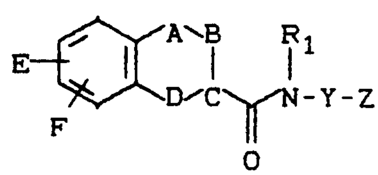

(XXI),

in welcher
A, B, C, D, E, F, $R^1$, Y und Z die oben angegebene Bedeutung haben,
katalytisch mit Wasserstoff, mit komplexen Metallhydriden oder mit Boranen reduziert,
oder indem man
[F] Verbindungen der allgemeinen Formel (XXII)

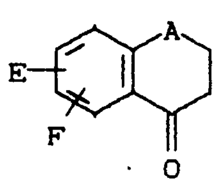

(XXII),

in der A, E und F die oben angegebene Bedeutung haben mit Formaldehyd und Aminen der Formel (XV)

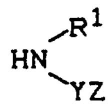

(XV),

worin

$R^1$, Y und Z die oben angegebene Bedeutung haben.

mit den Maßgaben, daß

Z nicht für Amino steht und

$R^2$ nicht für Wasserstoff steht, wenn

$R^3$ für Alkyl oder Aryl steht,

umsetzt und die erhaltenen Zwischenprodukte der allgemeinen Formel (XXIII)

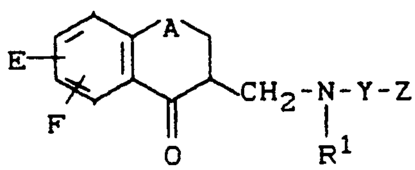

(XXIII),

in welcher

A, E, F, $R^1$, Y und Z die oben angegebene Bedeutung haben,

durch Reduktion der Carbonylfunktion oder durch partielle Reduktion der Carbonylfunktion zur Alkoholfunktion, anschließender, Eliminierung von Wasser und gegebenenfalls einer Hydrierung der C=C-Doppelbindung mit Wasserstoff weiter umsetzt.

Folgende Formelschemata erläutern die Verfahren zur Herstellung der erfindungsgemäßen Verbindungen der allgemeinen Formel I:

**Verfahren A:**

**Variante A1:**

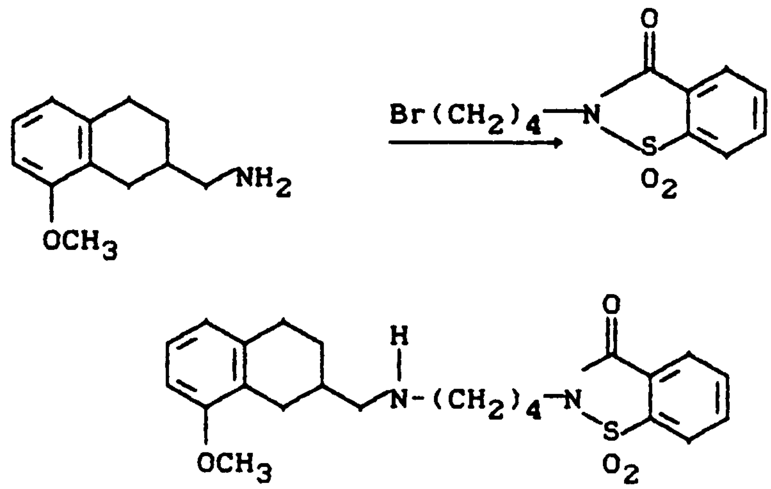

**Variante A2:**

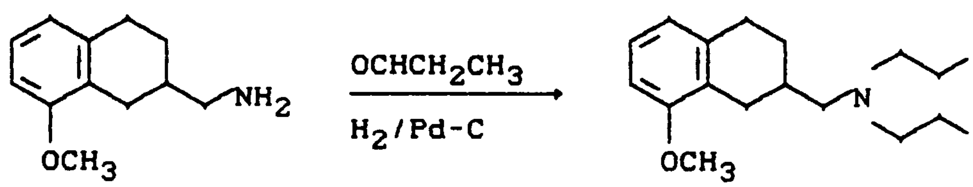


**Variante A3:**

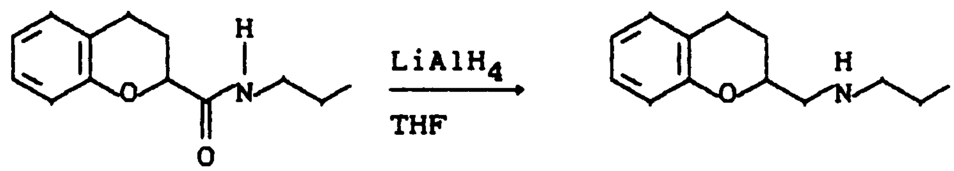

**Variante A4:**

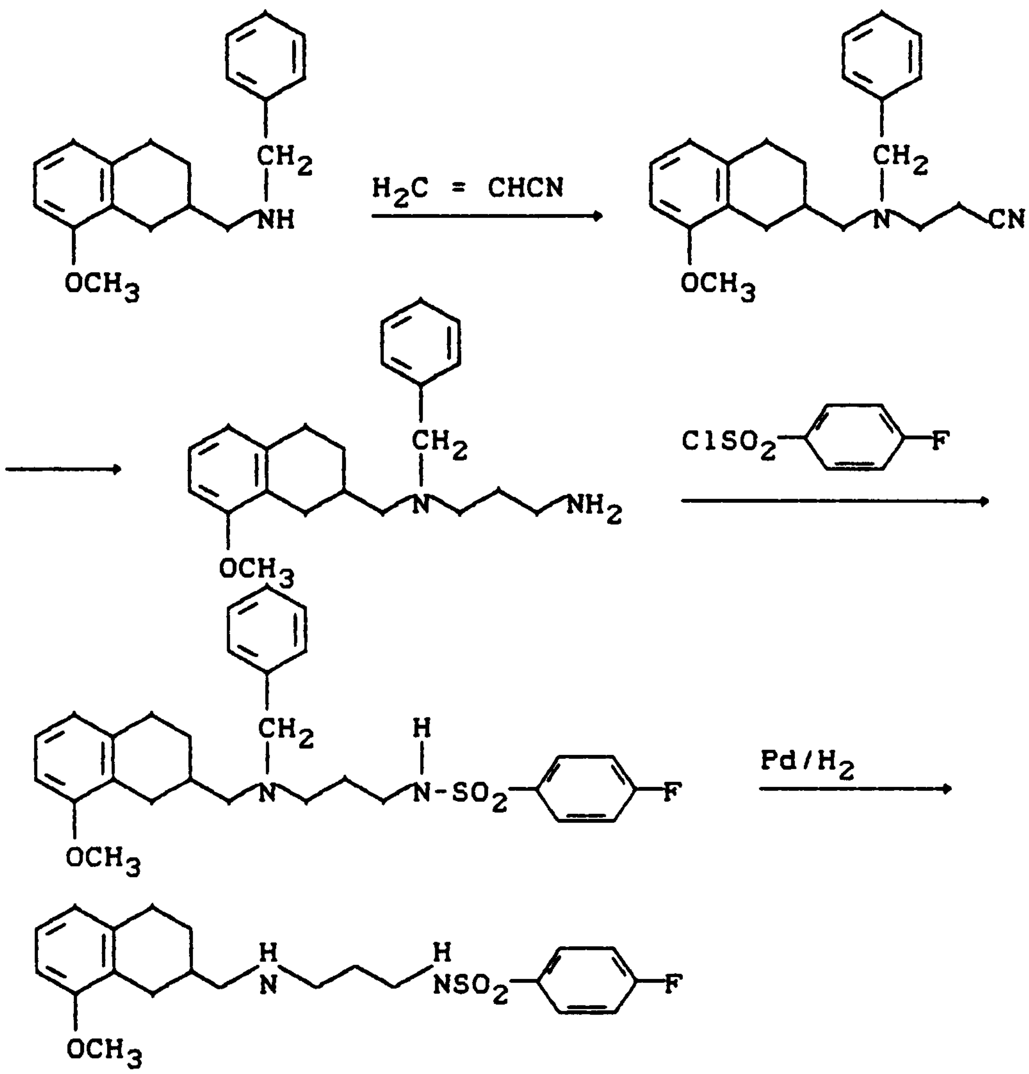


**Verfahren B:**

**Variante B1:**

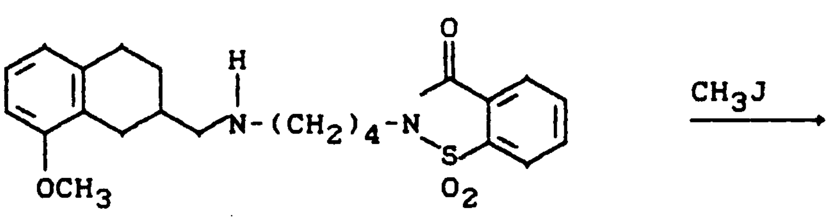

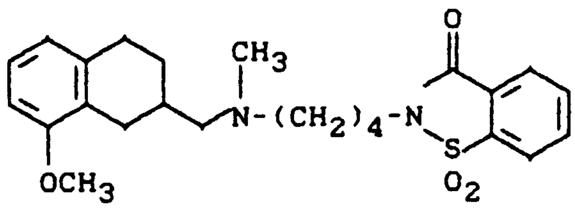

Variante B2:

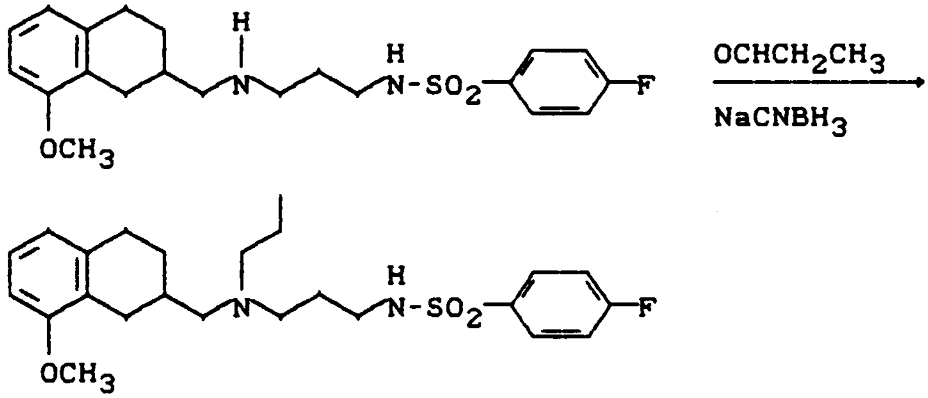


Variante B3:

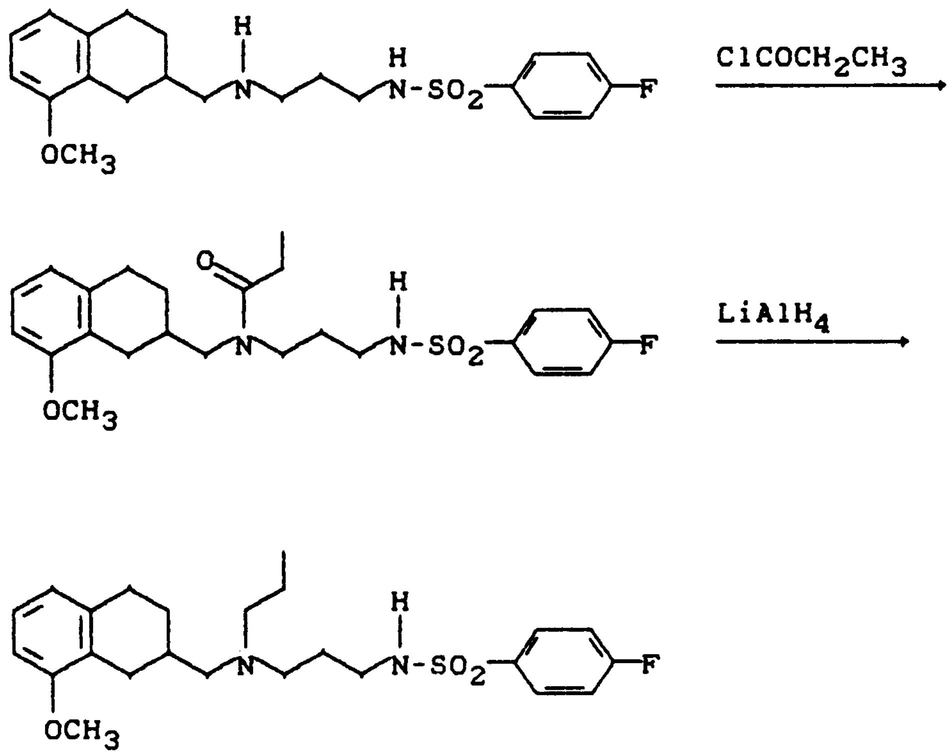

Verfahren C:

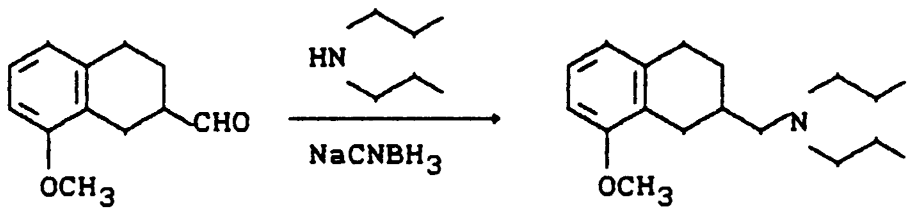


Verfahren D:

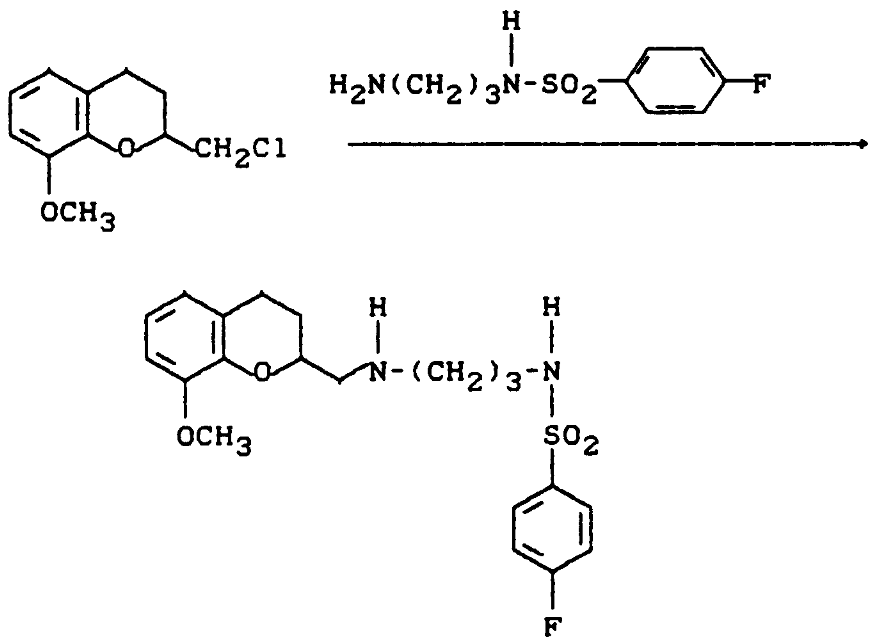

Verfahren E:

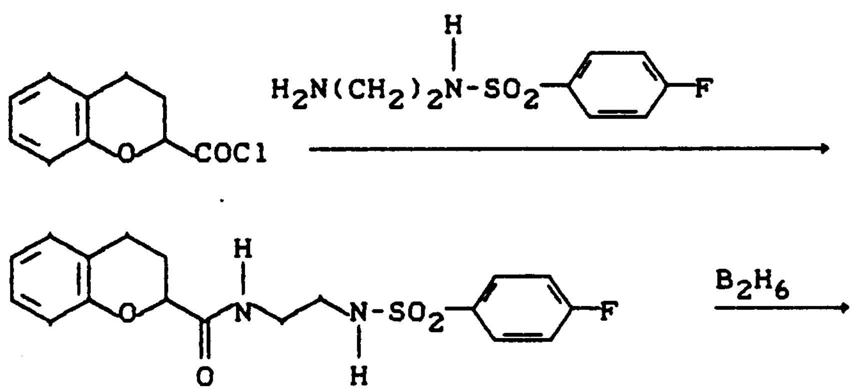

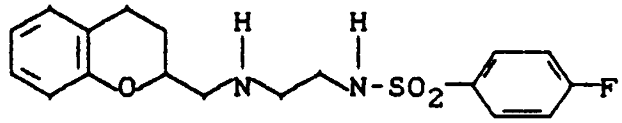

**Verfahren F:**

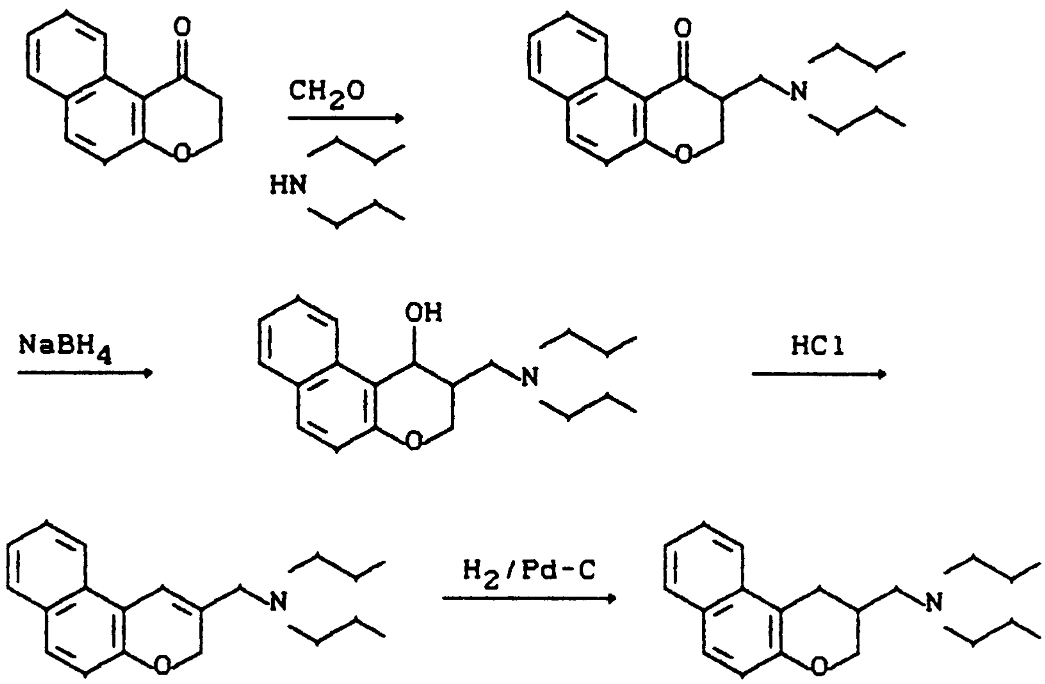


Die als Ausgangsmaterialien verwendeten Amine der Formeln (II) und (X) sind an sich bekannt und können in an sich bekannter Weise aus den entsprechenden Ketonen durch reduktive Aminierung, Alkylierung oder reduktive Alkylierung hergestellt werden (vgl. GB 1 043 857, J. Med. Chem. 29, 1619, 1968, J. Med. Chem. 11, 844 (1968), EP-A 0 192 288). P.A. Robins, J. Walev, J. Chem. Soc. 1958, 409, J. Chem. Soc. 1942, 689).

Als Lösemittel für die Umsetzung der Amine (II) und (X) mit den Alkylierungsmitteln (III) und (XI) können hier die üblichen organischen Lösemittel verwendet werden, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium.

Leaving groups für Alkylierungsmittel (L) sind an sich bekannt (Lit. H.R. Christen, Grundlagen der organischen Chemie Sauerländer-Diesterweg-Salle 1975). Beispielsweise seien hier Chlor, Brom, Jod, Tosylat, Mesylat oder die Gruppe $-OSO_2CF_3$ genannt.

Leaving groups für Acylierungsmittel (M) sind an sich bekannt (Lit. H.R. Christen, Grundlagen der organischen Chemie, Sauerländer-Diesterweg-Salle 1975). Beispielsweise seien hier Chlor, Brom, Alkoxy($C_1$ bis $C_4$), Aryloxy, Imidazolyl, Thiazolyl, Methansulfonyloxy oder Alkoxy($C_1$ bis $C_4$)carbonyl genannt.

Die Umsetzung wird im allgemeinen in einem Temperaturbereich von 0 °C bis +150 °C, bevorzugt in einem Bereich von Raumtemperatur bis +80 °C, durchgeführt.

Die Reaktion wird im allgemeinen bei Normaldruck durchgeführt. Es ist jedoch ebenso möglich, die Reaktion bei erhöhtem oder erniedrigtem Druck durchzuführen.

Als Reaktionsbeschleuniger werden im allgemeinen Alkalijodide eingesetzt, bevorzugt sind Natriumjodid oder Kaliumjodid.

Die Base wird hierbei in einer Menge von 1 bis 5, bevorzugt von 1 bis 2 Mol, bezogen auf 1 Mol der Halogenverbindung, eingesetzt. Die Halogenverbindung wird bevorzugt in einer bis zu 10-fachen, bevorzugt in einer bis zu 5-fachen, Überschußmenge über die Verbindungen der Formel (II) oder (X) eingesetzt.

Die reduktive Alkylierung der Amine (II) und (X) mit den Aldehyden (IV) und (XII) erfolgt im allgemeinen in einem Schritt. Ist das Amin (II) ein primäres Amin kann die Reaktion auch zweistufig durchgeführt werden, wobei zunächst eine Schiff'sche Base bzw. ein Enamin erhalten wird.

Die Herstellung der Schiff'schen Basen bzw. Enamine in ersten Schritt erfolgt in inerten organischen Lösemitteln, gegebenenfalls in Anwesenheit eines Katalysators und gegebenenfalls in Anwesenheit eines wasserbindenden Mittels. Das erfindungsgemäße Verfahren kann in 2 Schritten, d.h., mit Isolierung der Zwischenprodukte durchgeführt werden. Ebenso ist es möglich, die Reduktion als Eintopfverfahren durchzuführen.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldiethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid oder Essigsäure. Außerdem ist es möglich, Gemische der genannten Lösemittel zu verwenden.

Als Katalysatoren werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Das bei der Reaktion gebildete Wasser kann gegebenenfalls im Gemisch mit dem verwendeten Lösemittel während oder nach der Reaktion z.B. durch Destillation oder durch Zugabe von wasserbindenden Mitteln, wie beispielsweise Phosphorpentoxid oder bevorzugt durch Molekularsieb entfernt werden.

Die Reaktion wird im allgemeinen in einem Temperaturbereich von 0 °C bis 150 °C, bevorzugt von +20 °C bis +100 °C, durchgeführt.

Die Umsetzung kann bei normalem, erhöhtem und bei erniedrigtem Druck durchgeführt werden (z.B. 0,5 - 5 bar). Im allgemeinen arbeitet man bei Normaldruck.

Bei der Durchführung wird die Verbindung (XV) in einer Menge von 0,1 - 10, bevorzugt von 0,5 - 5 Mol, bezogen auf 1 mol der Verbindungen (II) oder (X), eingesetzt.

Die Reduktion der Schiff'schen Basen bzw. Enamine im zweiten Schritt erfolgt entweder durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden in inerten Lösemitteln, gegebenenfalls in Anwesenheit eines Katalysators.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion mit Natriumcyanoborhydrid werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder mit Arylresten wie beispielsweise Methansulfonsäure, Ethansulfonsäure, Benzolsulfonsäure oder Toluolsulfonsäure.

Bei der Durchführung des erfindungsgemäßen Verfahrens hat es sich als günstig erwiesen, die Umsetzung der Aldehyde (IV) und (XII) mit den Aminen (II) und (X) in einem inerten Lösemittel, bevorzugt in Essigsäure oder Alkoholen, wie beispielsweise Methanol, Ethanol, Propanol oder Isopropanol oder deren Gemischen, in Anwesenheit von anorganischen oder organischen Säuren wie beispielsweise Chlorwasserstoffsäure oder Essigsäure, und in Anwesenheit eines Reduktionsmittels, bevorzugt von komplexen Hydriden wie beispielsweise Natriumborhydrid oder Natriumcyanoborhydrid, gegebenenfalls in Anwesenheit eines wasserentziehenden Mittels, bevorzugt Molekularsieb, als Eintopfverfahren durchzuführen.

In diesem Fall wird die Reaktion in einem Temperaturbereich von 0 °C bis +150 °C, bevorzugt von 0 °C bis +100 °C bei Normaldruck durchgeführt. Es ist ebenso möglich, die Reaktion bei Unterdruck oder bei Überdruck (z.B im Bombenrohr) durchzuführen.

Das Überführen von funktionellen Gruppen in andere funktionelle Gruppen in den oben aufgeführten Herstellungsverfahren erfolgt je nach Art der funktionellen Gruppen durch Oxidation, Reduktion, Hydrolyse oder durch Umsetzung mit elektrophilen Reagenzien und soll im folgenden erläutert werden.

1. Die Reduktion der Nitrilgruppe zur Aminogruppe erfolgt im allgemeinen mit Metallhydriden, bevorzugt mit Lithiumaluminiumhydrid, Aluminiumhydrid (hergestellt z.B. durch Umsetzung von Lithiumaluminumhydrid mit 100%iger Schwefelsäure oder mit Aluminiumchlorid) oder deren Gemischen in inerten Lösemitteln wie Ethern oder Chlorkohlenwasserstoffen, bevorzugt in Ethern wie beispielsweise Tetrahydrofuran, Diethylether oder Dioxan in einem Temperaturbereich von -20 °C bis +100 °C, bevorzugt von 0 °C bis +50 °C, bei Normaldruck.

Die Reduktion ist außerdem durch Hydrieren der Nitrile in inerten Lösemitteln wie Alkoholen z.B. Methanol, Ethanol, Propanol oder Isopropanol in Gegenwart eines Edelmetallkatalysators wie Platin, Palladium, Palladium auf Tierkohle oder Raney-Nickel, in einem Temperaturbereich von 0 °C bis +150 °C, bevorzugt von Raumtemperatur bis +100 °C, bei Normaldruck oder bei Überdruck möglich.

Die Reaktion kann durch folgendes Schema verdeutlicht werden:

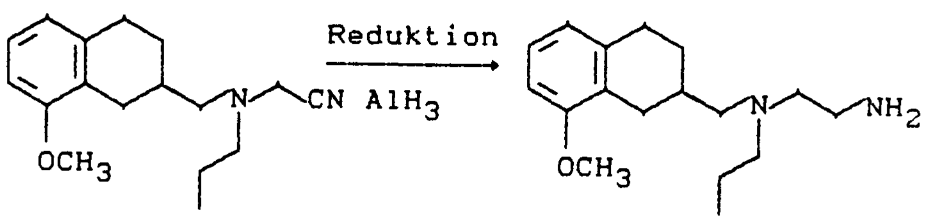


2. Die Reduktion von Alkoxycarbonylgruppen in Alkoholgruppen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0 °C bis +150 °C, bevorzugt von +20 °C bis +100 °C, bei Normaldruck.

Die Reaktion kann durch folgendes Schema verdeutlicht werden:

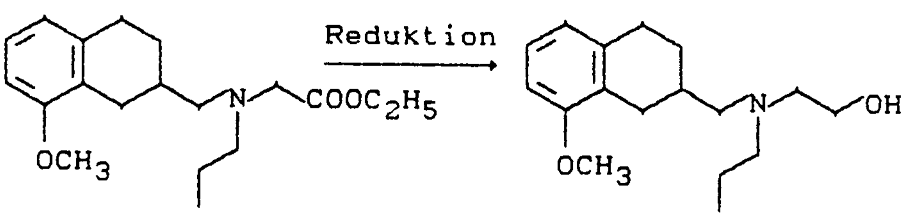


3. Die Hydrolyse der Nitrilgruppe zur Carbonamidgruppe erfolgt im allgemeinen mit Hilfe von starken Mineralsäuren, bevorzugt mit Chlorwasserstoff in inerten Lösemitteln wie Wasser und/oder Alkoholen wie beispielsweise Methanol, Ethanol, Propanol oder Isopropanol in einem Temperaturbereich von 0 °C bis +150 °C, bevorzugt von +20 °C bis +100 °C, bei Normaldruck.

Die Reaktion kann durch folgendes Schema erläutert werden:

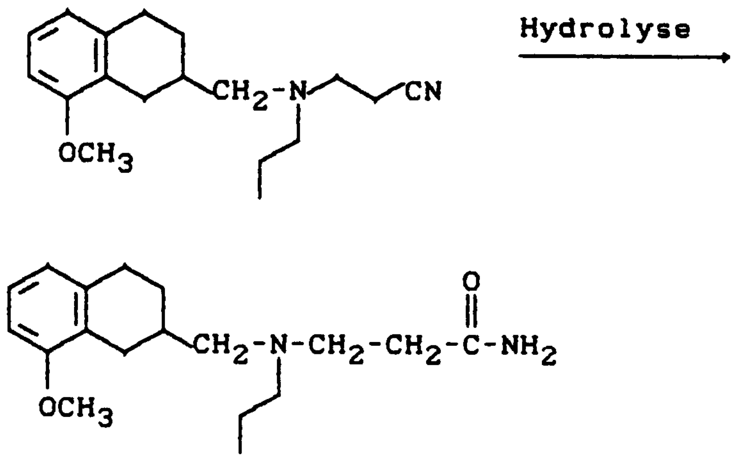


4. Durch Umsetzung von NH- oder OH-aciden Verbindungen (Z in Formel (I) ist $NR^2R^3$, wobei $R^2$ = H und $R^3$ = H, Alkyl, Aryl oder Aralkyl ist), mit elektrophilen Reagenzien erhält man eine Vielzahl weiterer erfindungsgemäßer Verbindungen:

a) Die Überführung von Aminen in Carbonamide erfolgt im allgemeinen durch Umsetzung mit Carbonsäureestern in inerten Lösemitteln wie Ethern oder deren Gemischen oder Kohlenwasserstoffen, bevorzugt in Ethern wie beispielsweise Diethylether, Tetrahydrofuran oder Dioxan, gegebenenfalls in Anwesenheit von Basen wie Alkalimetalle, Alkalihydride, Alkalialkoholate oder lithiumorganischen Verbindungen, bevorzugt in Anwesenheit von Alkalimetallen, wie beispielsweise Natrium oder Alkalihydriden wie Natriumhydrid oder Kaliumhydrid in einem Temperaturbereich von +20 °C bis +150 °C, bevorzugt bei der Siedetemperatur des verwendeten Lösemittels bei Normaldruck.

Darüber hinaus ist es möglich, die Amide mit Carbonsäurehalogeniden oder -anhydriden, bevorzugt mit Carbonsäurechloriden in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran, oder Halogenkohlenwasserstoffen wie Methylenchlorid oder Chloroform, gegebenenfalls in Anwesenheit von Basen wie Alkalicarbonaten beispielsweise Natriumcarbonat oder Kaliumcarbonat, oder organischen Aminen wie beispielsweise Triethylamin oder Pyridin, in einem Temperaturbereich von -20 °C bis +100 °C, bevorzugt von 0 °C bis +60 °C, bei Normaldruck herzustellen.

Die Reaktion kann durch folgendes Schema verdeutlicht werden:

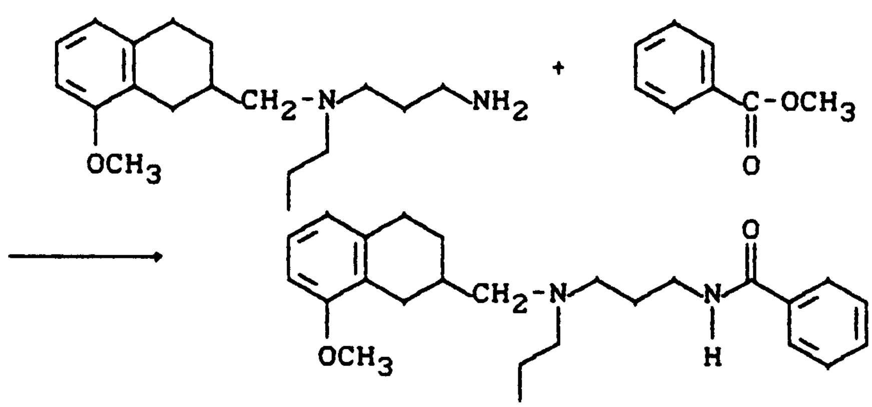

b) Die Überführung von Aminen in Carbamate erfolgt im allgemeinen mit Kohlensäureestern, bevorzugt mit Kohlensäureestern, die einen Phenylesterrest tragen oder mit Chlorkohlensäureestern, in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan, in einem Temperaturbereich von +20 °C bis +150 °C, bevorzugt von +20 °C bis +100 °C, bei

Normaldruck. Die Reaktion kann auch in einem Zweiphasensystem durchgeführt werden, wobei die wäßrige Phase eine Hilfsbase wie Natrium- oder Kaliumcarbonat oder -hydrogencarbonat enthält.

Die Reaktion kann durch folgendes Schema erläutert werden:

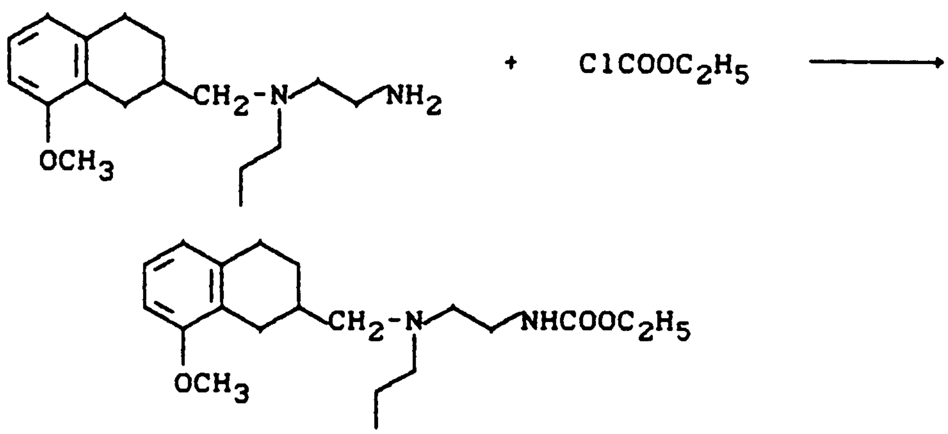

c) Die Überführung von Aminen in Harnstoffe erfolgt im allgemeinen durch Umsetzung mit Isocyanaten in inerten Lösemitteln wie Ethern, Kohlenwasserstoffen oder Halogenkohlenwasserstoffen oder deren Gemischen, bevorzugt in Ethern wie beispielsweise Diethylether oder Tetrahydrofuran, oder in Halogenkohlenwasserstoffen wie beispielsweise Methylenchlorid oder Chloroform, in einem Temperaturbereich von -20 °C bis +150 °C, bevorzugt von 0 °C bis +100 °C, bei Normaldruck.

Die Reaktion kann durch folgendes Schema verdeutlicht werden:

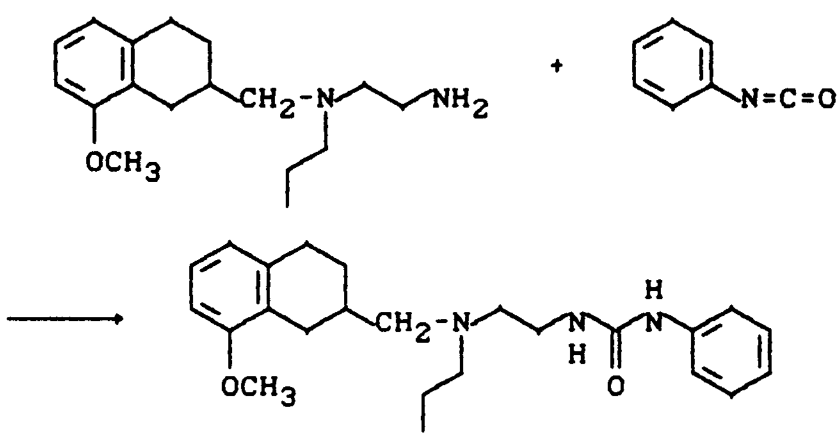

d) Die Überführung von Aminen in Sulfonamide bzw. Aminosulfamoylderivate erfolgt im allgemeinen mit Sulfonsäurehalogeniden bzw. mit Amidosulfonsäurehalogeniden, bevorzugt mit den entsprechenden Chloriden in inerten Lösemitteln wie Ethern, Kohlenwasserstoffen oder Halogenkohlenwasserstoffen oder deren Gemische, bevorzugt in Halogenkohlenwasserstoffen wie beispielsweise Methylenchlorid oder Chloroform, gegebenenfalls in Anwesenheit von Basen wie Alkalihydroxide, Alkalicarbonate, Alkalialkoholate oder organische Amine, bevorzugt mit Alkalihydroxiden wie Natriumhydroxid oder Kaliumhydroxid, Alkalicarbonaten wie beispielsweise Natriumcarbonat oder Kaliumcarbonat, oder organische Aminen wie Triethylamin oder Pyridin, in einem Temperaturbereich von -20 °C bis +100 °C, bevorzugt von 0 °C bis +50 °C bei Normaldruck.

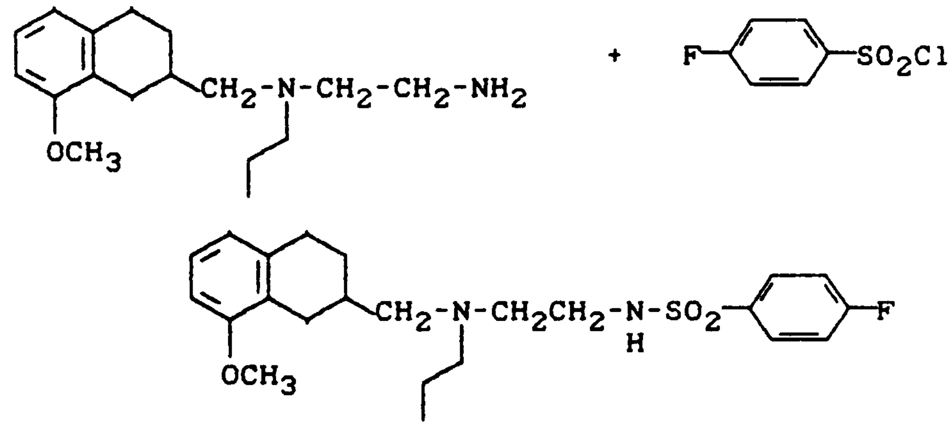

e) Cyclische Sulfonamide werden im allgemeinen durch Reaktion intramolekularer Elektrophile in inerten dipolar aprotischen Lösemitteln, bevorzugt in Dimethylformamid, Hexamethylphosphorsäuretriamid oder Dimethylsulfoxid, gegebenenfalls in Anwesenheit von Basen wie Alkalimetalle, Alkalihydride, Alkaliamide, Alkalialkoholate oder lithiumorganischen Verbindungen, bevorzugt in Anwesenheit von Alkalihydriden wie Natriumhydrid oder Kaliumhydrid, oder Alkaliamiden wie Natriumamid oder Lithiumdiisopropylamid, gegebenenfalls in Gegenwart katalytischer Mengen eines Alkaliiodides, beispielsweise Natriumiodid oder Kaliumiodid in einem Temperaturbereich von -20 °C bis +100 °C, bevorzugt von 0 °C bis +50 °C, bei Normaldruck hergestellt.

Die Reaktion läßt sich durch folgendes Schema verdeutlichen:

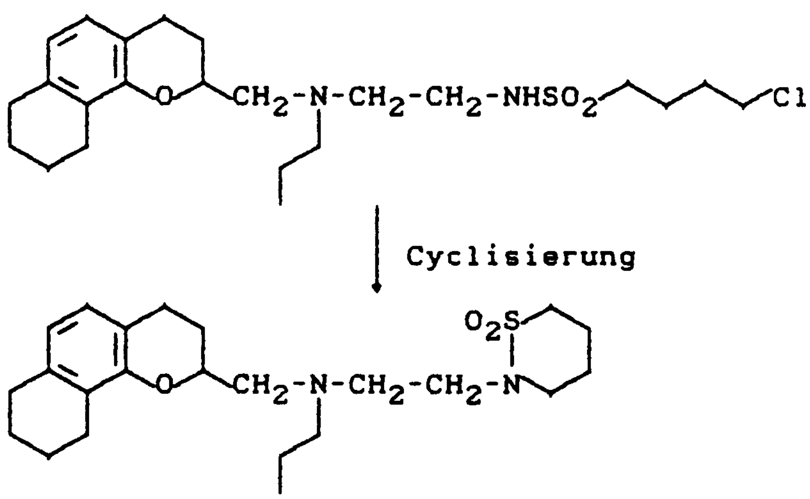


f) Die Überführung der Hydroxygruppe in Kohlensäureester erfolgt im allgemeinen durch Umsetzen mit Halogenameisensäureestern, bevorzugt mit Chlorameisensäureestern in inerten Lösemitteln wie Ethern, Kohlenwasserstoffen oder Halogenkohlenwasserstoffen, bevorzugt in Halogenkohlenwasserstoffen wie Methylenchlorid oder Chloroform, oder in Ethern wie Diethylether oder Tetrahydrofuran, gegebenenfalls in Anwesenheit von Basen wie Alkylihydroxide, Alkalicarbonate oder organischen Aminen, bevorzugt in Anwesenheit von organischen Aminen wie Triethylamin, Pyridin, Picolin oder Dimethylaminopyridine in einem Temperaturbereich von -20 °C bis +100 °C, bevorzugt von 0 °C bis Raumtemperatur bei Normaldruck.

Die Reaktion läßt sich durch folgendes Schema verdeutlichen:

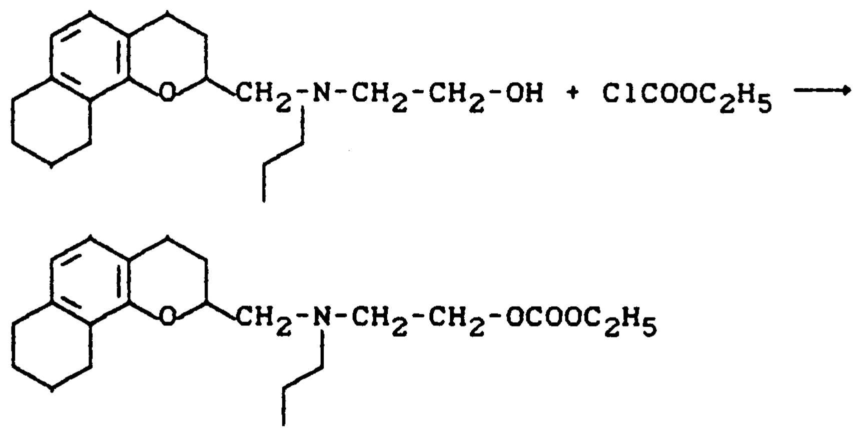

5. Die Oxidation der Thioethergruppe zu Sulfoxiden bzw. Sulfonen erfolgt im allgemeinen mit Oxidationsmitteln wie Peroxoverbindungen oder Wasserstoffperoxid selbst, bevorzugt mit Wasserstoffperoxid, in inerten Lösemitteln wie Carbonsäuren und Carbonsäureanhydriden, bevorzugt in Essigsäure, in einem Temperaturbereich von -20 °C bis +100 °C, bevorzugt von 0 °C bis +50 °C.

Die Reaktion läßt sich durch folgendes Schema verdeutlichen:

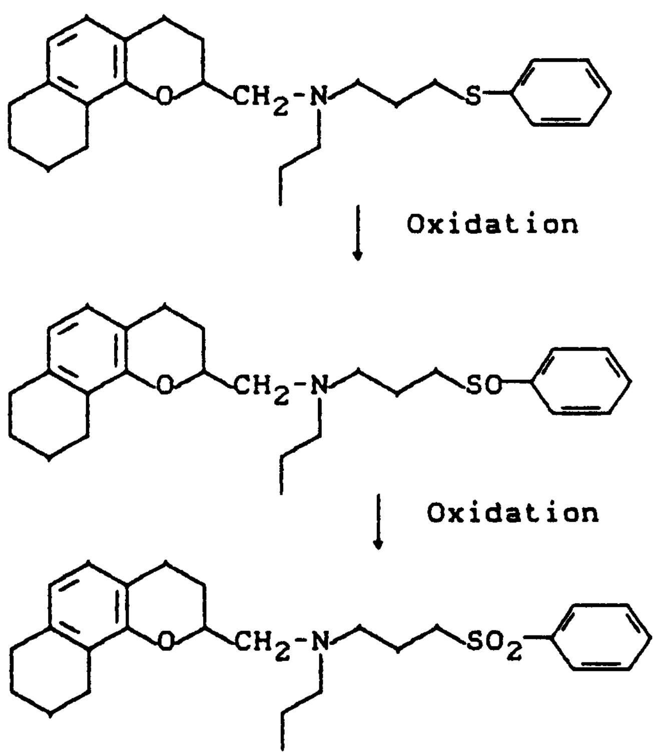


Die als Ausgangsstoffe eingesetzten Amine der allgemeinen Formel (XV), (XVI) und (XVII) sind bekannt oder können nach bekannten Methoden hergestellt werden [Houben-Weyl's "Methoden der organischen Chemie" Bd XI/1 und XI/2).

Als Amine können beispielsweise erfindungsgemäß verwendet werden:
Ammoniak, Methylamin, Ethylamin, Propylamin, Isopropylamin, Butylamin, 4-Dimethylaminobutylamin, 4-Diethylaminobutylamin, 3-Dimethylaminopropylamin, 3-Diethylaminopropylamin, 2-Dimethylaminoethylamin, 2-Diethylaminoethylamin, 2-Amino-1-ethoxycarbonylamidoethan, 3-Amino-1-ethoxycarbonylamido-propan, 4-Amino-1-ethoxycarbonylamido-butan, 3-Aminochinuclidin, 2-[(Phenylaminocarbonyl)amino]ethylamin, 2-[-

(Phenylaminocarbonyl)amino]propylamin, 4-Aminomethyl-piperidin, 4-(Ethoxycarbonyl)amino-ethyl-piperidin, N-Methylpiperazin, 4-Amino-1-carboxyethyl-piperidin, N,N-Dimethylpropyliden-diamin, N,N-Diethylpropyliden-diamin, N,N-Diethylethyliden-diamin, N,N-Dimethylethylen-diamin, N-(2-Aminoethyl)ethylcarbamat, N-(2-Aminoethyl)propylcarbamat.

Die Umsetzungen der Verbindungen der allgemeinen Formel (XIX), (XXII) und (XIV) erfolgen mit Aminen der allgemeinen Formel (XV), (XVI) und (XVII) in inerten organischen Lösemitteln, gegebenenfalls in Anwesenheit eines Katalysators und gegebenenfalls in Anwesenheit von wasserverbindenden Mitteln.

Als inerte Lösemittel eignen sich hierbei die üblichen organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Butylmethylether, Dioxan, Tetrahydrofuran, Glykoldimethylether oder Diethylenglykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexamethylphosphorsäuretriamid, oder Essigsäure. Außerdem ist es möglich, Gemische der genannten Lösemittel zu verwenden.

Als Katalysatoren werden im allgemeinen Säuren verwendet. Hierzu gehören bevorzugt anorganische Säuren wie beispielsweise Salzsäure oder Schwefelsäure, oder organische Sulfon- oder Carbonsäuren wie beispielsweise Methansulfonsäure, Ethansulfonsäure, Benzolsulfonsäure, Toluolsulfonsäure, Essigsäure oder Propionsäure.

Das bei der Reaktion gebildete Wasser kann gegebenenfalls im Gemisch mit dem verwendeten Lösemittel während oder nach der Reaktion, z.B. durch Destillation oder durch Zugabe von wasserbindenden Mitteln, wie beispielsweise Phosphorpentoxid oder bevorzugt durch Molekularsieb, entfernt werden.

Die Reaktionen werden im allgemeinen in einem Temperaturbereich von 0 °C bis +150 °C, bevorzugt von +20 °C bis +100 °C, durchgeführt.

Die Umsetzung kann bei normalen, erhöhtem oder bei erniedrigtem Druck durchgeführt werden (z.B. 0,5 - 5 bar). Im allgemeinen arbeitet man bei Normaldruck.

Die Reduktionen der Verbindungen der Formeln (XX), (XXI) und (XXIII) erfolgen entweder durch Wasserstoff in Wasser oder inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators.

Bevorzugt werden die Reaktionen mit Hydriden, wie komplexen Borhydriden oder Aluminiumhydriden, durchgeführt. Besonders bevorzugt werden hierbei Natriumborhydrid, Lithiumaluminiumhydrid, Natriumcyanoborhydrid oder Borwasserstoffsäure eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether oder Diethylenglykoldimethylether, oder Amide wie Hexamethylphosphorsäuretriamid, oder Dimethylformamid oder Essigsäure. Ebenso ist es möglich, Gemische der genannten Lösemittel zu verwenden.

Als Katalysatoren bei der Reduktion mit Natriumcyanoborhydrid werden im allgemeinen Säuren verwendet. Hierzu gehören bevorzugt anorganische Säuren wie beispielsweise Salzsäure oder Schwefelsäure oder organische Carbonsäuren oder Sulfonsäuren, wie beispielsweise Essigsäure, Trifluoressigsäure, Trichloressigsäure, Methansulfonsäure, Ethansulfonsäure, Benzolsulfonsäure oder Toluolsulfonsäure.

Die Reduktion der Säureamide erfolgt entweder durch Wasserstoff in Wasser oder inerten organischen Lösemitteln in Alkoholen, Ethern oder Halogenkohlenwasserstoffen oder deren Gemischen, mit Katalysatoren wie Raney-Nickel, Palladium, Palladium auf Tierkohle oder Platin, oder aber mit Hydriden in inerten Lösemitteln, gegebenenfalls in Anwesenheit eines Katalysators, oder mit Boranen, Diboranen oder ihren Komplexverbindungen.

Bevorzugt werden die Reaktionen mit Hydriden, wie komplexen Borhydriden oder Aluminiumhydriden, durchgeführt. Besonders bevorzugt werden hierbei Natriumborhydrid, Lithiumaluminiumhydrid, Natriumcyanoborhydrid oder Borwasserstoffsäure eingesetzt.

Die Verbindungen der allgemeinen Formel (XIX) sind an sich bekannt oder können nach üblichen Methoden hergestellt werden (J. Med. Chem. 1972, 15, 8650, J. Gen. Chem. (USSR) 36, 3856 (1960).

Die Verbindungen der Formel (XXII) sind bekannt oder können nach üblichen Methoden hergestellt werden (J. Med. Chem., 1972, 15, Nr. 8, Publs. Soc. Chim. Tr. 1958, 325, J. Am. Chem. Soc. (9, 2341, 1947).

Die Halogenverbindungen der allgemeinen Formeln (III) und (XI) sind bekannt oder können nach bekannten Methoden hergestellt werden [Beilstein's Handbuch der organischen Chemie 2, 197, 201, 250, 278; 3, 9, 10; 21, 461, 462, 463].

Als Halogenverbindungen können beispielsweise erfindungsgemäß verwendet werden:
Chloracetonitril, 2-Chlorpropionnitril, 3-Chlorbutyronitril, 3-Brompropylphthalimid, 3-Chlorpropylphthalimid, 2-Bromethylphthalimid, 4-Brombutylphthalimid, 4-Chlorbutylphthalimid, Chloressigsäurediethylamid, Chloressigsäuredimethylamid, Chloressigsäuremethylester, Chloressigsäureethylester, Bromessigsäureethylester, Bromessigsäuremethylester, 2-δ-Brombutyl-1,2-benzoisothiazol-3(2H)-on-1,1-dioxid, 2-γ-Brompropyl-1,2-benzoisothiazol-3(2H)-on-1,1-dioxid.

Die als Ausgangsstoffe eingesetzten Aldehyde der allgemeinen Formeln (IV) und (XII) sind bekannt oder können nach bekannten Methoden hergestellt werden [Beilstein's Handbuch der organischen Chemie 1, 594, 629, 662].

Als Aldehyde können beispielsweise erfindungsgemäß verwendet werden:
Acetaldehyd, Propionaldehyd, Butyraldehyd, Benzaldehyd.

Die erfindungsgemäßen Verbindungen können als Wirkstoff in Arzneimitteln verwendet werden.

Die erfindungsgemäßen Stoffe haben eine besonders hohe Affinität zu cerebralen 5-Hydroxy-tryptamin-Rezeptoren vom 5-$HT_1$-Typ.

Sie haben agonistische, partiell agonistische oder antagonistische Wirkungen am Serotonin-Rezeptor. Im Vergleich zu den strukturverwandten bekannten Verbindungen weisen sie überraschenderweise eine größere therapeutische Breite auf.

Die in der vorliegenden Erfindung beschriebenen hochaffinen Liganden für den Serotonin-1-Rezeptor stellen somit Wirkstoffe zur Bekämpfung von Krankheiten dar, die durch Störungen des serotoninergen Systems, insbesondere bei Involvierung von Rezeptoren, die hohe Affinität zu 5-Hydroxytryptamin besitzen (5-$HT_1$-Typ), gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlaf- und Nahrungsaufnahmestörungen. Weiterhin sind sie geeignet zur Beseitigung kognitiver Deficite, zur Verbesserung von Lern- und Gedächtnisleistungen und zur Behandlung der Alzheimer'schen Krankheit.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.

Auch eignen sie sich zur Prophylaxe und Bekämpfung der Folgen cerebraler Infarktgeschehen (Apoplexiacerebri) wie Schlaganfall, cerebraler Ischämien. Ebenso können die erfindungsgemäßen Verbindungen zur Bekämpfung von Schmerzzuständen eingesetzt werden. Auch eignen sie sich zur Bekämpfung von Erkrankungen des Intestinaltraktes, die durch Störungen des serotoninergen Systems wie auch durch Störungen des Kohlehydrathaushaltes gekennzeichnet sind.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägmaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Die jeweils aufgeführten $R_f$-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in steigendem Ausmaß die polare Laufmittelkomponente zugemischt, bis das gewünschte Produkt eluiert wird (DC-Kontrolle).

Bei allen Produkten wurde bei zuletzt ca. 13,3 Pa (0,1 Torr) das Lösemittel abdestilliert. Salze wurden bei diesem Druck über Nacht über Kaliumhydroxid und/oder Phosphorpentoxid aufbewahrt.

Ausgangsverbindungen und Herstellungsbeispiele

Beispiel 1

2-Aminomethyl-5-methoxy-chroman

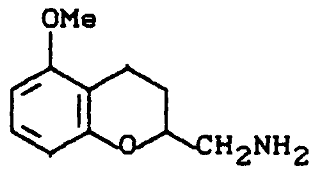

Zur siedenden Suspension von 0,30 g (8,2 mmol), Lithiumaluminiumhydrid in 5 ml THF wurden 0,85 g (4,1 mmol) 2-Carbonamido-5-methoxy-chroman, in 5 ml THF zugetropft. Nach 3 h Erhitzen zum Rückfluß wurde abgekühlt und nacheinander mit 0,3 ml Wasser und 0,5 ml 20 % Natronlauge versetzt. Filtration über Kieselgur und Einengen ergeben 0,62 g Rohrprodukt, das durch Flashchromatographie (Toluol-Essigester-, dann -Ethanolgradienten) gereinigt wurde.

Ausbeute: 0,52 g (66 %).

Rf: (Toluol/Methanol 4:1) = 0,16

IR ($CHCl_3$): 3389, 3009, 2947, 1607, 1593, 1470.

Beispiel 2

2-(N-Propyl)aminomethyl-chroman

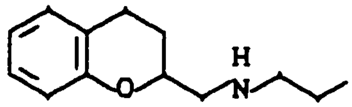

Die Verbindung wurde analog der Vorschrift von Beispiel 1 hergestellt.

IR($CHCl_3$): 3320 (breit), 3010, 2965, 2879, 1607, 1584, 1490, 1457, 1235.

Beispiel 3

2-(N-Propyl)aminomethyl-5-methoxy-chroman

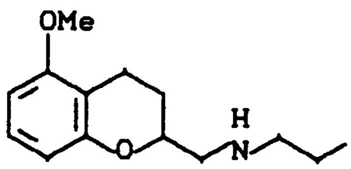

Die Verbindung wurde analog der Vorschrift von Beispiel 1 hergestellt.

$^1$H-NMR ($CDCl_3$): 0,85 (t; 3H), 1,55 (quint.; 3H), 1,7 (m; 1H), 2,0 (m; 1H), 2,5-3,0 (m; 6H), 3,7 (s; 3H), 4,1 (m, 1H), 6,4 (d; 1H), 6.5 (d; 1H), 7,0 (t, 1H).

Beispiel 4

2-Aminomethyl-benzo(h)chroman-hydrochlorid

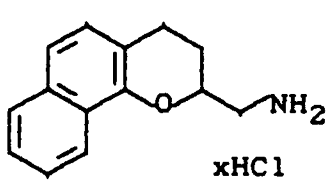

Die Verbindung wurde analog Beispiel 24 aus 2-Carbonamido-benzo(h)chroman hergestellt.

Beispiel 5

1-Methoxy-5,6-dihydro-7-nitromethyl-naphthalin

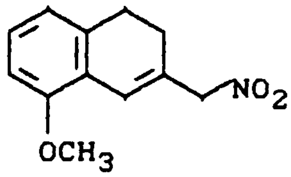

44,1 g 8-Methoxy-tetralon-2 (0,25 mol) wurden in 402 ml Nitromethan (7,50 mol) und 2,5 ml Ethylendiamin 3h unter Argon bei 70 ° C gerührt. Nachdem sich der Ansatz auf Raumtemperatur abgekühlt hat, wird ein gelbes Kristallisat abgesaugt. Aus der Lösung wird das Nitromethan unter Vakuum abdestilliert. Der Rückstand wird in 500 ml Toluol gelöst. Unter Vakuum wird das Lösungsmittel wieder abdestilliert, um Nitromethanreste zu entfernen. Dann wird der Rückstand wieder in 200 ml Toluol gelöst. Ein unlöslicher Anteil wird abfiltriert und die Lösung wird auf 50 ml eingeengt. Anschließend wird die konzentrierte Lösung auf 500 g Kieselgel mit Toluol chromatographiert. Nach dem Einengen der Hauptfraktion erhält man das Endprodukt als gelbes Öl.
Ausbeute: 40,4 g = 73 % der Theorie
Rf = 0,45

Beispiel 6

2-Aminomethyl-8-methoxytetralin

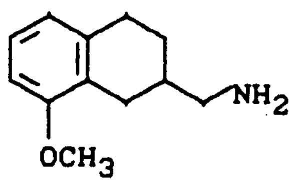

14,9 g der Verbindung aus Beispiel 5 (68 mmol) werden in 300 ml Methanol mit elementarem Wasserstoff hydriert. Als Katalysator werden 3,4 g Palladium/Aktivkohle eingesetzt. Die Temperatur wird durch leichte Kühlung auf 25-30 ° C begrenzt.

Anschließend wird der Katalysator abgesaugt und die Reaktionslösung unter Vakuum weitgehend eingeengt. Dann werden 300 ml Toluol zugesetzt und unter Vakuum wieder abdestilliert, um Methanolreste zu entfernen. Der Rückstand wird wieder in 300 ml Toluol gelöst, und die Lösung wird mit 5 %iger Kaliumcarbonatlösung und Wasser gewaschen. Die organische Phase wird über Natriumsulfat getrocknet. Dann wird das Lösungsmittel wieder unter Vakuum abdestilliert.

Das Rohrprodukt wird in 25 ml Toluol gelöst und die Lösung auf 60 g Kieselgel 60 (Fa. Merck) aufgetragen. Zuerst wird mit Toluol gewaschen und danach mit Methanol/Triethylamin 95:5 das Endprodukt eluiert. Nach Einengen der methanolischen Lösung erhält man ein hellgelbes Öl.
Ausbeute: 9,5 g = 73 % der Theorie
Rf: 0,28 (Kieselgel; Methanol/Triethylamin 95:5)

Beispiel 7

2-(N-Benzyl)-aminomethyl-8-methoxytetralin

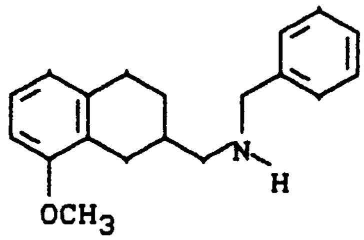

13,4 g 2-Amiomethyl-8-methoxytetralin (70 mmol) werden in 420 ml Methanol gelöst. Dann werden 3,0 ml Essigsäure (52,5 mmol) zugetropft. Nach 5 min. werden noch 5,3 g Natriumcyanoborhydrid (84 mmol) zugesetzt. Die Reaktionslösung wird auf 60 ° C erwärmt und dann wird eine Lösung auf 7,4 g Benzaldehyd (70 mmol) in 74 ml Methanol innerhalb von 30 min. zugetropft. Anschließend wird der Ansatz nach 3h unter Rückfluß gekocht.

Nach Abkühlung wird das Lösungsmittel unter Vakuum abdestilliert und der Rückstand in 420 ml Dichlormethan und 210 ml Wasser gelöst. Die organische Phase wird nochmals mit Wasser gewaschen und über Natriumsulfat getrocknet. Das Lösungsmittel wird wieder unter Vakuum abdestilliert und der Rückstand mit 700 ml Diethylether und 140 ml 5N Natronlauge verrührt. Die organische Phase wird mit Wasser neutral gewaschen und über Natriumsulfat getrocknet. Das Lösungsmittel wird unter Vakuum abdestilliert.

Der Rückstand wird auf 200 g Kieselgel 60 (Fa. Merck) chromatographiert. Als Laufmittel wird Diisopropylether verwendet. Das Endprodukt erhält man dabei als farbloses zähes Öl.
Ausbeute: 11,7 g = 59 % der Theorie
Rf = 0,25 (Kieselgel; Toluol/Methanol 95:5)

Beispiel 8

2-[N-Benzyl-N-2′-cyanoethyl]-aminomethyl-8-methoxytetralin

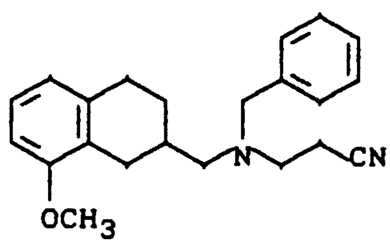

Unter Argon wurden 11,7 g der Verbindung aus Beispiel 7 (41,6 mmol) und 0,15 g Kupferacetat in 13,7 ml Acrylnitril (208 mmol) 2h unter Rückfluß gekocht. Anschließend wurde der Ansatz mit 7 ml Acrylnitril verdünnt. Nachdem sich der Ansatz langsam auf Raumtemperatur abgekühlt hatte wurde er nach 2h auf 10 ° C gekühlt. Das Kristallisat wurde abgesaugt und bei 40 ° C unter Vakuum getrocknet.
Ausbeute: 10,6 g = 76 % der Theorie
Rf = 0,77 (Kieselgel; Toluol/Methanol 95:5)
Fp: = 111-112 ° C

Beispiel 9

2-[N-Benzyl-N-3'-aminopropyl]-aminomethyl-8-methoxytetralin

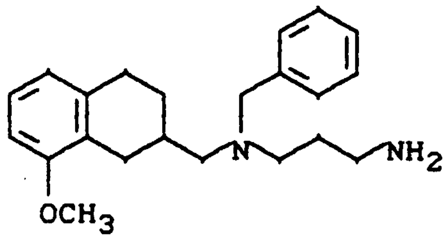

Unter Argon werden 4,8 g Lithiumaluminiumhydrid (125,6 mmol) in 315 ml absolutem Diethylether suspendiert. Dann werden innerhalb von 20 min. 10,5 g der Verbindung aus Beispiel 8 (31,4 mmol) portionsweise eingetragen. Die Temperatur wird dabei durch leichte Kühlung auf 20-25 °C begrenzt. Danach wird der Ansatz noch 3h bei Raumtemperatur gerührt.

Anschließend wird eine Mischung aus 260 ml Tetrahydrofuran und 15 ml Wasser zugetropft. Der Ansatz wird nach 30 min. gerührt und die Fällung danach abgesaugt. Die Lösung wird auf 250 ml eingeengt und in 2,5 l Wasser eingerührt. Die Mischung wird mit Toluol extrahiert und die organische Phase mit Wasser neutral gewaschen und über Natriumsulfat getrocknet. Nachdem das Lösungsmittel unter Vakuum abdestilliert wurde, verbleibt das Endprodukt als hellgelbes Öl.

Ausbeute: 10,1 g = 95 % der Theorie
Rf = 0,32 (Kieselgel; Methanol/Toluol 95:5)

Beispiel 10 und Beispiel 11

2-{N-[4-(1,1-Dioxido-3-oxo-2,3-dihydro-benzisothiazol-2-yl)butyl]aminomethyl}-8-methoxytetralin

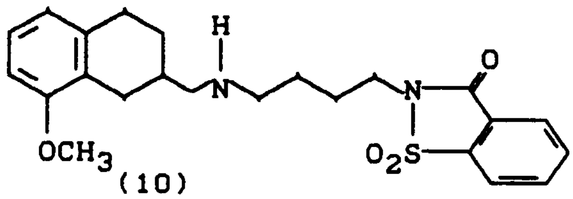

(10)

2-{N-Di[4-(1,1-Dioxido-3-oxo-2,3-dihydro-benzisothiazol-2-yl)butyl]aminomethyl}-8-methoxytetralin

(11)

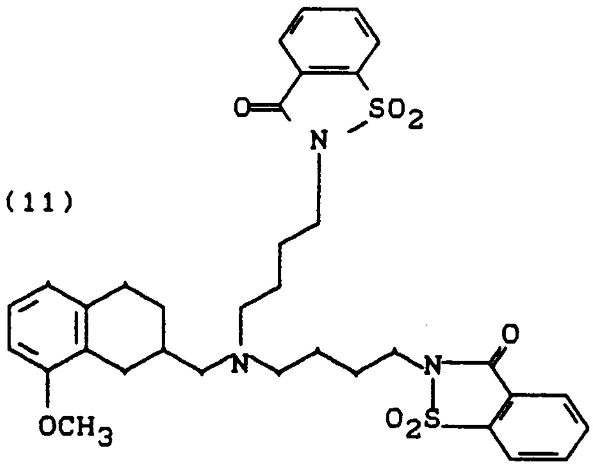

0,95 g 2-Aminomethyl-8-methoxytetralin (5 mmol), 0,70 ml Triethylamin (5 mmol) und 1,59 g 2-(4-Brombutyl)-1,2-benzisothiazol-3(2H)-on-1,1-dioxid (5 mmol) werden in 19 ml Dimethylformamid 24h bei 40 ° C gerührt. Anschließend wird die Reaktionslösung in eine Mischung aus 190 ml 5 %iger Natriumchloridlösung, 60 ml Toluol und 5 ml 1N Salzsäure eingerührt. Nachdem die organische Phase abgetrennt wurde, wird die wäßrige Phase mit dem harzig als Hydrochlorid ausgefallenem Endprodukt mit 60 ml Toluol und 7 ml Triethylamin (50 mmol) verrührt. Die organische Phase wird wieder abgetrennt, mit Wasser neutral gewaschen und über Natriumsulfat getrocknet. Das Lösungsmittel wird unter Vakuum abdestilliert. Der Rückstand wird auf Kieselgel 60 (Fa. Merck) chromatographiert. Toluol/Methanol 85:15 dient dabei als Laufmittel. Die Hauptfraktionen enthalten die beiden Endprodukte als gelbe, zähe Öle.

Beide Basen lassen sich aus toluolischer Lösung mit etherischer Chlorwasserstofflösung als Hydrochlorid fällen.

Beispiel 10

Ausbeute: 0,59 g = 28 % der Theorie
Rf: 0,30 Kieselgel; (Toluol/Methanol 85:15)

Beispiel 11

Ausbeute: 0,50 g = 15 % der Theorie
Rf: 0,73 Kieselgel; (Toluol/Methanol 85:15)

Beispiel 12

2-{N-Methyl-N[4-(1,1-Dioxido-3-oxo-2,3-dihydro-benzisothiazol-2-yl)butyl]aminomethyl}-8-methoxytetralin

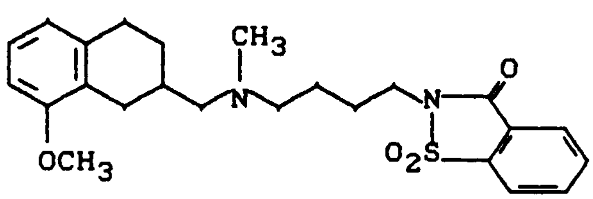

2,9 g 2-Aminomethyl-8-methoxytetralin (15 mmol) 2,1 ml Triethylamin (15 mmol) und 4,8 g 2-(4-Brombutyl)-1,2-benzisothiazol-3(2H)-on-1,1-dioxid (15 mmol) werden in 58 ml Dimethylformamid 50h bei 40 ° C gerührt. Dann wird eine Lösung aus 1,4 g Methyljodid (10 mmol) und 3,2 ml Dimethylformamid zugetropft und weitere 18h bei 40 ° C gerührt.

Anschließend wird die Reaktionslösung in eine Mischung aus 600 ml Wasser, 3,8 g Kaliumcarbonat (27,5 mmol) und 300 ml Toluol eingerührt. Die organische Phase wird mit Wasser neutral gewaschen und über Natriumsulfat getrocknet. Das Lösungsmittel wird unter Vakuum abdestilliert.

Der Rückstand wird in einem Diisopropylether-Toluol Gemisch (80:20) gelöst und durch Filtration über 20 g Kieselgel vorgereinigt. Aus dem nach Abdestillation des Lösungsmittels erhaltenen Rückstand wird durch eine Säulenchromatographie auf Kieselgel 60 (Fa. Merck) das Endprodukt isoliert. Als Laufmittel wird dabei Essigester verwendet.

Aus toluolischer Lösung läßt sich mit etherischer Chlorwassertofflösung das Hydrochlorid als Feststoff fällen.

Ausbeute: 0,85 = 12,9 % der Theorie

Rf = 0,25 (Kieselgel; Essigester oder Toluol/Methanol 95:5)

Beispiel 13 und Beispiel 14

2-{N-[4-(1,1-Dioxido-2H-naphth[1,8-cd]isothiazol-2-yl)butyl]aminomethyl}-8-methoxytetralin

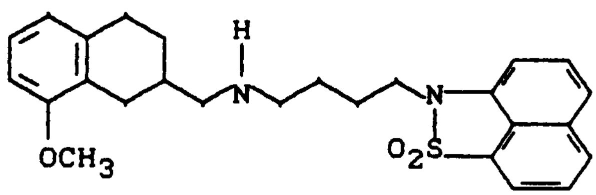

(13)

2-{N-Di[4-(1,1-Dioxido-2H-naphth[1,8-cd]isothiazol-2-yl)butyl]aminomethyl}-8-methoxytetralin

(14)

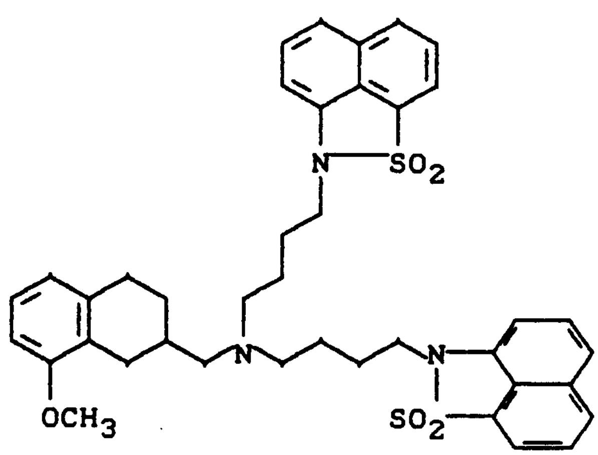

1,6 g 2-Amino-8-methoxytetralin (8,4 mmol), 1,16 ml Triethylamin (8,4 mmol) und 1,59 g 2-(4-Brombutyl)-2H-naphth[1,8-cd]isothiazol-1,1-dioxid werden in 32 ml Dimethylformamid 48h bei 40 ° C gerührt. Anschließend wird die Reaktionslösung in eine Mischung aus 320 ml 5 %iger Natriumchloridlösung, 100 ml Toluol, 8,4 ml 1N Salzsäure und Kieselgur eingerührt. Das Kieselgur mit dem darin verfestigten Endprodukt -Hydrochlorid wird abgesaugt - und in eine Mischung aus 300 ml Wasser und 100 ml Toluol eingetragen.

Durch Zutropfen von 9 ml 1N Natronlauge wird das Endprodukt in die organische Phase überführt.

Nach einer Filtration wird die organische Phase abgetrennt, mit Wasser neutral gewaschen und über Natriumsulfat getrocknet. Nachdem das Lösungsmittel unter Vakuum abdestilliert wurde, wird der Rückstand durch eine Säulenchromatographie auf Kieselgel 60 (Fa. Merck) zu den beiden Endprodukten getrennt. Als Laufmittel wird dabei Essigester verwendet.

Beide Basen lassen sich aus etherischer Lösung mit etherischer Chlorwasserstofflösung als Hydrochlorid fällen.

Beispiel 13

Ausbeute: 1,51 g = 39,8 % der Theorie
Rf = 0,28

Beispiel 14

Ausbeute: 0,63 g = 10,6 % der Theorie
Rf = 0,60

Beispiel 15

2-{N-[4-(1,1-Dioxido-3-oxo-4-phenyl-3,4-dihydro-2H-1,2,4-benzothiadiazin-2-yl)butyl]aminomethyl}8-methoxytetralin

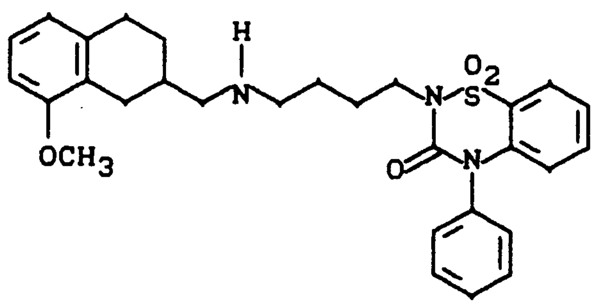

0,95 g 2-Aminomethyl-8-methoxytetralin (5 mmol), 0,70 ml Triethylamin (5 mmol) und 2,05 g 2-(4-Brombutyl)-3-oxo-4-phenyl-3,4-dihydro-2H-1,2,4-benzothiadiazin-1,1-dioxid werden in 19 ml Dimethylformamid 24h bei 40 °C gerührt. Anschließend wird die Reaktionslösung in eine Mischung aus 190 ml 5 %iger Natriumchloridlösung, 60 ml Toluol und 5 ml 1N Salzsäure eingerührt. Nachdem die organische Phase abgetrennt wurde, wird die wäßrige Phase mit dem harzig als Hydrochlorid ausgefallenen Endprodukt mit 60 ml Toluol und 7 ml Triethylamin (50 mmol) verrührt. Die organische Phase wird wieder abgetrennt, mit Wasser neutral gewaschen und über Natriumsulfat getrocknet. Das Lösungsmittel wird unter Vakuum abdestilliert. Der Rückstand wird auf Kieselgel 60 (Fa. Merck) chromatographiert. Toluol/Methanol 85:15 dient dabei als Laufmittel.

Aus toluolischer Lösung läßt sich mit etherischer Chlorwasserstofflösung das Hydrochlorid als Feststoff fällen.

Ausbeute: 0,83 g = 31,9 % der Theorie
Rf = 0,28 (Kieselgel; Toluol/Methanol 85:15).
Fp: 148 °C

Beispiel 16

2-{N-Benzyl-N-[3-(4-fluorphenylsulfonylamido)propyl]aminomethyl}8-methoxytetralin

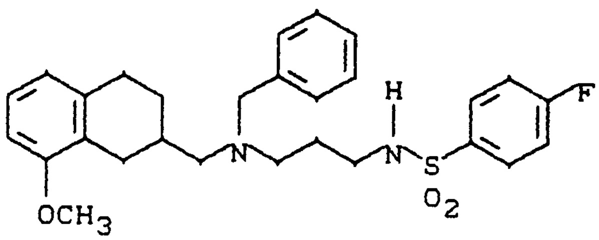

0,95 g (2,8 mmol) der Verbindung aus Beispiel 9 werden in 19 ml Dichlormethan gelöst. Nach Zusatz von 0,39 g (2,8 mmol) fein gemahlenem Kaliumcarbonat wird eine Lösung aus 0,60 g 4-Fluorbenzolsulfonsäurechlorid und 9,5 ml Dichlormethan bei 15-20 °C zugetropft. Anschließend wird die Reaktionslösung noch 18h bei Raumtemperatur gerührt.

Danach werden 15 ml Wasser zugesetzt. Die organische Phase wird mit Wasser neutral gewaschen, über Natriumsulfat getrocknet und unter Vakuum eingeengt. Der Rückstand wird in 50 ml Diisopropylether gelöst, und die Lösung filtriert. Anschließend wird die Lösung wieder auf ca. 10 ml eingeengt und auf 25 g Kieselgel chromatographiert. Als Laufmittel wird Diisopropylether benutzt. Das Endprodukt erhält man als hellbraunes zähes Öl.

Ausbeute: 1,08 g = 78 % der Theorie
Rf = 0,58 (Kieselgel; Toluol/Methanol 95:5)
Rf = 0,23 (Kieselgel; Diisopropylether)

Beispiel 17

2-{N-[3-(4-fluorphenylsulfonylamido)propyl]aminomethyl}8-methoxytetralin

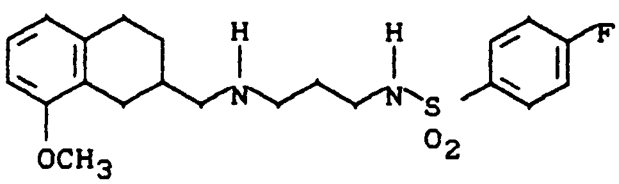

1,0 g der Verbindung aus Beispiel 16 (2 mmol) werden in 50 ml Methanol und 2,4 ml methanolischer Chlorwasserstofflösung (0,9 mmol/ml) mit elementarem Wasserstoff hydriert. Als Katalysator werden 0,1 g Palladium/Aktivkohle eingesetzt.

Anschließend wird der Katalysator abfiltriert und die Lösung unter Vakuum auf 10 ml eingeengt. Dann wird die Lösung in eine Mischung aus 100 ml Wasser, 50 ml Dichlormethan und 2,5 ml 1N Natronlauge eingerührt. Die organische Phase wird mit Wasser neutral gewaschen, über Natriumsulfat getrocknet und unter Vakuum eingeengt.

Der Rückstand wird auf 20 g Kieselgel aufgetragen, zuerst mit Diisopropylether Ausgangsproduktreste, dann mit Methanol das Endprodukt eluiert. Nachdem das Methanol abdestilliert wurde, wird der Rückstand durch verrühren mit Diisopropylether kristallin. Das weiße Kristallisat wird abgesaugt und bei 50 °C im Vakuum getrocknet.

Ausbeute: 0,47 g = 58 % der Theorie
Rf = 0,25 (Kieselgel; Toluol/Methanol 85:15)
Fp: 111 °C/112-114 °C

Beispiel 18

2-{N-Benzyl-N-[3-(ethoxycarbonylamido)propyl]aminomethyl}8-methoxytetralin

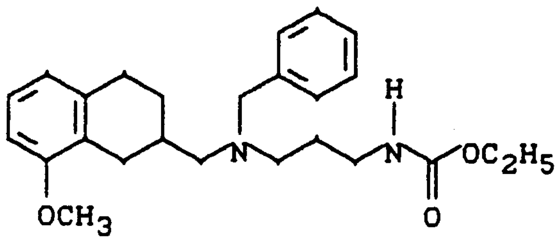

Die Verbindung wird analog der Arbeitsvorschrift für die Verbindung 16 aus 1,80 g der Verbindung aus Beispiel 9 (5,3 mmol) und 0,55 ml Chlorameisensäureethylester (5,8 mmol) als zähes Öl erhalten.
Ausbeute: 2,00 g = 92 % der Theorie
Rf = 0,50 (Kieselgel; Toluol/Methanol 95:5)
Rf = 0,30 (Kieselgel; Diisopropylether)

Beispiel 19

2-{N-[3-(ethoxycarbonylamido)propyl]aminomethyl}8-methoxytetralin

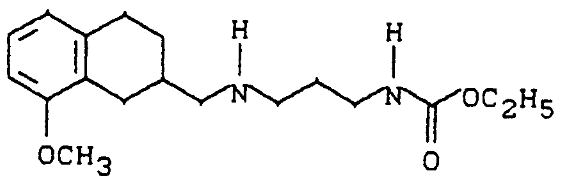

x HCl

Die Verbindung wird analog der Arbeitsvorschrift für Verbindung 17 aus 1,80 g der Verbindung aus Beispiel 18, 90 ml Methanol, 7,3 ml methanolischer Chlorwasserstofflösung und 0,22 g Palladium/Aktivkohle nach Hydrierung als zähes Öl erhalten.

Die Base wurde in Ether gelöst und ein unlöslicher Anteil abfiltriert. Mit etherischer Chlorwasserstofflösung wird aus der Lösung das Hydrochlorid gefällt. Der weiße Farbstoff wird abgesaugt und bei 40 °C im Vakuum getrocknet.
Ausbeute: 0,60 g = 38 % der Theorie
Rf = 0,40 (Kieselgel; Toluol/Methanol 70:30)
Rf = 0,20 (Base)
Fp: 188 °/191-193 °C

Beispiel 20

2-{N-[4-(1,1-Dioxido-3-oxo-2,3-dihydro-benzisothiazol-2-yl)butyl-N-propyl]aminomethyl}-chroman

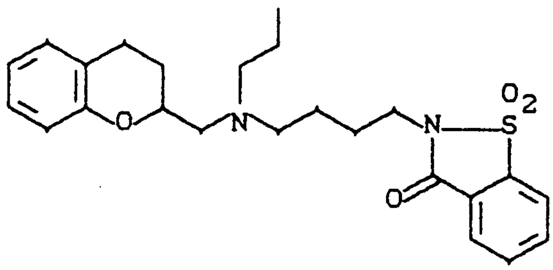

Die Mischung von 4,90 g (24 mmol) 3,4-Dihydro-2-(N-propyl)aminomethyl-2H-chromen, 8,60 g (27 mmol) 2-(4-Brombutyl)-1,2-benzisothiazol-3(2H)-on-1,1-dioxid, 6,6 g (48 mmol) gepulvertem Kaliumcarbonat und 0,1 g Natriumjodid in 100 ml wasserfreiem Dimethylformamid wurde 12h bei 60-70 °C gerührt.

Nach Filtration und Einengen bei ca. 2 Torr wurde durch Flashchromatographie (Toluol-Essigester-Gradient; Kieselgel) gereinigt. Auf diese Weise wurden 4,5 g (44 %) Produkt als viskoses Öl gewonnen.

Rf: (Toluol/Methanol 4:1) 0,47

IR ($CHCl_3$): 3011, 2958, 2874, 1733, 1598, 1584, 1489.

Das Hydrochlorid wurde durch Behandlung mit etherischer Salzsäure in Ether als farbloser, amorpher Feststoff gewonnen.

$^1H$-NMR ($CD_3OD$): 1,0 (t; 3H), 1,6-2,2 (m; 8H), 2,7-3,0 (m; 2H), 5,2-3,6 (m; darunter Lösungsmittel-Signal, $CHD_2OD$), 3,9 (m; 2H), 4,5 (m; 1H), 6,75-7,1 (m; 4H), 7,8-8,2 (m; 4H), 7,9 (s; -OH, -NH-)

Beispiel 21

3-{N-[4-(1,1-Dioxido-3-oxo-2,3-dihydro-benzisothiazol-2-yl)butyl]amiomethyl}-chroman

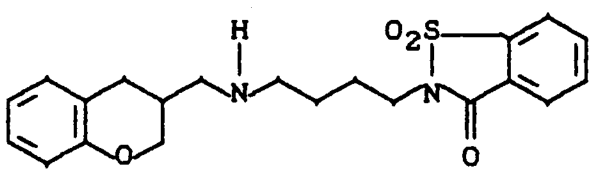

Darstellung erfolgte analog der Vorschrift von Beispiel 20 aus 3-Aminomethylchroman.

Rf: (Toluol/Methanol 4:1): 0,33.

Das Hydrochlorid erhält man durch Ausfällung mit etherischer Salzsäure.

Amorpher, hygroskopischer Feststoff.

$^1H$-NHR (Hydrochlorid; $CD_3OD$): 1,8-2,0 (m; NH), 2,4 (m; 1H), 2,65 ("dd"; 1H) 2,9-3,2 (m; 5H), 3,75 (t; 2H), 4,0 (m; 1H), 4,25 (m; 1H), 6,7-7,1 (m; 4H), 7,9-8,1 (m; 4H)

Beispiel 22

3-{N,N-Di[1,1-dioxido-3-oxo-2,3-dihydro-benzisothiazol-2-yl)butyl]aminomethyl}-chroman

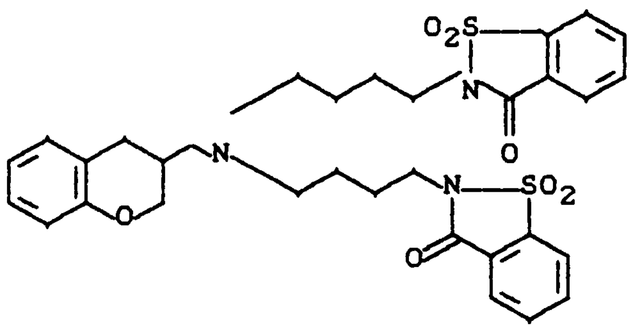

Neben dem Beispiel 21 bildete sich bei der Umsetzung von 3-Aminomethyl-chroman mit 4-Brombutylsaccharin die Verbindung 22.
Rf: (Toluol/Essigester 1:1): 0,58
Das Hydrochlorid wurde durch Behandeln mit etherischer Salzsäure als amorpher Feststoff gewonnen:
$^1$H-NMR (Hydrochlorid, $CD_3OD$): 1,8-2,0 (m; 8H), 2,5-2,8 (m; 2H), 2,9-3,4 (m; enthält Signale des LM), 3,75 (m; 4H), 4,0 (m; 1H), 4,25 (m; 1H), 6,7-7,1 (m; 4H), 7,9-8,1 (m; 8H) sowie Signale von ca. 8 % Diethylether.

Beispiel 23

2-[N-2-(4-Fluorphenylsulfonamido)ethyl]carbonamido-2H-chroman

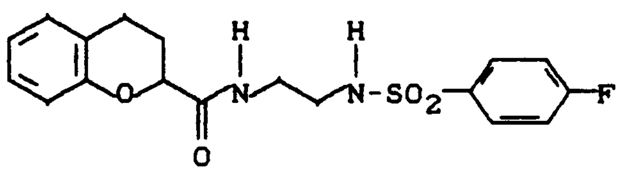

Die Umsetzung von 2-Chromancarbonsäurechlorid und 2-(4-Fluorphenylsulfonamido)ethylamin in Gegenwart von Natriumhydrogencarbonat in Ether/Dioxan ergibt Beispiel 23.
Rf: (Toluol/Essigester 1:1): 0,35

Beispiel 24

2-[N-(4-Fluorphenylsulfonylamido)ethyl]aminomethylchroman-hydrochlorid

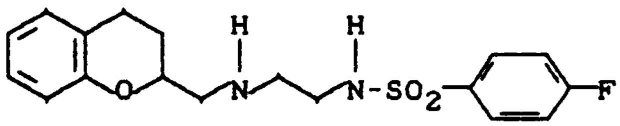

x HCl

Die Lösung von 4,50 g (11,9 mmol) der Verbindung aus Beispiel 23 in 100 ml absolutem THF wurden bei 0 ° C zu 16,6 ml (16,6 mmol) einer 1M Lösung von $BH_3$ in THF gegeben.

Dann wurden 50 ml THF zugetropft. Nach 2h Rühren bei Raumtempertur wurde auf 0 ° abgekühlt und mit 2 ml konzentrierter Salzsäure versetzt.

Der Niederschlag wurde nach 30 min abgesaugt und mit THF und n-Hexan gewaschen. Auf diese Weise erhielt man 3,35 g (70 %) Produkt als farblose Kristalle.
Schmelzpunkt: 212-214 ° C
MS: 364, 231, 176 (100 %), 95

1H-NMR ($CD_3OD$): 1,75 (m; 1H), 2,1 (m; 1H), 2,7-3,0 (m; 2H), 3,1-3,5 (m; enthält Signal des Lösemittels), 4,4 (m; 1H), 6,8 (m; 2H), 7,1 (m; 2H), 7,2-7,4 (m; 2H), 8,0 (m, 2H).

Beispiel 25

2-Aminomethyl-7,8,9,10-tetrahydro-benzo(h)chroman-hydrochlorid

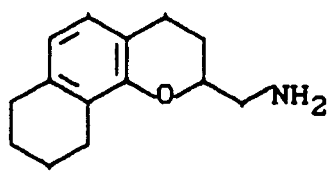

x HCl

Die Darstellung erfolgt aus dem entsprechenden Carbonamid mit Boran-THF-Komplexe analog der Arbeitsvorschrift von Beispiel 24.

$^1$H-NMR ($CD_3OD$): 1,4-1,9 (m; 5H), 2,0-2,1 (m; 1H), 2,6-3,0 (m; 6H), 3,1-3,4 (m; mehrere H; auch $CD_2HOD$), 4,2-4,3 (m; 1H), 4,9 (s; $H_2O$, $-NH_2$), 6,6 (d; 1H), 6,8 (d; 1H).

Mit Natriumhydrogencarbonat/Essigesterextraktion wurde die freie Base erhalten.
Rf: (Dichlormethan/Methanol 10:1): 0,27

Beispiel 26

2-{N-[4-(1,1-Dioxido-3-oxo-2,4-dihydro-benzisothiazol-2-yl)-butyl]aminomethyl}-7,8,9,10-tetrahydro-benzo-(H)chroman

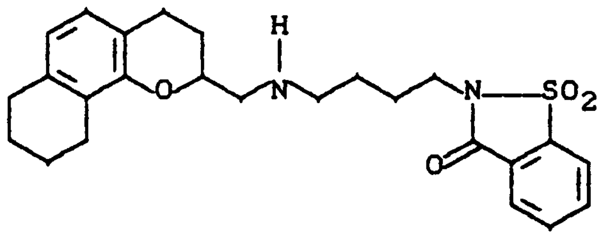

Die Verbindung aus Beispiel 25 wurde analog nach der Arbeitsweise aus Beispiel 20 dargestellt.
MS: 454, 424, 267, 200
Rf: ($CH_2Cl_2/CH_3OH$ 10:1), 0,58
Das entsprechende Hydrochlorid ist amorph.
$^1$H-NMR ($CD_3OD$): 1,6-2,1 (m; 10H), 2,5-3,0 (m; 5H), 3,1-3,4 (m; mehrere H, darunter $CD_2HOD$), 3,9 (t; 2H), 4,3 (m; 1H), 4,9 (s; $H_2O$, NH), 6,6 (d; 1H), 6,75 (d; 1H), 7,9-8,1 (m; 4H).

Beispiel 27

2-(N,N-{di[4-(1,1-dioxido-3-oxo-2,3-dihydro-benzisothiazol-2-yl)butyl}aminomethyl)-7,8,9,10-tetrahydro-benzo(h)chroman

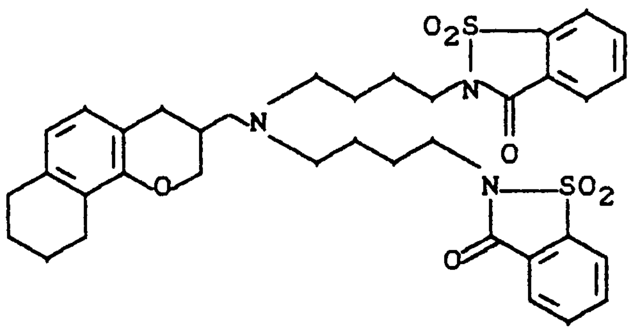

Die Verbindung entsteht bei der Darstellung des Beispiels 26.
Rf: ($CH_2Cl_2/CH_3OH$ 10:1) 0,86
Das amorphe Hydrochlorid der Verbindung wurde mit etherischer Salzsäure in Ether gefällt.
$^1$H-NMR ($CD_3OD$): 1,4-2,1 (m; 14H), 2,5-3,0 (m; 7H), 3,2-3,6 (m; mehrere H; darunter $CD_2HOD$), 3,9 (m; 4H), 4,5 (m; 1H),, 4,9 (s; $H_2O$, NH), 6,5 (d; 1H), 6,75 (d; 1H), 7,9-8,1 (m; 8H).

Beispiel 28

2-{N-[4-(1,1-Dioxido-3-oxo-2,3-dihydro-benzisothiazol-2-yl)butyl]aminomethyl}chroman

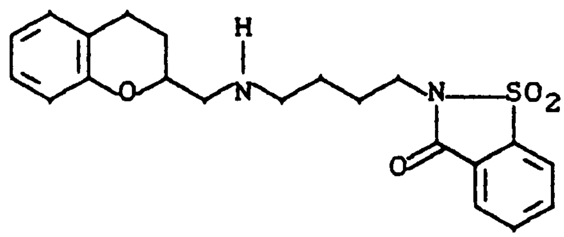

Die Verbindung wird analog der Arbeitsvorschrift von Beispiel 20 aus 2-Aminomethyl-3,4-dihydro-2H-chromen dargestellt.

Das entsprechende Hydrochlorid hat einen Schmelzpunkt von 188 ° C-195 ° C.
Rf: (Toluol/Essigester 1:1) = 0,37

Beispiel 29

2-{N-[4-(1,1-Dioxido-3-oxo-2,3-dihydro-benzisothiazol-2-yl)butyl]aminomethyl}-8-methoxy-chroman

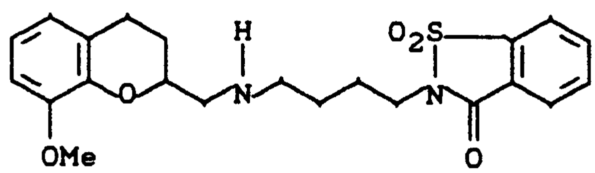

Die Verbindung wird analog der Arbeitsvorschrift von Beispiel 20 aus 2-Aminomethyl-3,4-dihydro-8-methoxy-2H-chromen dargestellt.
Rf: (Toluol/Methanol 4:1): 0,33
Das entsprechende Hydrochlorid hat einen Schmelzpunkt von 173 ° -178 ° C.

Beispiel 30

2-{N-[4-(1,1-Dioxido-3-oxo-2,3-dihydro-benzisothiazol-2-yl)butyl]aminomethyl}-5-methoxy-chroman

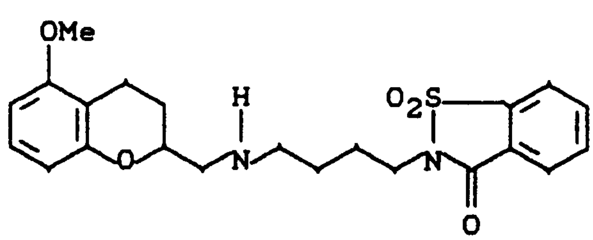

Die Verbindung wird analog der Arbeitsvorschrift von Beispiel 20 aus 2-Aminomethyl-3,4-dihydro-5-methoxy-2H-chromen dargestellt.
Rf: (Toluol/Methanol 4:1) 0,3
Das Hydrochlorid hat einen Schmelzpunkt von 253-257 °C.

Beispiel 31

2-{N-[4-(1,1-Dioxido-3-oxo-2,3-dihydro-benzisothiazol-2-yl)butyl-N-propyl]aminomethyl}-5-methoxy-chroman

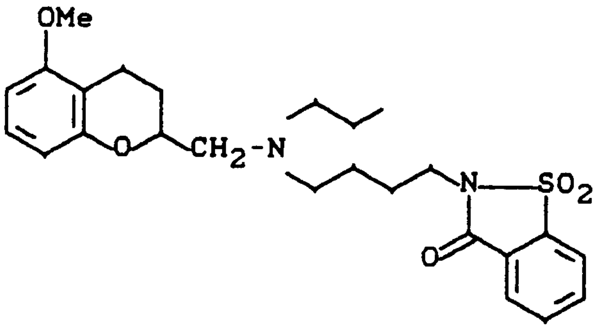

Die Verbindung wird analog der Arbeitsvorschrift von Beispiel 20 aus 3,4-Dihydro-2-(N-propyl)aminomethyl-5-methoxy-2H-chromen dargestellt.
Rf: (Toluol/Methanol 4:1), 0,33
MS: 472, 309, 196
Das entsprechende Hydrochlorid zeigt folgende Verschiebungen auf:
$^1$H-NMR ($CD_3OD$): 1,0 (t; 3H), 1,6-2,2 (m; 8H), 2,5-2,9 (m; 2H), 3,1-3,5 (m; enthält Signal von $CD_2HOD$), 3,7 (s; 3H), 3,9 (t; 2H), 4,4 (m; 1H), 4,9 (s; $H_2O$, -NH), 6,5 (m; 2H), 7,0 (m; 1H), 7,8-8,1 (m; 4H).

Beispiel 32

2-{N-[4(1,1-Dioxido-3-oxo-2,3-dihydro-benzisothiazol-2-yl)butyl]aminomethyl}benzo(h)chroman

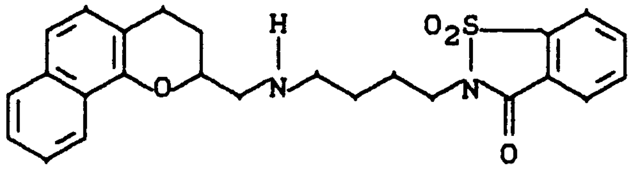

Die Verbindung wird analog der Arbeitsvorschrift des Beispiels 20 aus 2-Aminomethyl-3,4-dihydro-2H-benzo(h)chromen-hydrochlorid dargestellt.
Fp: 97-102 °C

Beispiel 33

2-{N-[4-(1,1-Dioxido-2H-naphth[1,8-cd]isothiazol-2-yl)butyl]aminomethyl}-chroman

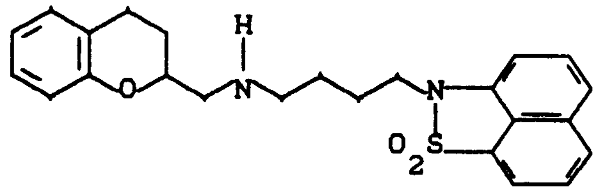

Die Verbindung wird nach der Arbeitsvorschrift des Beispiels 20 aus 2-Aminomethyl-3,4-dihydro-2H-chromen und 2-(4-Brombutyl)-2H-naphth[1,8-cd]isothiazol-1,1-dioxid (zugänglich aus 2H-Naphth[1,8-cd]-isothiazol-1,1-dioxid und 1,4-Dibrombutan mit Base) hergestellt.
Rf: (Toluol/Essigester 1:1) = 0,41
Das entsprechende Hydrochlorid ist amorph.
$^1$H-NMR ($CD_3OD$): 1,7 (m; 1H), 2,0 (m; 5H), 2,6-2,9 (m; 2H), 3,2-3,5 (m; darunter Signale des Lösemittels), 3,9 (m; 2H), 4,5 (m; 1H), 6,65 (dd; 1H), 6,75 (ddd; 1H), 6,9-7,1 (m; 2H), 7,5-7,6 (m; 2H), 7,8 (dd; 1H), 8,05 (d; 1H), 8,2 (d; 1H).

Beispiel 34

2-{N-Benzyl-N-[3-(N-benzyl-4-fluorphenyl-sulfonamido)propyl]aminomethyl}8-methoxytetralin

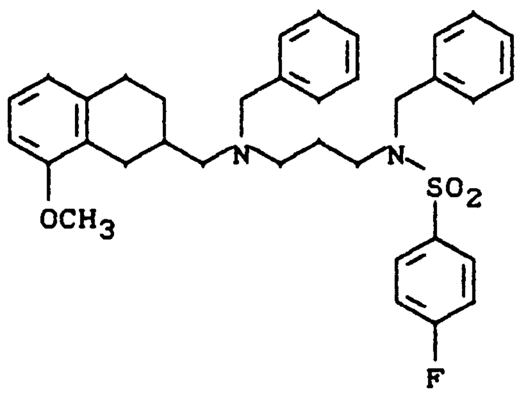

79 mg Natriumhydrid (3,3 mmol) werden unter Argon in 15 ml trockenem Dimethylformamid suspendiert. Anschließend wird innerhalb von 30 min eine Lösung von 1,49 g der Verbindung aus Beispiel 16 (3 mmol) in 15 ml trockenem Dimethylformamid bei 20-25 °C zugetropft. Danach wird der Ansatz noch 1h auf 30 °C erwärmt. Dann wird innerhalb von 15 min eine Lösung von 0,56 g Benzylbromid (3 mmol) in 15 ml trockenem Dimethylformamid zugetropft. Zur Vervollständigung der Reaktion wird der Ansatz noch 18h bei Raumtemperatur gerührt.

Zur Aufarbeitung wird der Ansatz in einer Mischung aus 450 ml 5 %iger Natriumchloridlösung und 150 ml Toluol eingerührt. Die organische Phase wird mit Wasser neutral gewaschen, über Natriumsulfat getrocknet und das Lösungsmittel abdestilliert.

Das Rohprodukt wird auf 90 g Kieselgel 60 mit Diisopropylether chromatographiert. Man erhält ein farbloses Harz.
Ausbeute: 1,26 g = 76 % der Theorie
Rf = 0,52 (Kieselgel, Diisopropylether)

Beispiel 35

2-{N-[3-(N-Benzyl-4-fluorphenylsulfonamido)propyl]aminomethyl}8-methoxytetralin

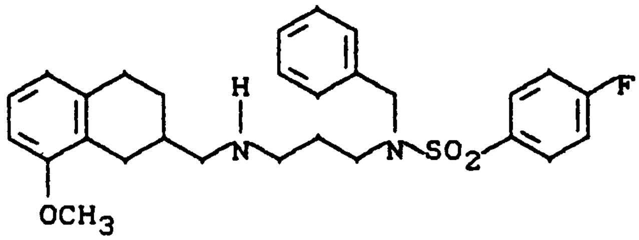

Die Verbindung wird analog der Arbeitsvorschrift für Beispiel 17 aus 1,15 g der Verbindung aus Beispiel 34 (2,1 mmol), 55 ml Methanol, 5,9 ml methanolischer Chlorwasserstofflösung (3,2 mmol) und 0,11 g Palladium/Aktivkohle nach Hydrierung als zähes Öl erhalten.

Die Base kristallisiert hier nicht aus Diisopropylether und wurde daher durch Zutropfen von etherischer Chlorwasserstofflösung als Hydrochlorid ausgefällt. Die Fällung wurde abgesaugt und bei 50 °C/0,1 mb getrocknet. Man erhält einen weißen Feststoff.
Ausbeute: 0,70 g = 70 % der Theorie
Fp: 130 °C/157-158 °C
Rf = 0,40 (Kieselgel; Toluol/Methanol 85:15)

Beispiel 36

2-{N-Benzyl-N-[3-(N-methyl-4-fluorphenylsulfonamido)propyl]aminomethyl}8-methoxytetralin

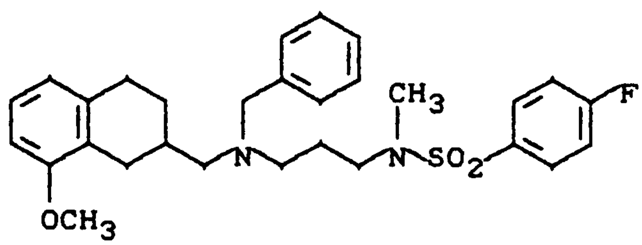

Die Verbindung wird analog der Arbeitsvorschrift für Beispiel 34 aus 3,97 g der Verbindung aus Beispiel 16 (8 mmol), 211 mg Natriumhydrid (8,8 mmol) und 1,14 g Methyljodid (8 mmol) in trockenem Dimethylformamid hergestellt.

Das Rohprodukt wird auf 250 g Kieselgel 60 mit Diisopropylether chromatographiert. Man erhält ein farbloses Harz.
Ausbeute: 4,03 g = 99 % der Theorie
Rf = 0,47 (Kieselgel, Diisopropylether)

Beispiel 37

2-{N-[2-(2-carbonamido-N-(8-methoxytetralin-2-yl-methyl)phenylsulfonamido)ethyl]aminomethyl}8-methoxytetralin

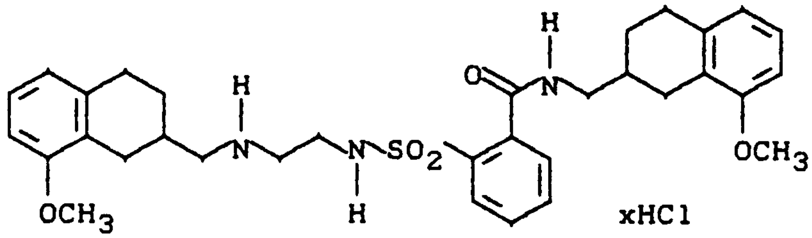

1,90 g der Verbindung aus Beispiel 6 (10 mmol), 1,00 g Triethylamin (10 mmol) und 2,90 g 2-(2-Brombutyl)-1,2-benzisothiazol-3(2H)-on-1,1-dioxid werden in 38 ml Dimethylformamid 24h bei 40 °C gerührt.

Zur Aufarbeitung wird der Ansatz in eine Mischung aus 380 ml 5 %iger Natriumchloridlösung, 130 ml Toluol und 10 ml 1n Salzsäure eingerührt, wobei das Endprodukt als Hydrochlorid harzig ausfällt. Die flüssigen Phasen werden abgegossen und das Harz mit 400 ml 5 %iger Natriumchloridlösung und 130 ml Toluol versetzt. Dann wird unter Rühren vorsichtig 1n Natronlauge zugetropft, wobei der pH-Wert bei max. 11 gehalten wird. Die organische Phase wird dann mit Wasser neutral gewaschen und über Natriumsulfat getrocknet. Dann wird das Lösungsmittel bei 30 °C und 40 mb abdestilliert.

Das erhaltene Rohprodukt wird auf 90 g Kieselgel 60 mit Essigester chromatographiert. Man erhält ein gelbes Harz.

Die Base läßt sich aus toluolische Lösung durch Zutropfen von etherischer Chlorwasserstofflösung als Hydrochlorid fällen.

Ausbeute: 1,51 g = 51 % der Theorie
Rf = 0,32 (Kieselgel; Toluol/Methanol 85,15)
Rf = 0,15 (Essigester)

Beispiel 38

2-{N-[3-(N-Methyl-4-fluorphenylsulfonamido)propyl]aminomethyl}8-methoxytetralin

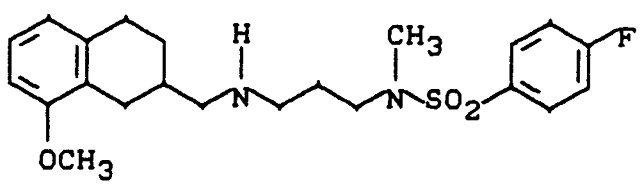

Die Verbindung wird analog der Arbeitsvorschrift für Beispiel 17 aus 3,90 g der Verbindung aus Beispiel 36 (7,6 mmol), 195 ml Methanol, 21 ml methanolischer Chlorwasserstofflösung (11,4 mmol) und 0,38 g Palladium/Aktivkohle nach Hydrierung als zähes Öl erhalten.

Die Base kristallisiert aus Diisopropylether. Man erhält einen weißen Feststoff.

Ausbeute: 2,15 g = 67 % der Theorie; Fp.: 66-67 °C
Rf = 0,23 (Kieselgel; Toluol/Methanol 85:15)

Beispiel 39

2-{N-Benzyl-N-[3-(phenylcarbonamido)propyl]aminomethyl}8-methoxytetralin

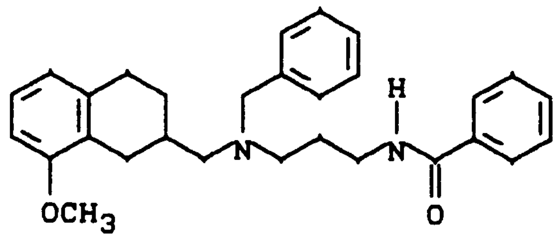

Die Verbindung wurde analog der Arbeitsvorschrift für Beispiel 16 aus 3,39 g der Verbindung aus Beispiel 9 g (10 mmol), 1,38 g Kaliumcarbonat (10 mmol) und 1,41 g Benzoylchlorid (10 mmol) in Dichlormethan hergestellt.

Das Rohprodukt wurde auf 90 g Kieselgel 60 mit Diisopropylether (zum Auftragen in wenig Toluol gelöst) chromatographiert. Das reine Produkt kristallisiert danach aus der Hauptfraktion aus. Das Kristallisat wurde abgesaugt und bei 50 ° C/1 mb getrocknet. Man erhält ein weißes Kristallisat.
Ausbeute: 3,6 g = 82 % der Theorie
Fp: 95,5-96,5 ° C
Rf = 0,10 (Kieselgel; Toluol/Methanol 85:15)
Rf = 0,10 (Diisopropylether)

Beispiel 40

2-{N-Benzyl-N-[3-(N-benzyl-phenylcarbonamido)propyl]aminomethyl}8-methoxytetralin

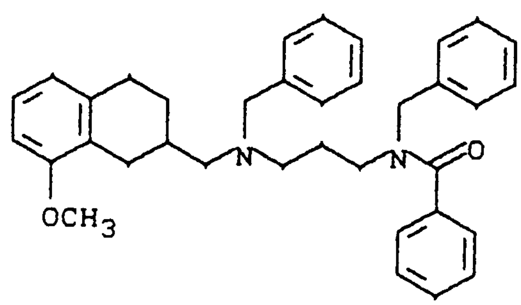

Die Verbindung wird analog der Arbeitsvorschrift für Beispiel 34 aus 1,77 g der Verbindung aus Beispiel 39 (4 mmol), 106 mg Natriumhydrid (4,4 mmol) und 0,68 g Benzylbromid (4 mmol) in trockenem Dimethylformamid hergestellt.

Das Rohprodukt wird auf 90 g Kieselgel 60 mit Diisopropylether chromatographiert. Man erhält ein farbloses Harz.
Ausbeute: 1,73 g = 81 % der Theorie
Rf = 0,28 (Kieselgel; Diisopropylether)

Beispiel 41

2-{N-Benzyl-N-[3-(N-methyl-phenylcarbonamido)propyl]aminomethyl}8-methoxytetralin

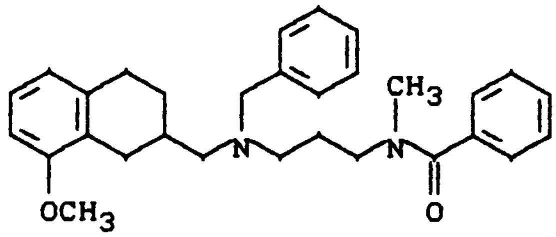

Die Verbindung wird analog der Arbeitsvorschrift für Beispiel 34 aus 1,77 g der Verbindung aus Beispiel 39 (4 mmol), 106 ml Natriumhydrid (4,4 mmol) und 0,57 g Methyljodid (4 mmol) in trockenem Dimethylformamid hergestellt.

Das Rohprodukt wird auf 250 g Kieselgel 60 mit Diisopropylether/Methanol 95:5 chromatographiert. Man erhält ein farbloses Harz.
Ausbeute: 1,62 g = 89 % der Theorie
Rf = 0,52 (Kieselgel; Toluol/Methanol 90:10)

Beispiel 42

2-{N-[3-(N-Benzyl-phenylcarbonamido)propyl]aminomethyl}8-methoxytetralin

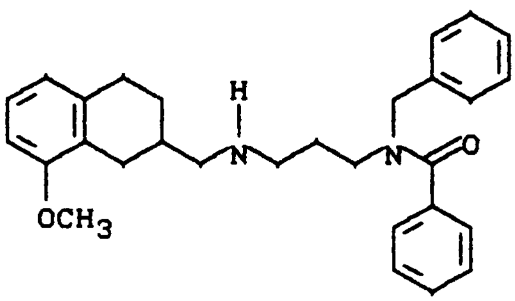

Die Verbindung wird analog der Arbeitsvorschrift für Beispiel 17 aus 1,68 g der Verbindung aus Beispiel 40 (3,2 mmol), 84 ml Methanol, 8,9 ml methanolischer Chlorwasserstofflösung (4,8 mmol) und 0,16 g Palladium/Aktivkohle nach Hydrierung als zähes Öl erhalten.

Die Base wurde ins Hydrochlorid überführt.

Man erhält einen weißen Feststoff
Ausbeute: 1,18 g = 76 % der Theorie
Rf = 0,32 (Kieselgel; Toluol/Methanol 85:15)

Beispiel 43

2-{N-[3-(N-methyl-phenylcarbonamido)propyl]aminomethyl}8-methoxytetralin

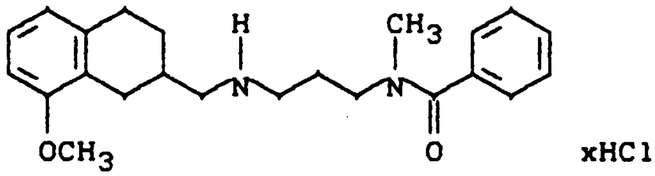

Die Verbindung wird analog der Arbeitsvorschrift für Beispiel 17 aus 1,46 g der Verbindung aus Beispiel 41 (3,2 mmol), 73 ml Methanol, 8,9 ml methanolischer Chlorwasserstofflösung (4,8 mmol) und 0,16 g Palladium/Aktivkohle nach Hydrierung als zähes Öl erhalten.

Die Base wurde analog der Arbeitsvorschrift von Beispiel 19 ins Hydrochlorid überführt.

Man erhält einen weißen Feststoff

Fp.: 128-130 °C

Rf = 0,38 (Kieselgel; Toluol/Methanol 70:30)

Rf = 0,28 (Base)

Beispiel 44

2-{N-[3-(Phenylcarbonamido)propyl]aminomethyl}8-methoxytetralin

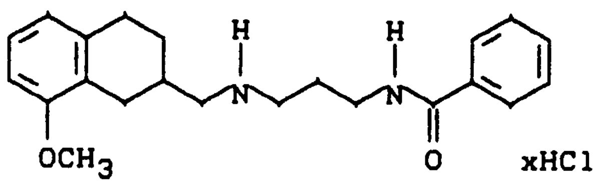

Die Verbindung wird analog der Arbeitsvorschrift für Beispiel 17 aus 1,20 g der Verbindung aus Beisspiel 39 (2,7 mmol), 60 ml Methanol, 7,6 ml methanolischer Chlorwasserstofflösung (4,1 mmol) und 0,14 g Palladium/Aktivkohle nach Hydrierung als zähes Öl erhalten.

Die Base wurde analog der Arbeitsvorschrift von Beispiel 19 ins Hydrochlorid überführt.

Man erhält einen weißen Feststoff.

Fp.: 134,5-137 °C

Rf = 0,35 (Kieselgel; Toluol/Methanol 70:30)

Rf = 0,22 (Base)

Beispiel 45

2-{N-Methyl-N-[3-(N-methyl-4-fluorphenylsulfonamido)propyl]aminomethyl}-8-methoxytetralin

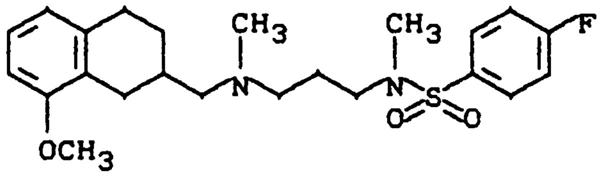

Die Verbindung wird analog der Arbeitsvorschrift für Beispiel 7 aus 1,05 g der Verbindung aus Beispiel 38 (2,5 mmol), 0,12 g Essigsäure (1,88 mmol), 0,19 g Natriumcyanborhydrid (3,0 mmol), 33 ml Methanol und einer Lösung von 0,21 g 37 %iger wäßriger Formaldehydlösung (2,5 mmol) in 5 ml Methanol hergestellt.

Die Base erhält man als farbloses zähes Öl.

Ausbeute: 1,03 g = 95 % der Theorie

Rf = 0,47 (Kieselgel, Toluol/Methanol 85:15).

Beispiel 46

2-{N-[4-(1,1-dioxido-3-oxo-2,3-dihydro-benzisothiazol-2-yl)butyl]aminomethyl}chinolin

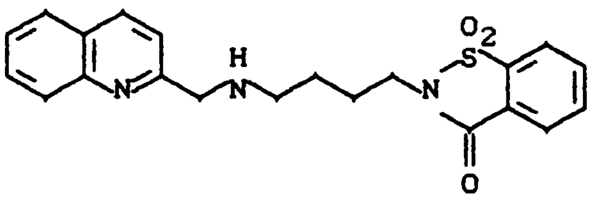

Zu einer Mischung von 4,9 g (31 mmol) 2-Aminomethylchinolin und 2,8 g (28 mmol) Triethylamin in 100 ml wasserfreiem Dimethylformamid tropft man die Lösung von 8,91 g (28 mmol) 2-(4-Brombutyl)-1,2-benzisothiazol-3(2H)-on-1,1-dioxid in 30 ml wasserfreiem Dimethylformamid und rührt 14 h bei 40 °C. Anschließend gießt man den Ansatz in 600 ml Wasser, extrahiert mit Essigester, wäscht die organische Phase mit Wasser und trocknet über Natriumsulfat. Nach dem Filtrieren und Abdampfen des Lösungsmittels erhält man 8 g eines braunen, viskosen Öls, das durch Säulenchromatographie an Kieselgel (Laufmittel: Methylenchlorid/Methanol/konz. Ammoniak-Lösung (10:0,1:0,1)) gereinigt wird.

Ausbeute: 1,75 g = 16 % der Theorie, gelbes Öl.

Rf = 0,32 (Kieselgel; Methylenchlorid/ Methanol 100:5).

$^1$H-NMR ($CD_2Cl_2$): 1,65-1,72 (m; -$\underline{CH_2}$-$CH_2$-; 2H), 1,9-2,0 (m; -$\underline{CH_2}$-; 2H), 2,3-2,6 (m; NH), 2,8 (t; NH-$\underline{CH_2}$-; 2H), 3,82 (t; -CON-$\underline{CH_2}$;2H), 4,09 (s; NH-$\underline{CH_2}$-ar; 2H), 7,45-8,15 (m; Ar; 10H).

Die Base läßt sich aus methanolischer Lösung durch Zutropfen äquimolarer Mengen methanolischer Naphthalin-1,5-disulfonsäure-Lösung als Naphthalin-1,5-disulfonsaures Salz fällen. Fp: 224 °C.

Beispiel 47:

2-{N-[4-(1,1-Dioxido-2,3-dihydro-benzisothiazol-2-yl)butyl] aminomethyl}-8-methoxy-tetralin

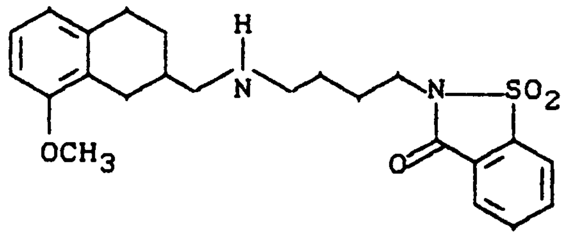

2 g 2-Aminomethyl-8-methoxytetralin (10,5 mmol), 3,2 g 2-(4-Brombutyl)-1,2-benzisothiazol-(2H)-1,1-dioxid (10,5 mmol) und 1,46 ml Triethylamin (10,5 mmol) werden in 40 ml Dimethylformamid 24 h bei 40 °C gerührt. Anschließend wird die Reaktionslösung in eine Mischung aus 440 ml Wasser und 140 ml Toluol eingerührt. Die organische Phase wird mit Wasser gewaschen und über Natriumsulfat getrocknet. Das Lösungsmittel wird unter Vakuum abdestilliert. Der Rückstand wird durch Säulenchromatographie auf Kieselgel 60 (Fa. Merck) mit Toluol/Methanol 70:30 gereinigt. Der nach Abdestillieren des Lösungsmittels aus der Hauptfraktion erhaltene Rückstand kristallisiert nach Verreiben mit Ether.
Ausbeute: 900 mg = 21 % d. Theorie
Fp: 94-95 °C
DC: Rf = 0,3
Kieselgel/Toluol-Methanol 70:30

Beispiel 48

2-{N-[4-(N-Methyl-4-fluorphenylsulfonamido)butyl]aminomethyl}-8-methoxy-tetralin

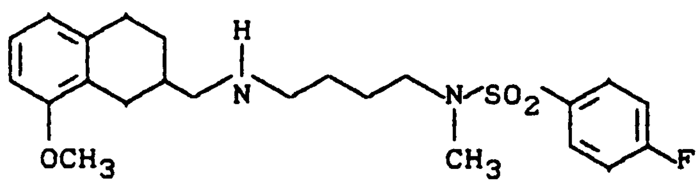

Die Verbindung wird analog der Arbeitsvorschrift aus Beispiel 47 aus 1 g 2-Aminomethyl-8-methoxytetralin (5,2 mmol), 1,7 g N-Methyl-N-(4-brombutyl)-4-fluorphenylsulfonamid (5,2 mmol) und 0,73 ml Triethylamin (5,2 mmol) in 20 ml Dimethylformamid hergestellt.

Das Endprodukt wird aus etherischer Lösung mit etherischer Chlorwasserstofflösung als Hydrochlorid gefällt.
Ausbeute: 400 mg = 16 % d. Theorie
Fp: 144 °C
DC: Rf = 0,44
Kieselgel/Toluol-Methanol 70:30

Beispiel 49

2-{N-[4-(N-Methyl-naphthalinyl-2-sulfonamido)butyl]aminomethyl}-8-methoxy-tetralin

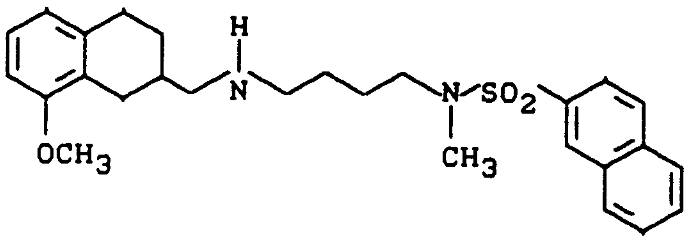

Die Verbindung wird analog der Arbeitsvorschrift aus Beispiel 47 aus 1 g 2-Aminomethyl-8-methoxytetralin (5,2 mmol), 1,9 g N-Methyl-N-(4-brombutyl)-naphthalin-2-sulfonamid (5,2 mmol) und 0,73 ml Triethylamin (5,2 mmol) in 20 ml Dimethylformamid hergestellt.

Das Endprodukt wird auf Kieselgel mit Toluol/Methanol 85:15 chromatographiert.

Das Endprodukt wird in Dichlormethan gelöst und mit etherischer Chlorwasserstofflösung als Hydrochlorid gefällt.

Ausbeute: 900 mg = 35 % d. Theorie
Fp: 194-196 °C
DC: Rf = 0,13
Kieselgel/Toluol-Methanol 85:15

Beispiel 50

2-{N-[4-(N-Methyl-naphthalinyl-1-sulfonamido)butyl]aminomethyl}-8-methoxy-tetralin

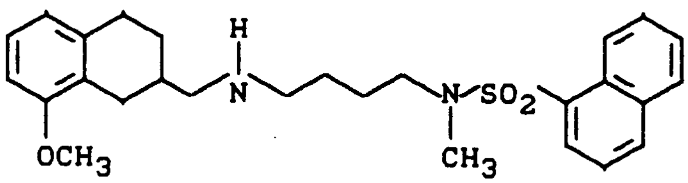

Die Verbindung wird analog der Arbeitsvorschrift in Beispiel 47 aus 2 g 2-Aminomethyl-8-methoxytetralin (10,5 mmol), 3,7 g N-Methyl-N-(4-brombutyl)-naphthalin-2-sulfonamid (10,5 mmol) und 1,46 ml Triethylamin (10,5 mmol) in 40 ml Dimethylformamid hergestellt.

Das Endprodukt wird auf Kieselgel mit Ethanol chromatographiert.

Das Endprodukt wird in Ethanol gelöst und mit etherischer Chlorwasserstofflösung als Hydrochlorid gefällt.

Ausbeute: 1,0 g = 19% d. Theorie
Fp: 161-164 °C
DC: Rf = 0,45
Kieselgel/Toluol-Methanol 70:30

Beispiel 51

2-{N-[4-(N-pyridinyl-2-methansulfonamido)butyl]aminomethyl}-8-methoxy-tetralin

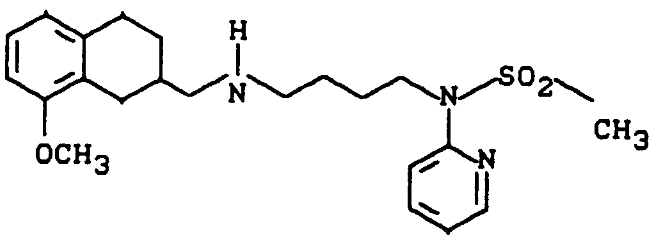

1,60 g 2-Aminomethyl-8-methoxytetralin (8,4 mmol), 2,28 g N-(4-Brombutyl)-pyridinyl-2-methansulfonamid (7,4 mmol) und 0,85 g Triethylamin (8,4 mmol) werden unter Argon in 32 ml Dimethylformamid 24 h bei 40 ° C gerührt.

Anschließend wird die Reaktionslösung in eine Mischung aus 160 ml 5 %iger Natriumchloridlösung, 80 ml Toluol und 8,5 ml 1 n Natronlauge verrührt. Die organische Phase wird 1 x mit 40 ml Wasser gewaschen und über Natriumsulfat getrocknet.

Nachdem das Lösungsmittel unter Vakuum abdestilliert wurde, wird das erhaltene Rohprodukt auf Kieselgel 60 (Fa. Merck) mit Methanol chromatographiert.

Ausbeute: 1,9 g = 61% d. Theorie
DC: Rf = 0,25
Kieselgel/Methanol

Beispiel 52

8′-Methoxy-2′-[4(2-methylsulfonylimino-1,2-dihydropyridin-1-yl)butyl]aminomethyl-1,2,3,4-tetrahydronaphthalin

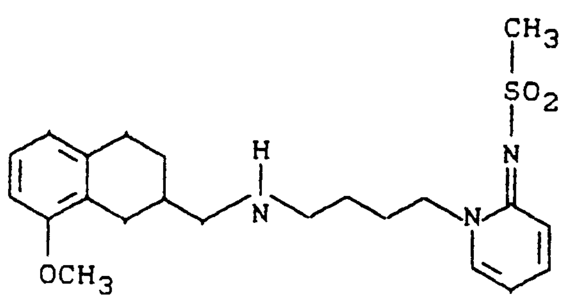

Die Verbindung wird analog der Arbeitsvorschrift in Beispiel 51 aus 1,60 g 2-Aminomethyl-8-methoxytetralin(8,4 mmol), 2,28 g 1-(4-Brombutyl)-2-methylsulfonylimino-1,2-dihydropyridin (7,4 mmol) und 0,85 ml Triethylamin (8,4 mmol) in 32 ml Dimethylformamid hergestellt.

Ausbeute: 2,1 g = 68% d. Theorie
DC: Rf = 0,15
Kieselgel/Toluol-Methanol 70:30
Rf = 0,43
Kieselgel/Methanol-Triethylamin 95:5

Beispiel 53

2-{N-[4-(1,1-Dioxido-3-oxo-2,3-dihydro-benzisothiazol-2-yl)pentyl] amino}-8-methoxy-chroman-hydrochlorid

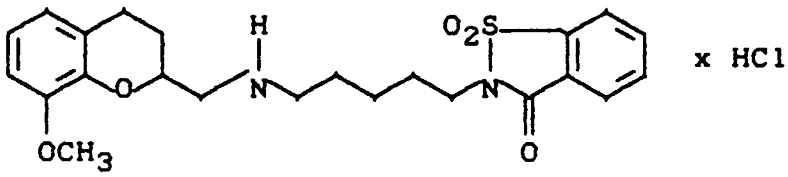

Die Titelverbindung wurde in Analogie zu der Vorschrift für Beispiel 20 hergestellt.
Fp. 110°-118°C (nach Sintern)

Beispiel 54

2-{N-[2-(N-Methyl-4-fluorphenylsulfonamido)ethyl]aminomethyl}chroman-hydrochlorid.

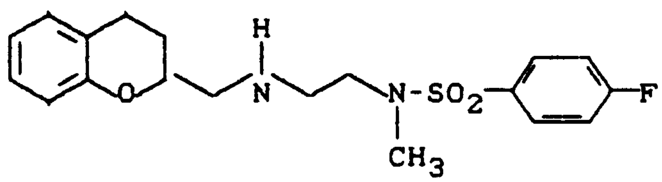

Die Titelverbindung wurde in Analogie zu der Vorschrift für Beispiel 20 hergestellt.
Fp: 143°C-155°C

Beispiel 55

2-{N-[4-(5,5-Dioxido-6H-dibenzo[c,1] [1,2] thiazin-6-yl)butyl}aminomethylchroman-1,5-naphthalindisulfonsäure Salz (Stöchiometrie 2:1)

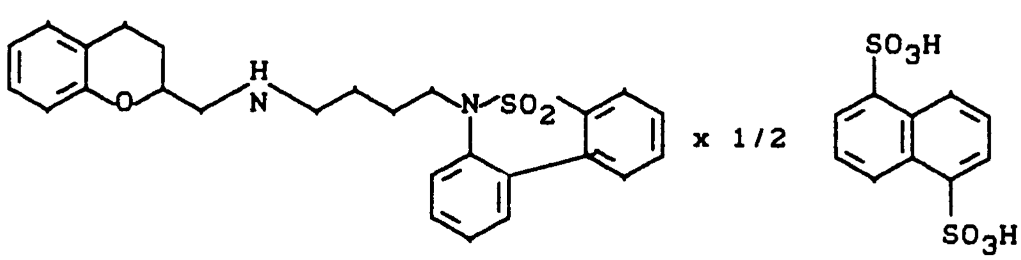

Die freie Base [$R_F$ ($CH_2Cl_2$/$CH_3OH$ 10:1) = 0,39] wurde durch Behandeln mit 1,5-Naphthalindisulfonsäure in Aceton/Ether in das 2:1 Salz überführt. Hellbeiger Feststoff.
Fp: > 240°C (Zersetzung)
Bei der Umsetzung wurde außerdem die Verbindung von Beispiel 56 erhalten.

Beispiel 56

2-{N,N-Bis(5,5-dioxido-6H-dibenzo[c,e][1,2]-thiazin-6-yl)butyl]}aminomethylchroman-1,5-naphthalindisulfonsäure-Salz (2:1)

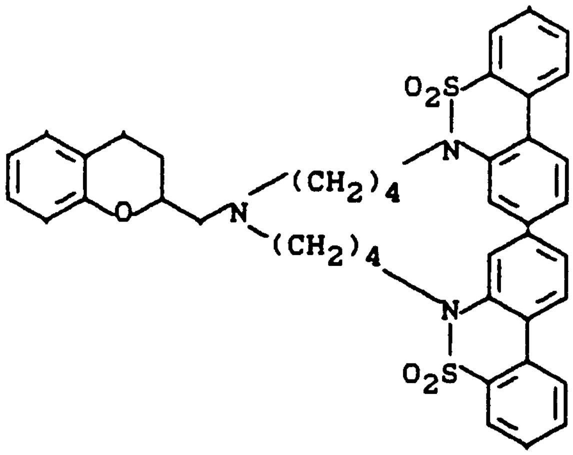

Die freie Base wurde neben der Verbindung von Beispiel 55 als unpolarere Komponente erhalten; $R_f$-($CH_2Cl_2/CH_3OH$ 10:1) = 0,91.

Das 2:1 Salz mit 1,5-Naphthalindisulfonsäure wurde wie in Beispiel 55 erhalten (farbloser Farbstoff). Fp: ab 170 °C (Zersetzung).

Beispiel 57

2-{N-[4-(5,5-Dioxido-6H-dibenzo[c,e][1,2]-thiazin-6-yl)butyl]}aminomethyl-8-methoxychroman Hydrochlorid

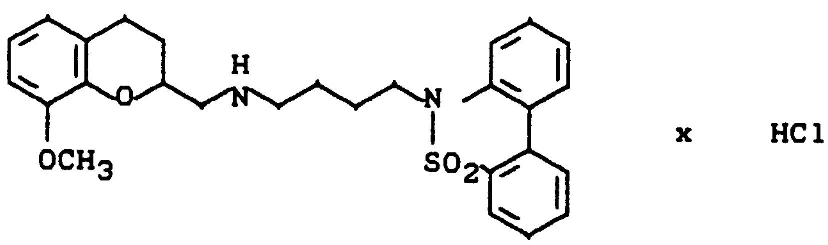

Die freie Base [$R_F$($CH_2Cl_2/CH_3OH$ 10:1) = 0,25] ergab nach Behandeln mit etherischem Chlorwasserstoff in Methanol das Hydrochlorid als amorphen, beigen Farbstoff.

Beispiel 58

2-{N-[4-(1,1-Dioxido-2H-naphth[1,8-cd]isothiazol-2-yl)butyl]}aminomethyl-8-methoxy-chroman Hydrochlorid

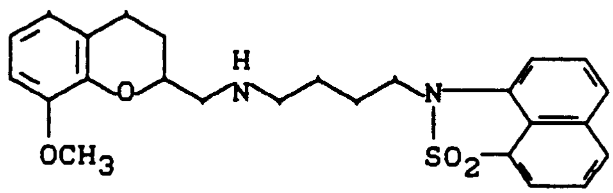

x HCl

Die freie Base [$R_F$($CH_2Cl_2$/$CH_3OH$ 10:1) = 0,3] ergab nach Behandeln mit etherischem Chlorwasserstoff in Essigester das Hydrochlorid als leicht grünliche Kristalle.
Fp = 159°-162°C.

Beispiel 59

2-{N-[3-(1,3-Dimethyl-uracil-6-yl)amino]propyl}aminomethyl-8-methoxy-chorman Hydrochlorid

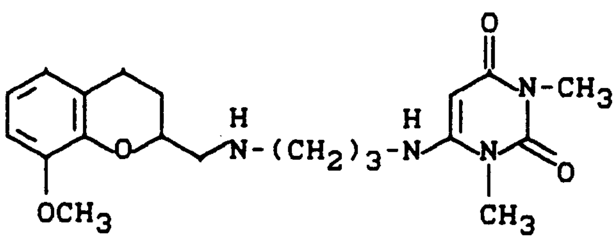

x HCl

Die Titelverbindung wurde in Analogie zur Vorschrift für Beispiel 20 hergestellt.

Die freie Base [$R_f$ ($CH_2Cl_2$/$CH_3OH$ 10:1) = 0,19], gelöst in Methanol, wurde mit etherischem Chlorwasserstoff behandelt. Nach Zugabe von Ether wurde das Hydrochlorid als farbloser Feststoff erhalten. Fp: ab 195°C Zersetzung (Aufschäumen).

Beispiel 60

2-{N-[4-(2,3-Dihydro-1,1-dioxido-benzisothiazol-2-yl)butyl]}aminomethyl-chroman Hydrochlorid

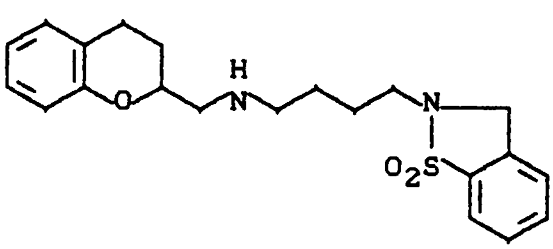

x HCl

Die Titelverbindung wurde in Analogie zu der Vorschrift für Beispiel 20 hergestellt.

Das Hydrochlorid wurde aus Isopropanol umkristallisiert.
Fp: 215°-216°C

Beispiel 61

2-{N-[4-(2,3-Dihydro-1,1-dioxido-benzisothiazol-2-yl)butyl]}aminomethyl-8-methoxy-chroman Hydrochlorid

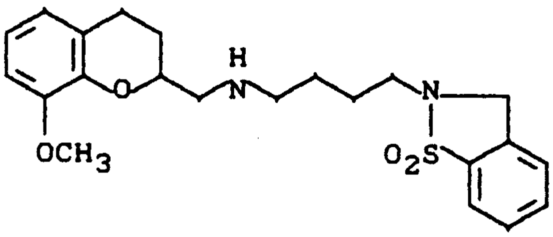

x HCl

Die Titelverbindung wurde in Analogie zu der Vorschrift für Beispiel 20 hergestellt.

Die freie Base [$R_F$ ($CH_2Cl_2/CH_3OH$ 10:1) = 0,27] wurde in Methanol gelöst und mit etherischem Chlorwasserstoff behandelt, das nach Zugabe von Ether ausgefallene Hydrochlorid wurde aus Essigester umkristallisiert.
Fp: 135°-138°C.

Beispiel 62

2-{N-[4-(4-Fluorphenylsulfonamido)butyl]}aminomethyl-chroman-Hydrochlorid

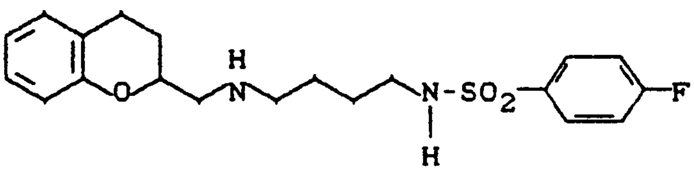

x HCl

Die Titelverbindung wurde in Analogie zu der Vorschrift für Beispiel 24 hergestellt.
Fp. 202-206°C
Die in Tabelle 1 aufgeführten Verbindungen (63-70) wurden analog der folgenden Arbeitsvorschrift dargestellt:
1,89 g 8-Methoxy-2-aminomethyl-1,3-dihydronaphthalin (10 mMol), x g I (10 mMol) und 1,39 ml Triethylamin (10 mMol) werden in 19 ml Dimethylformamid unter Argon -24 h bei 40°C gerührt.

Anschließend wird die Reaktionslösung langsam in eine gerührte Mischung aus 90 ml 5 %iger Natriumchloridlösung, 10 ml 1 N Natronlauge und 50 ml Toluol gegossen. Es wird noch 10 min weitergerührt und dann die organische Phase abgetrennt. Die wässrige Phase wird noch 1 x mit 25 ml Toluol extrahiert. Die vereinigten organischen Phasen werden 2 x mit 25 ml Wasser gewaschen, über Natriumsulfat getrocknet und das Lösungsmittel bei 40°C unter Vakuum abdestilliert.

Das erhaltende Rohprodukt wird durch eine Säulenchromatographie auf Kieselgel mit Ethanol (Beispiel 63-67) bzw. Essigester (Beispiel 68-70) als Fließmittel gereinigt.

Die saure Base wird in Ether/Ethanol 90:10 (Beispiel 63-67) bzw. Ether (Beispiel 68-70) gelöst und mit etherischer Chlorwasserstofflösung als Hydrochlorid ausgefällt.

Die Ausbeuten liegen zwischen 30 bis 40 % d. Theorie.

Tabelle 1

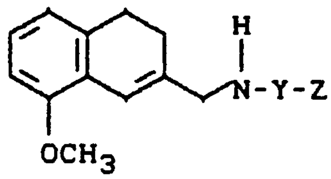

| Bsp.-Nr. | Y -Z | RF-Wert auf Kieselgel mit Toluol/Methanol 70:30 | Fp °C |
| --- | --- | --- | --- |
| 63 | 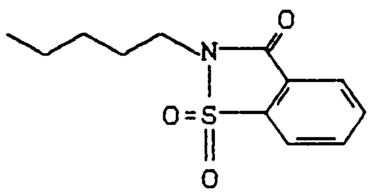 | 0,48 | 180° C |
| 64 | 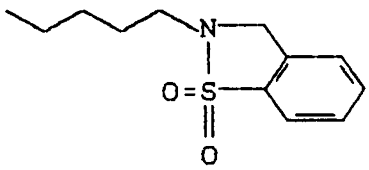 | 0,38 | 150-152° C |
| 65 | 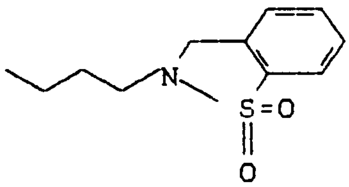 | 0,40 | 196-198° C |
| 66 | 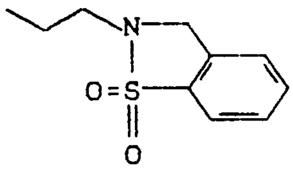 | 0,58 | 183-185° C |
| 67 | 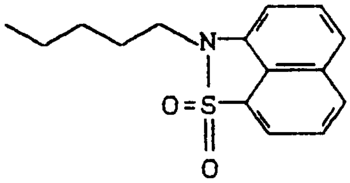 | 0,48 | - |

| Bsp.-Nr. | Y - Z | RF-Wert auf Kieselgel mit Toluol/Methanol 70:30 | Fp °C |
|---|---|---|---|
| 68 | 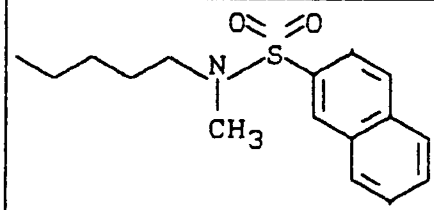 | 0,48 | - |
| 69 | 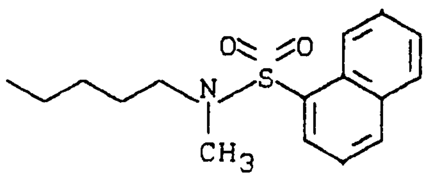 | 0,48 | 129-133° C |
| 70 | 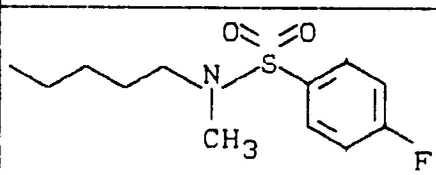 | 0,43 | 125° C |

Die im folgenden aufgeführten Beispiele 71 und 72 wurden analog der Vorschrift für die Beispiele 65 und 66 aus 8-Methoxy-2-aminomethyl-1,2,3,4-tetrahydronaphthalin, 2-(3-Brompropyl-1)benzoisothiazol-1,1-dioxid, bzw. 2-(2-Brommethyl-1)benzisothiazol-1-dioxid und Triethylamin in Dimethylformamid hergestellt.

Beispiel 71

8-Methoxy-2-aminomethyl-N[3-(Benzisothiazol-1,1-dioxyd-2-yl)propyl]-1,2,3,4-tetrahydronaphthalin

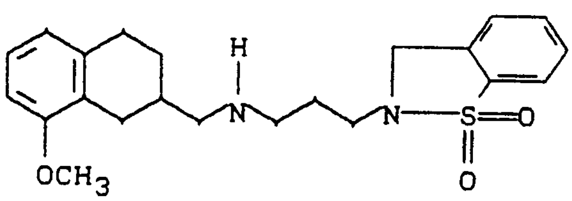

$R_F$ = 0,393 (Hydrochlorid), auf Kieselgel mit Toluol/Methanol (70:30).
Fp = 166-168 °C (Hydrochlorid)
Ausbeute: 27 % d.Th.

Beispiel 72

8-Methoxy-2-aminomethyl-N[2-(Benzisothiazol-1,1-dioxid-2-yl)ethyl-1]-1,2,3,4-tetrahydronaphthalin

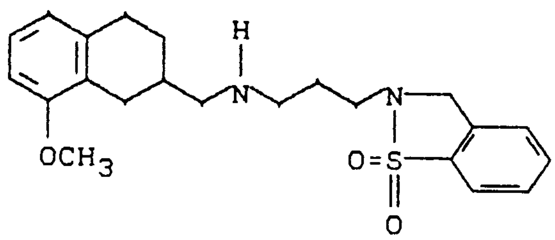

$R_F$ = 0,483 (Hydrochlorid), auf Kieselgel mit Toluol/Methanol (70:30)
Fp: 236-238 °C (Hydrochlorid)
Ausbeute: 38 % d.Th.

In Analogie zur Vorschrift des Beispiels 20 wurden die in Tabelle 2 aufgeführten Verbindungen hergestellt:

Tabelle 2

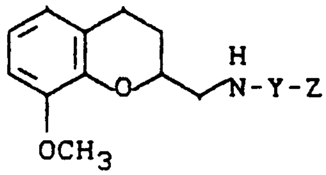

| Beispiel Nr.: | Y - Z | DC RF-Wert oder MS | $Fp^{0}C$ | Salz |
|---|---|---|---|---|
| 73 | 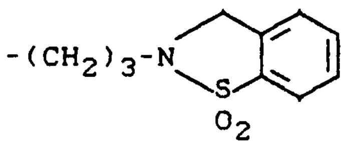 | | 195-197 | x HCL |
| 74 | 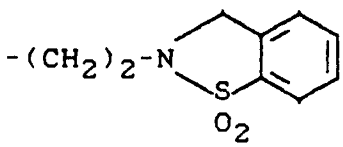 | 0,50 $CH_2Cl_2/CH_3OH$ (10:1) | | |
| 75 | 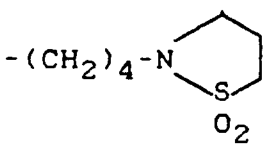 | | | |
| 76 | 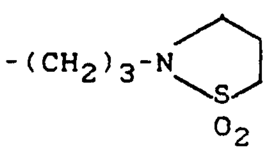 | | | |
| 77 | 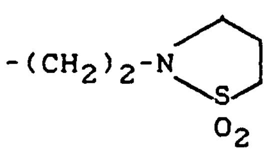 | | | |
| 78 | 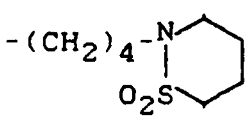 | MS: (freie Base) 382, 219 (100%) 84 | | |
| 79 | 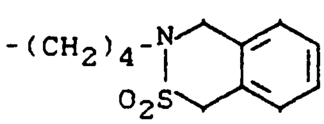 | MS: (freie Base) 430, 267 (100%) 105, 86, 84 | | amorph |

Tabelle 2 (Fortsetzung)

| Beispiel Nr.: | Y - Z | DC RF-Wert oder MS | Fp° C | Salz |
|---|---|---|---|---|
| 80 | 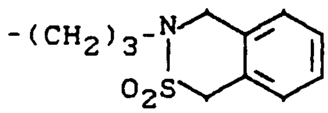 | | | |
| 81 | 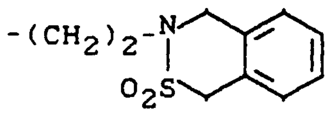 | 0,16 $CHCl_2$/ $C_3H_8OH$ (20:1) | 158-159 | Oxa-lat |

In Analogie zur Vorschrift des Beispiels 20 wurden die in den Tabellen 3, 4 und 5 aufgeführten Verbindungen hergestellt:

Tabelle 3

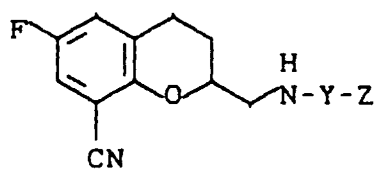

x HCl

| Beispiel-N. | Y - Z | DC/$R_f$-Wert auf Kieselgel Toluol/Methanol (70:30)* oder Ethanol** | Fp° C |
|---|---|---|---|
| 82 | 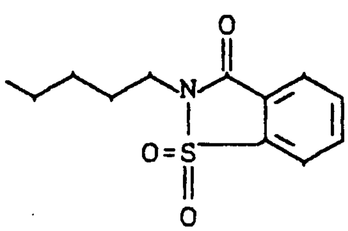 | 0,25** | 109-111° C |

Tabelle 3 (Fortsetzung)

| Beispiel-N. | Y - Z | DC/$R_f$-Wert auf Kieselgel Toluol/Methanol (70:30)* oder Ethanol** | Fp° C |
|---|---|---|---|
| 83 | 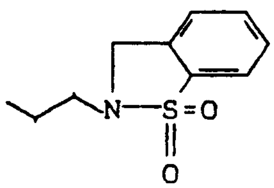 | 0,49* | 199-201° C |
| 84 | 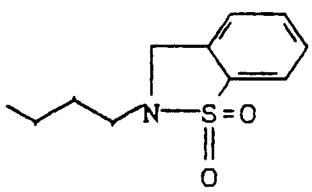 | | |
| 85 | 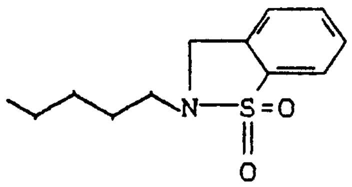 | 0,38* | 216-218° C |

Tabelle 4

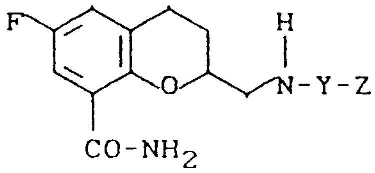 x HCl

| Beispiel-Nr. | Y - Z | DC/$R_F$-Wert auf Kieselgel/ Ethanol | Fp° C |
|---|---|---|---|
| 86 | 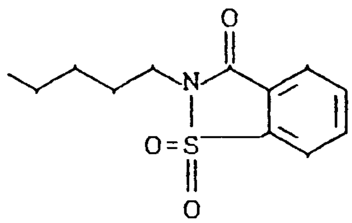 | 0,28 | 140-142° C |
| 87 | 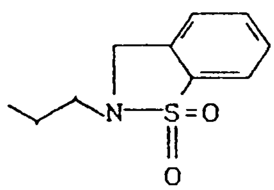 | | |
| 88 | 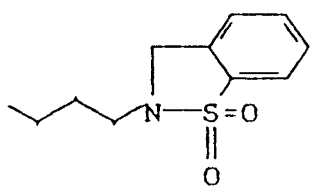 | | |
| 89 | 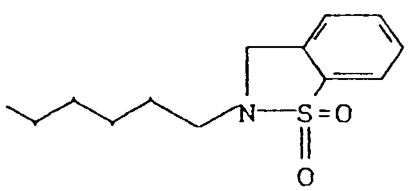 | | |

Tabelle 5

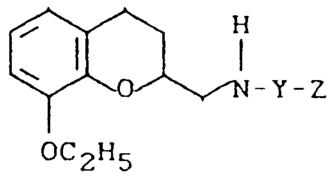

x HCl

| Beispiel-Nr. | Y - Z | DC / $R_F$-Wert | Fp° C |
|---|---|---|---|
| 90 | 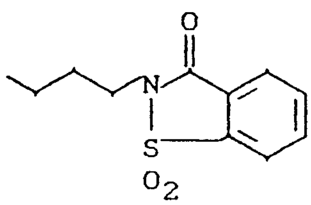 | 0,38<br>$ClCH_2CH_2Cl$ / iPrOH<br>10 : 1 | 185° C |
| 91 | 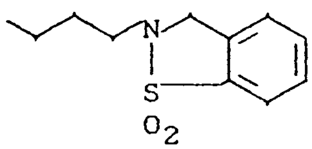 | 0,22<br>$ClCH_2CH_2Cl$ / iPrOH<br>10 : 1 | 153° C |

Beispiel 92

(-)-{2-[N-[4-(1,1-Dioxido-3-oxo-2,3-dihydro-benzisothiazol-2-yl)butyl]aminomethyl]chroman} und

(+)-{2-[N-[4-(1,1-Dioxido-3-oxo-2,3-dihydro-benzisothiazol-2-yl)butyl]aminomethyl]chroman}

Beispiel 92 A

Chroman-2-carbonsäurechlorid

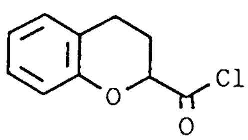

89,0 g (0,5 Mol) Chroman-2-carbonsäure und 71,4 g (0,6 Mol) Thionylchlorid werden bis zur Beendigung der Gasentwicklung (4 - 5 Stunden) auf 80 °C erhitzt. Destillieren liefert 96,0 g (98 %) Chroman-2-carbonsäurechlorid vom Schmelzpunkt 77 - 80 °C / 13,3 Pa (0,1 Torr).

Beispiel 92 B

Chroman-2-carbonsäure-N-[1-(S)-phenyl-ethyl]amid (Diastereomere)

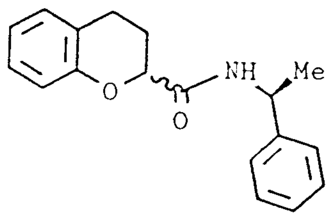

In die Lösung von 59,0 g (0,3 Mol) Chroman-2-carbonsäurechlorid in 200 ml Dichlormethan werden bei 10 °C unter Rühren 39,9 g (0,33 Mol) (S)-(-)-1-Phenylethylamin und 30,9 g (0,3 Mol) Triethylamin getropft. Nach Rühren über Nacht wird der Ansatz auf Eis gegeben. Trennen der Phasen, Waschen der organischen Phase mit gesättigter Kochsalzlösung und Trocknen über wasserfreiem Natriumsulfat liefern 87,8 g rohes Chroman-2-carbonsäure-N-[1-(S)phenylethyl]amid als 1:1 Gemisch der Diastereomeren. Nach Kristallisieren (4 x) aus Ethanol werden 15,8 g eines einheitlichen Diastereomers (de ≧ 99,5) vom Schmelzpunkt 127 - 128 °C erhalten; $\alpha_D$ = + 17,5 (c = 1, Tetrahydrofuran).

| | | | | |
|---|---|---|---|---|
| $C_{18}H_{19}NO_2$ | ber. | C 76,84 | H 6,81 | N 4,98 |
| 281,4) | gef. | 76,9 | 7,18 | 4,97 |

Eindampfen der Mutterlaugen ergab 61,2 g eines Gemisches der Diasteromeren.

Beispiel 92 C

Chroman-2-carbonsäure-N-[1-(R)-[phenylethyl]amid (Diastereomere)

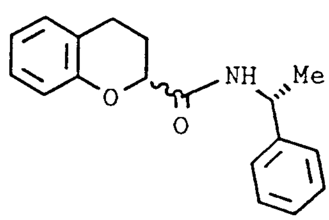

Entsprechend Beispiel 92 B erhält man bei Verwendung des (R)-(+)-1-Phenylethylamins nach Kristallisieren (4 x) aus Ethanol 15,0 g eines einheitlichen Diastereomers (de = 100 %) vom Schmelzpunkt 127 - 128 °C; $\alpha_D$ = -17,2 (C = 1, Tetrahydrofuran).

| | | | | |
|---|---|---|---|---|
| $C_{18}H_{19}NO_2$ | ber. | C 76,84 | H 6,81 | N 4,98 |
| (281,4) | gef. | 76,8 | 7,22 | 5,17 |

Beispiel 92 D

N-[1-(S)-phenylethyl]-2-aminomethyl-chroman (Diastereomer A)

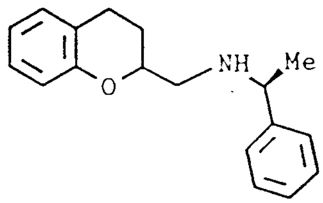

In die auf 0°C gekühlte Lösung von 0,2 Mol Diboran in 400 ml trockenem Tetrahydrofuran werden unter Rühen 16,2 g (0,058 Mol) des nach Beispiel 92B hergestellten einheitlichen Diastereomers (Chroman-2-carbonsäure-N-[-(S)-phenylethyl]amid; $\alpha_D$ = +17,5°) in 300 ml trockenem Tetrahydrofuran getropft. Nach Rühren über Nacht bei 20°C wird der Ansatz 1 Stunde zum Rückfluß erhitzt, abgekühlt und vorsichtig mit 10 %iger wäßriger Salzsäure zersetzt.

Nach dem Abziehen des Lösungsmittels im Vakuum wird der Rückstand mit verdünnter Natronlauge alkalisch (pH ˜ 8,5) gestellt und ausgeethert. Waschen der vereinigten organischen Extrakte mit gesättigter Kochsalzlösung, Trocknen über wasserfreiem Natriumsulfat und Eindampfen liefern 17,4 g rohes N-[1-(S)-phenethyl]-2-aminomethylchroman; de >99,5. Zur Analyse werden 0,5 g im Kugelrohr bei 190°C / 2,66 Pa (0,2 Torr) destilliert.

| | | | | |
|---|---|---|---|---|
| $C_{18}H_{21}NO$ (267,4) | ber. gef. | C 80,86 80,7 | H 7,92 8,01 | N 5,24 5,41 |

Beispiel 92 E

(+)-N-[1-(S)-Phenylethyl]-2-aminomethyl-chroman (Diastereomer B)

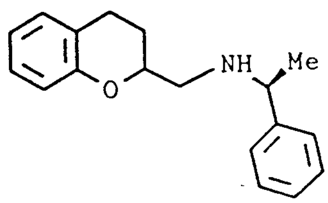

Nach Beispiel 92 D werden 42,2 g (0,15 Mol) des bei der Abtrennung des einheitlichen Diastereomers gemäß Beispiel 92 B angefallenen Gemisches der Diastereomeren mit 0,3 Mol Diboran in Tetrahydrofuran umgesetzt. Aufarbeiten liefert 42,8 g rohes N-[1-(S)-Phenylethyl]-2-aminomethylchroman als Gemisch der Diastereomeren, das an 600 g Kieselgel mit Toluol/Essigsäureethylester chromatografiert wird. Man erhält 9,0 g eines einheitlichen N-[1-(S)-Phenylethyl]-2-aminomethylchromans, das mit einem gemäß Beispiel 92 D erhaltenen Präparat (Diastereomer A) identisch ist, und 25,4 g eines einheitlichen, weniger polaren N-[1-(S)-Phenylethyl]-2-aminomethylchormans; de > 99,5 (Diastereomer B). Zur Analyse werden 0,5 g im Kugelrohr bei 195 °C / 6,65 Pa (0,05 Torr) destilliert $\alpha_D$ = +42,3° (c = 1, Tetrahydrofuran)

| | | | | |
|---|---|---|---|---|
| $C_{18}H_{21}NO$ (163,2) | gef. | C 80,7 | H 8,08 | N 5,38 |

Beispiel 92 F

(-)-2-Aminomethylchroman

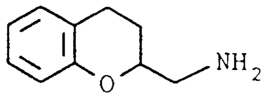

27,5 g (0,1 Mol) einheitliches N-[1-(S)-Phenylethyl]-2-aminomethylchroman (hergestellt nach Beispiel 92 D) werden in 400 ml Ethanol 24 Stunden bei 50 °C und 10 bar hydriert (Pd/C 5 %). Nach Eindampfen werden 15,6 g farbloses Öl erhalten. Filtrieren über Kieselgel mit Toluol/Essigsäureethylester und darauf mit Methanol liefern 12,5 g 2-Aminomethylchroman vom Schmelzpunkt 100 - 110 °C / 5,32 Pa (0,04 Torr) (Kugelrohr); ee ≧ 97,5 % $\alpha_D$ = -122,8 ° (C = 1, Trichlormethan).

| | | | | |
|---|---|---|---|---|
| $C_{10}H_{13}NO$ (163,2) | ber.<br>gef. | C 73,59<br>73,7 | H 8,03<br>8,39 | N 8,58<br>8,85 |

Beispiel 92 G

(+)-2-Aminomethylchroman

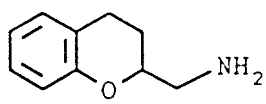

Analog Beispiel 92 F werden 21,5 g (0,08 Mol) einheitliches N-[1-(S)-Phenylethyl]-2-aminomethylchroman (hergestellt nach Beispiel 92 E, $\alpha_D$ = + 42,3) in 400 ml Ethanol hydriert (Pd/C 5%). 10,2 g einheitliches (+)-2-Aminomethylchroman vom Schmelzpunkt 100 b 110 °C / 3,99 Pa (0,03 Torr) (Kugelrohr) werden erhalten; ee ≧ 97,0 %, $\alpha_D$ = +128,8 ° (c = 1, Trichlormethan).

| | | | | |
|---|---|---|---|---|
| $C_{10}H_{13}NO$ (163,2) | gef. | C 73,3 | H 8,11 | N 8,82 |

Beispiel 92 H

2-[N-[4-(1,1-Dioxido-3-oxo-2,3-dihydro-benzisothiazol-2-yl)butyl]aminomethyl]chroman und Hydrochlorid [(-)-Isomeres]

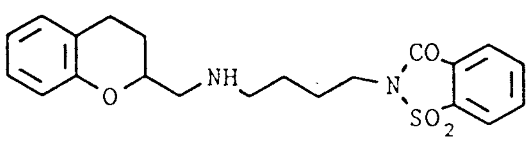

5,4 g (0,033 Mol) 2-Aminomethylchroman (Beispiel 92 F, $\alpha_D$ = -122,8°), 6,1 g (0,06 Mol) Triethylamin, 9,5 g (0,03 Mol) N-4-Brombutylsaccharin und 80 ml trockenes Dimethylformamid werden 4 Stunden auf 60 °C erwärmt. Nach Abziehen des Lösungsmittels bei 1,33 Pa (0,01 Torr) wird der Rückstand (13,5 g) in Toluol/Essigsäureethylester (5:1) aufgenommen und an 300 g Kieselgel chromatographiert. Das mit Toluol/Essigsäureester (1:1) eluierte Produkt (5,8 g) wird an 150 g Kieselgel rechromatographiert und ergibt 3,7 g einheitliches 2-[N-[4-(1,1-Dioxido-3-oxo-2,3-dihydro-benzisothiazol-2-yl)butyl]aminomethyl]chroman.
Hydrochlorid :
Schmelzpunkt 192 - 194 °C
(abgeschmolzene Kapillare) werden erhalten; ee ≧ 99%, $\alpha_D$ = -42,2 (c = 1, Trichlormethan).

| | | | | |
|---|---|---|---|---|
| $C_{21}H_{24}N_2O_4S$ x CHl (437) | ber. gef. | C 57,72 57,5 | H 5,78 5,81 | N 6,41 6,40 |

Beispiel 92l

2-[N-[4-(1,1-Dioxido-3-oxo-2,3-dihydro-benzisothiazol-2-yl)butyl]aminomethyl]chroman und Hydrochlorid [-(+)-Isomeres]

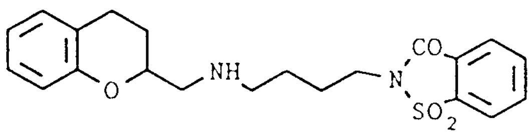

5,4 g (0,033 Mol) 2-Aminomethylchroman aus Beispiel 92 G ($\alpha_D$ = +122,8°) werden analog Beispiel 92 H mit N-(4-Brombutyl)saccharin umgesetzt, Chromatographie des Rohprodukts (11,6 g) an Kieselgel mit Toluol/Essigsäureethylester (5:1 bis 2:1) ergibt 5,4 g Rohprodukt, das an 175 g Kieselgel rechromatographiert wird. 4,2 g einheitliches 2-[N-[4-(1,1-Dioxido-3-oxo-2,3-dihydro-benzisothiazol-2-yl)butyl]aminomethyl]-chroman
Hydrochlorid:
Schmelzpunkt: 192 - 194°C
(abgeschmolzene Kapillare); ee ≧ 99 %, $\alpha_D$ = + 43,5° (c = 1, Trichlormethan).

| | | | | |
|---|---|---|---|---|
| $C_{21}H_{24}N_2O_4S$ x HCl (437) | gef. | C 57,4 | H 5,72 | N 6,33 |

Beispiel 93

(+)-{2-[N-[4-1,1-Dioxido-3-oxo-2,3-dihydro-benzisothiazol-2-yl)butyl]aminomethyl]-8-methoxy-chroman} und

(-)-{2-[N-[4-1,1-Dioxido-3-oxo-2,3-dihydro-benzisothiazol-2-yl)butyl]aminomethyl]-8-methoxy-chroman}

Beispiel 93 A

8-Methoxychroman-2-carbonsäure

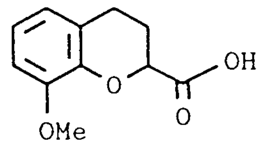

100,7 g (0,43 Mol) 8-Methoxychroman-2-carbonsäureethylester und 20,1 g (0,50 Mol) Natriumhydroxid werden in 800 ml Ethanol 24 Stunden bei 20 °C gerührt. Nach Einengen der Lösung versetzt man den Rückstand mit Wasser, extrahiert mit Diethylether und säuert die wäßrige Phase an. Extrahieren mit Diethylether liefert nach üblichem Aufarbeiten 95,2 kristalline 8-Methoxychroman-2-carbonsäure.

Beispiel 93 B

8-Methoxychroman-2-carbonsäurechlorid

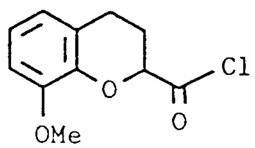

Analog zu Beispiel 92 A wurde 8-Methoxychroman-2-carbonsäurechlorid aus Beispiel 93 A erhalten und als Rohprodukt weiter verarbeitet.

Beispiel 93C

8-Methoxychroman-2-carbonsäure-N-[1-(S)-phenylethyl]amid (Diastereomere)

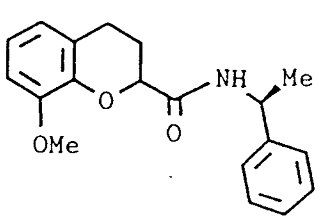

Analog Beispiel 92 B werden 88,7 g (0,39 Mol) rohes 8-Methoxychroman-2-carbonsäurechlorid(Beispiel 93 B) in 600 ml Dichlormethan mit 64,9 g (0,54 Mol) (S)-(-)-1-Phenylethylamin und 54,1 g (0,54 Mol)

Triethylamin bei 10 °C umgesetzt. Aufarbeiten liefert 135,2 g rohes 8-Methoxychroman-2-carbonsäure-N-[1-(S)-phenylethyl]amid als 1:1 Gemisch der Diastereomeren. Chromatographie an 2000 g Kieselgel mit Toluol/Essigsäureethylester ergibt 47,9 g einheitliches Diastereomer A und 33,9 g einheitliches Diastereomer B. 29,2 g eines Gemisches aus den beiden Diastereomeren A und B werden erneut chromatographiert

8-Methoxychroman-2-carbonsäure-N-[1-(S)-phenylethyl]amid

Diastereomer A

Schmelzpunkt 123 - 124 °C (Dichlormethan/Petrolether) de > 99 %, $\alpha_D$ = + 6,3 ° (c = 1, Tetrahydrofuran)

| | | | | |
|---|---|---|---|---|
| $C_{19}H_{21}NO_3$ | ber. | C 73,29 | H 6,80 | N 4,50 |
| (311,4) | gef. | 73,0 | 6,69 | 4,53 |

Diastereomer B

Schmelzpunkt 108 - 109 °C (Dichlormethan/Petrolether) de 100 %, $\alpha_D$ = + 40,2 ° (C = 1, Tetrahydrofuran)

| | | | | |
|---|---|---|---|---|
| $C_{19}H_{21}NO_2$ | gef. | C 73,2 | H 6,93 | N 4,61 |

Beispiel 93 D

(+)-N-[1-(S)-Phenylethyl]-2-aminomethyl-8-methoxy-chroman

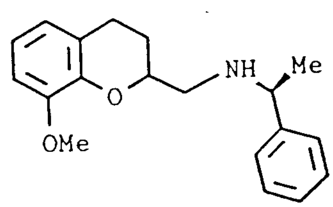

46,7 g (0,15 Mol) 8-Methoxychroman-2-carbonsäure-N-[1-(S)-phenylethyl]amid (Diastereomer A aus Beispiel 93 C; Schmelzpunkt 123-124 °C) werden analog Beispiel 92 D in 500 ml trockenem Tetrhydrofuran zu 0,35 Mol Diboran in 650 ml trockenem Tetrahydrofuran bei 10 °C getropft. Aufarbeiten liefert 50,8 g rohes N-[1-(S)-Phenylethyl]-8-2-aminomethyl-8-methoxychroman.
Chromatographie der aus zwei Ansätzen erhaltenen Rohprodukte an Kieselgel (2000 g) mit Toluol/Essigsäureethylester (10:1 bis 5:1) ergibt 69,2 g einheitliches (+)-N-[1-(S)-Phenylethyl]-2-aminomethyl-8-methoxy-chroman sowie 21,1 g 8-Methoxychroman-2-carbonsäure-N-[1-(S)-phenylethyl]amid.

Zur Analyse wird 1 g im Kugelrohr bei 160-170 °C/6,65 Pa (0,05 Torr) destilliert.
de = 100 %, $\alpha_D$ = +30,3 ° (c = 1, Tetrahydrofuran)

| | | | | |
|---|---|---|---|---|
| $C_{19}H_{23}NO_2$ | ber. | C 76,73 | H 7,80 | N 4,71 |
| (297,4) | gef. | 76,5 | 7,84 | 4,66 |

Beispiel 93 E

N-[1-(S)-Phenylethyl]-2-aminomethyl-8-methoxy-chroman (anderes Diastereomeres)

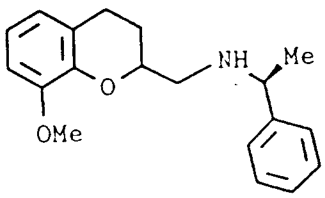

Analog zu Beispiel 93 D wurde aus Diastereomer B des Beispiels 93 C (Schmelzpunkt 108-109 °C) das Diastereomere zu Beispiel 93 D erhalten.
de = 100 %.

Zur Analyse wird im Kugelrohr bei 175 °C/6,65 Pa (0,05 Torr) destilliert.

| | | | | |
|---|---|---|---|---|
| $C_{19}H_{23}NO_2$ (297,4) | gef. | C 76,8 | H 7,87 | N 5,03 |

Beispiel 93 F

(+)-2-Aminomethyl-8-methoxychroman

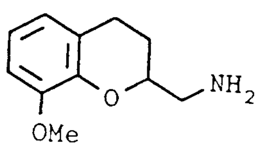

49,2 g (0,16 Mol) 2-N-[1-(S)-Phenylethyl]-aminomethyl-8-methoxychroman , nach Beispiel 93 D erhalten, werden in zwei Ansätzen in jeweils 400 ml Ethanol 24 Stunden bei 50 °C und 10 bar hydriert (Pd/C 5 %). Filtrieren und Eindampfen liefern 34,8 g rohes 2-Aminomethyl-8-methoxychroman, das an 600 g Kieselgel mit Toluol/Essigsäureethylester chromatographiert wird. Die mit Toluol/Essigsäureethylester (1:2) erhaltene Fraktion wird im Kugelrohr bei 170-180 °/2,66 Pa (0,02 Torr) destilliert und ergibt 20,1 g einheitliches 2-Aminomethyl-8-methoxychroman; ee = 96,7 %, $\alpha_D$ = +111,5 ° (c = 1, Trichlormethan)

| | | | | |
|---|---|---|---|---|
| $C_{11}H_{15}NO_2$ (193,2) | ber.<br>gef. | C 68,37<br>68,3 | H 7,82<br>8,02 | N 7,25<br>7,34 |

Beispiel 93 G

(-)-2-Aminomethyl-8-methoxychroman

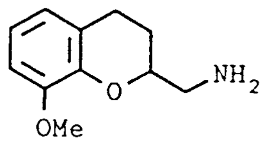

Analog Beispiel 93 F wurden aus Beispiel 93 E das Enantiomere zu Beispiel 93 F erhalten.
Siedepunkt: 160-170 ° C/9,31 Pa (0,07 Torr) (Kugelrohr)
Schmelzbereich: 49-55 ° C
ee = 96,1 %, $\alpha_D$ = -110,8 ° (c = 1, Trichlormethan)

| | | | | |
|---|---|---|---|---|
| $C_{11}H_{15}NO_2$ (193,2) | gef. | C 68,0 | H 7,88 | N 7,23 |

Beispiel 93 H

2-[N-[4-(1,1-Dioxido-3-oxo-2,3-dihydro-benzisothiazol-2-yl)butyl]aminomethyl]-methoxy-chroman und Hydrochlorid [( + )-Enantiomeres]

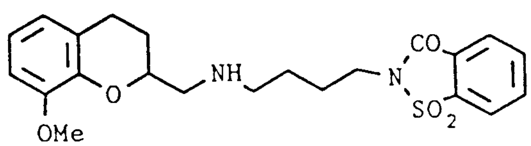

Die Lösung von 5,3 g (0,027 Mol) 2-Aminomethyl-8-methoxychroman, 9,5 g (0,03 Mol) N-4-Brombutylsaccharin, 3,03 g (0,03 Mol) Triethylamin und 80 ml Dimethylformamid rührt man 5 Stunden bei 60 ° C. Einengen der Lösung im Vakuum, Aufnehmen des Rückstands in Dichlormethan, Zugabe verdünnter Natronlauge (0,1 n NaOH)) und Wasser bis zum pH-Wert 8 und Trennen der Phasen liefert nach Waschen der organischen Phase bis zum pH-Wert 7 mit gesättigter Kochsalzlösung und Eindampfen 17,9 g Rohprodukt, das an 250 g Kieselgel mit Toluol/Methanol (10:1) chromatographiert wird.
Man erhält 10,5 g rohes 2-[N-[4-(1,1-Dioxido-3-oxo -2,3-dihydro-benzisothiazol-2-yl)butyl]aminomethyl]-methoxy-chroman, das an 300 g Kieselgel rechromatographiert wird. Mit Toluol/Methanol (10:1) erhält man 6,6 g einheitliches Produkt.
Hydrochlorid
Schmelzpunkt: 205-208 ° C (abgeschmolzene Kapillare) nach Umkristallisation aus Dichlormethan/Petrolether.
ee ≧ 99 %, $\alpha_D$ = + 53,1 °, (c = 1. Trichlormethan).

| | | | | |
|---|---|---|---|---|
| $C_{22}H_{26}N_2O_5S$ X HCl (467,0) | ber. gef. | C 56,58 56,8 | H 5,83 5,98 | N 6,00 5,97 |

Beispiel 93 I

2-[N-[4-(1,1-Dioxido-3-oxo-2,3-dihydro-benzisothiazol-2-yl)butyl]aminomethyl-8-methoxy-chroman und Hydrochlorid [(-)-Enantiomeres]

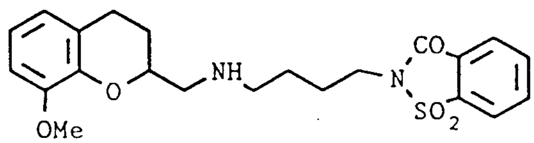

5,3 g (0,027 Mol) 2-Aminomethyl-8-methoxy-chroman, hergestellt nach Beispiel 93 G, 9,5 g (0,03 Mol) N-4-Brombutylsaccharin, 3,05 g (0,03 Mol) Triethylamin und 80 ml Dimethylformamid werden 4 Stunden auf 50 °C erhitzt. Aufarbeiten, wie im Beispiel 93 H beschrieben, liefert 16,6 g Rohprodukt, das über 175 g Kieselgel mit Toluol/Essigsäureethylester filtriert wird. Man erhält 3,4 g Dialkylierungsprodukt und 7,1 g Monoalkylierungsprodukt. Rechromatographie des Monoalkylierungsproduktes an 230 g Kieselgel mit Toluol/Methanol (10:1) liefert 2,5 g einheitliches 2-[N-[4-(1,1-Dioxido-3-oxo-2,3-dihydro-benzisothiazol-2-yl)butyl]-aminomethyl-8-methoxy-chroman.
Hydrochlorid
Schmelzpunkt: 208-210 °C (abgeschmolzene Kapillare) nach Umkristallision aus Dichlormethyn/Petrolether: ee ≧ 99%, $\alpha_D$ = -51,2 ° (c = 1, Trichlormethan).

| | | | | |
|---|---|---|---|---|
| $C_{22}H_{26}N_2O_5S$ x HCl (467,0) | gef. | C 56,5 | H 5,82 | N 5,95 |

**Patentansprüche**
**Patentansprüche für folgende Vertragsstaaten : AT, BE, CH, DE, FR, GB, GR, IT, LI, LU, NL, SE**

**1.** Substituierte Aminomethyltetraline sowie ihre heterocyclischen Analoga der allgemeinen Formel

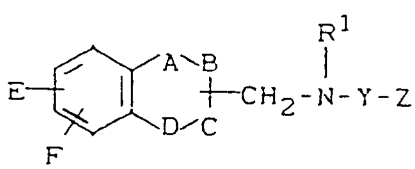
(I),

in welcher

Y - eine geradkettige Alkylenkette mit 2-5 Kohlenstoffatomen bedeutet,

Z - eine Gruppe der Formel

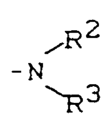

bedeutet,
wobei
$R^2$ und $R^3$ gleich oder verschieden sind und

- für Wasserstoff, Methyl, Ethyl, Propyl, oder
- für Phenyl, Benzyl oder Pyridyl stehen, das gegebenenfalls substituiert ist durch Fluor, Chlor, Methyl oder Methoxy
- für eine Gruppe der Formel

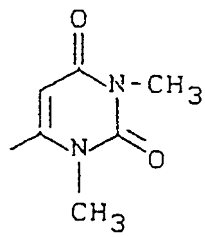

stehen oder
- für eine Gruppe der Formel $-COR^9$ oder $-SO_2R^{10}$ stehen,

worin

$R^9$
- für Methyl, Ethyl oder Ethoxy steht oder
- für Phenyl oder Benzyl steht, gegebenenfalls substituiert durch Methyl, Methoxy, Fluor oder Chlor

$R^{10}$
- für Methyl, Ethyl oder Propyl steht,
- für Phenyl, Naphthyl oder Benzyl steht, das gegebenenfalls durch Fluor oder Chlor substitutiert ist oder
- für eine Gruppe der Formel

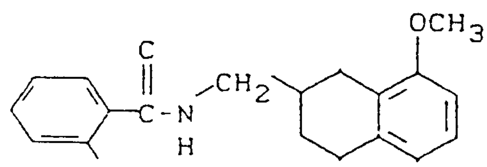

steht

$R^2$ und $R^3$ gemeinsam mit dem Stickstoffatom einen Heterocyclus aus der Reihe

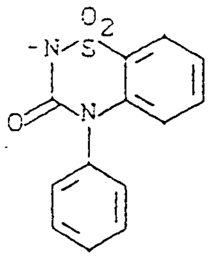
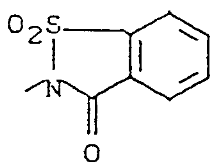
,
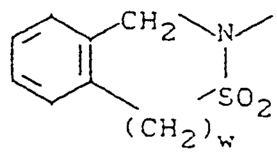

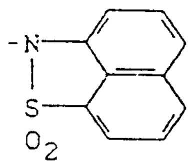
,
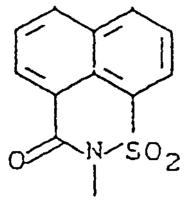
,
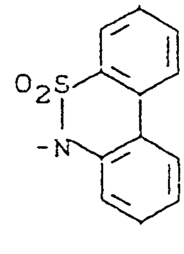

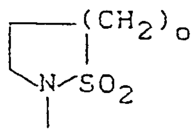
oder
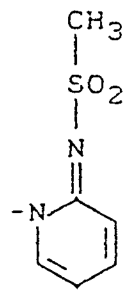

bilden,
wobei

w
- eine Zahl 0, 1 oder 2 bedeutet und

o
- eine Zahl 1 oder 2 bedeutet

$R^1$
- Wasserstoff, Ethyl, Methyl, Propyl oder Benzyl bedeutet, oder
- die Gruppe $-(Y^1-Z^1)$ bedeutet, wobei $Y^1$ und $Z^1$ gleich oder verschieden zu Y und Z sein können und die oben angegebene Bedeutung von Y und Z haben,

A und D für eine Gruppe der Formel $-CH_2-$, für Sauerstoff oder für den CH- oder N-Teil einer C=C-C=N-Doppelbindung stehen, mit der Maßgabe, daß entweder nur A oder nur D für Sauerstoff stehen darf,

B
- für eine Gruppe der Formel $>CH_2$ oder $\geqslant CH$ oder für den CH-Teil einer C=C- oder C=N-Doppelbindung steht,

C
- für eine Gruppe der Formel $\geqslant CH$ oder den C-Teil einer C=C oder C=N Doppelbindung steht,

E und F gleich oder verschieden sind und für Wasserstoff, Ethoxy, Methoxy, Fluor, Cyano oder eine Gruppe der Formel $-CONR^2R^3$ stehen, worin $R^2$ und $R^3$ Wasserstoff bedeuten,
E und F gemeinsam einen Phenyl oder Cyclohexanring bilden,
und deren Salze.

**2.** Substituierte Aminomethyltetraline sowie ihre heterocyclischen Analoga nach Anspruch 1 worin

Y
- eine geradkettige Alkylenkette mit 2-5 Kohlenstoffatomen bedeutet,

Z

- eine Gruppe der Formel

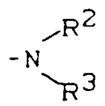

bedeutet,
worin

$R^2$ und $R^3$ gleich oder verschieden sind und

- für Wasserstoff, Methyl, Benzyl oder Pyridyl stehen
- für eine Gruppe der Formel

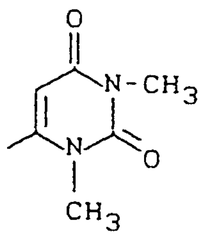

stehen oder

- für eine Gruppe der Formel $-COR^9$ oder $SO_2R^{10}$ stehen,

worin

$R^9$

- für Phenyl oder Ethoxy steht

$R^{10}$

- für Phenyl oder Naphthyl steht, das durch Fluor substituiert sein kann oder
- für Methyl steht
- für eine Gruppe der Formel

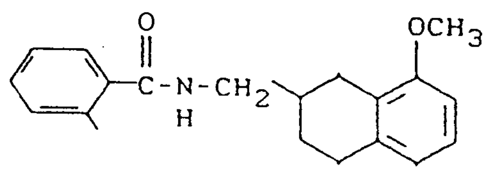

oder

$R^2$ und $R^3$ gemeinsam mit dem Stickstoffatom einen Heterocyclus der Formel

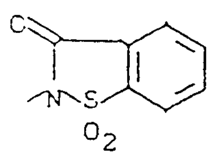 , 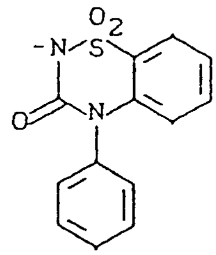 , 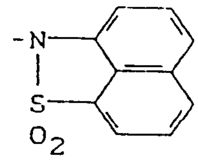 ,

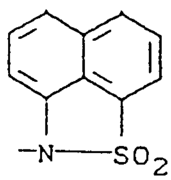 , 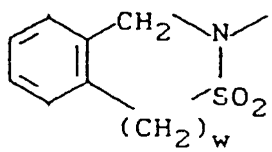 , 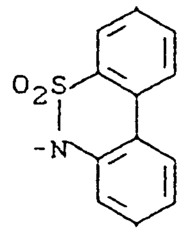 oder

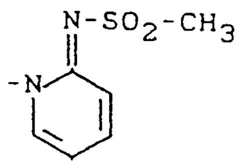 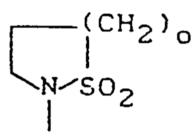

worin

w
- eine Zahl 0, 1 oder 2 bedeutet und

o
- eine Zahl 1 oder 2 bedeutet

bilden,

$R^1$
- Wasserstoff, Methyl, Propyl oder Benzyl bedeutet oder
- eine Gruppe $-(Y^1-Z^1)$ bedeutet, wobei $Y^1$ und $Z^1$ gleich oder verschieden zu Y und Z sein können und die oben angegebene Bedeutung von Y und Z haben

A und D für eine Gruppe der Formel $-CH_2-$, für Sauerstoff oder für den CH- oder N-Teil einer C=C-C=N-Doppelbindung stehen, mit der Maßgabe, daß entweder nur A oder nur D für Sauerstoff stehen darf,

B
- für eine Gruppe der Formel $>CH_2$ oder $>CH$ oder für den CH-Teil einer C=C- oder C=N-Doppelbindung steht,

C
- für eine Gruppe der Formel $>CH$ oder den C-Teil einer C=C oder C=N Doppelbindung steht,

E und F gleich oder verschieden sind und für Wasserstoff, Ethoxy, Methoxy, Fluor, Cyano oder eine Gruppe der Formel $-CONR^2R^3$ stehen, worin $R^2$ und $R^3$ Wasserstoff bedeuten,

E und F gemeinsam einen Cyclohexan oder Phenylring bilden,

und deren Salze.

**3.** (-)-2-{N-[4-(1,1-Dioxido-3-oxo-2,3-dihydro-benzisothiazol-2-yl)butyl]aminomethyl}chroman und dessen Salze nach Anspruch 1.

**4.** (+)-2-{N-[4-(1,1-Dioxido-3-oxo-2,3-dihydro-benzisothiazol-2-yl)butyl]aminomethyl}chroman und dessen Salze nach Anspruch 1.

5. (+)-2-{N-[4-(1,1-Dioxido-3-oxo-2,3-dihydro-benzisothiazol-2-yl)butyl]aminomethyl}-8-methoxy-chroman und dessen Salze nach Anspruch 1.

6. (-)-2-{N-[4-(1,1-Dioxido-3-oxo-2,3-dihydro-benzisothiazol-2-yl)-butyl]aminomethyl)-8-methoxy-chroman und dessen Salze nach Anspruch 1.

7. Substituierte Aminomethyltetraline sowie ihre heterocyclischen Analoga nach den Ansprüchen 1 bis 6, zur therapeutischen Behandlung.

8. Verfahren zur Herstellung von substituierten Aminomethyltetralinen sowie ihren heterocyclischen Analoga nach Anspruch 1 bis 6
dadurch gekennzeichnet, daß man
[A] Verbindungen der allgemeinen Formel (II)

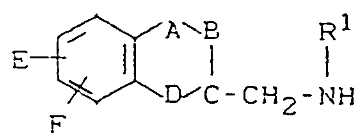
(II),

in welcher
A, B, C, D, E, F und $R^1$ die in Anspruch 1 angegebene Bedeutung haben,
(A1) mit Alkylierungsmitteln der Formel (III)

L-Y-Z (III),

in der
Y und Z die in Anspruch 1 angegebenen Bedeutungen haben, mit den Maßgaben, daß
Z nicht für Amino steht und
$R^2$ nicht für Wasserstoff steht, wenn
$R^3$ für Alkyl oder Aryl steht, und
L eine leaving group für Alkylierungsmittel bedeutet
umsetzt, oder
(A2) mit Aldehyden der Formel (IV)

OCH-$Y^2$-Z (IV),

in welcher
Z die oben angegebene Bedeutung hat und $Y^2$ eine um eine Methylengruppe verkürzte Alkylenkette Y ist,
reduktiv alkyliert,
oder
(A3) mit reaktiven Säurederivaten der allgemeinen Formel (V)

M-CO-$Y^2$-Z (V),

in welcher
$Y^2$ und Z die unter den Verfahrensvarianten [A1] angegebenen Bedeutungen haben und M eine leaving group für Alkylierungsmittel bedeutet,
umsetzt,
und die entsprechenden Säureamide mit Wasserstoff in Gegenwart eines Katalysators, mit Boranen oder mit komplexen Metallhydriden reduziert,
oder
(A4) mit Nitrilen der Formel

G-CN

in welcher
G für monochloriertes Niederalkyl($C_1$ bis $C_3$), Vinyl oder Niederalkyl($C_1$ bis $C_3$)-substituiertes Vinyl steht,
zu Verbindungen der Formel (VI) und (VII)

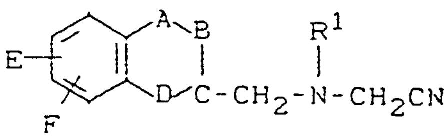

(VI)

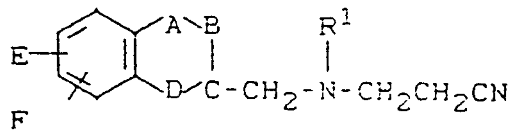

(VII),

worin
A, B, C, D, E, F und $R^1$ die oben angegebene Bedeutung haben,
alkyliert,
die erhaltenen Nitrile zu den Aminen (VIII) und (IX)

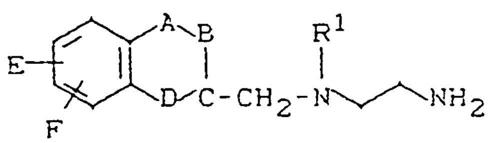

(VIII)

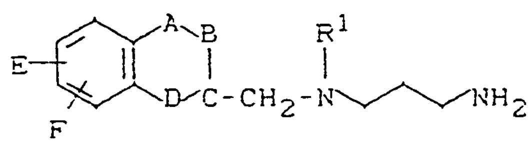

(IX),

worin
A, B, C, D, E, F und $R^1$ die oben genannte Bedeutung haben,
hydriert,
und diese in an sich bekannter Weise durch Alkylierung, reduktive Alkylierung, Acylierung, Reaktion mit Isocyanaten oder Sulfonylierungen umsetzt,
oder indem man
[B] Verbindungen der allgemeinen Formel (X)

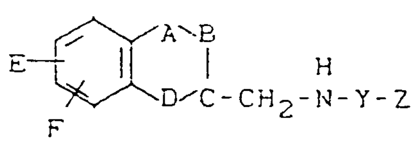

(X),

in welcher
A, B, C, D, E, F, Y und Z die oben angegebenen Bedeutungen haben,
mit den Maßgaben, daß
Z nicht für Amino steht und
$R^2$ nicht für Wasserstoff steht, wenn
$R^3$ für Alkyl oder Aryl steht,
(B1) mit Alkylierungsmitteln der Formel (XI)

$R^1$-L (XI),

worin

$R^1$ die oben angegebene Bedeutung hat und

L eine leaving group für Alkylierungsmittel bedeutet,

alkyliert,

oder

(B2) mit Aldehyden der Formel (XII)

$R^{14}$-CHO (XII),

in der $R^{14}$ eine um eine Methylengruppe verkürzter Rest $R^1$ bedeutet,
reduktiv alkyliert

oder

(B3) mit reaktiven Säurederivaten der allgemeinen Formel (XIII)

M-CO-$R^{14}$ (XIII)

in welcher

$R^{14}$ die oben angegebene Bedeutung hat und

M eine leaving group für Acylierungsmittel bedeutet,

umsetzt

und die entsprechenden Säureamide mit Wasserstoff in Gegenwart eines Katalysators oder mit komplexen Metallhydriden reduziert,

oder indem man

[C] Aldehyde der Formel (XIV)

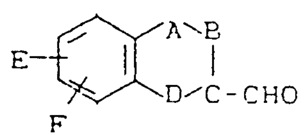 (XIV),

in der

A, B, C, D, E und F die oben angegebene Bedeutung haben,

mit Aminen der Formel (XV), (XVI) oder (XVII)

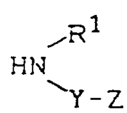

(XV)

$H_2N$-$R^1$ (XVI)

$H_2N$-Y-Z (XVII),

worin

$R^1$, Y und Z die oben angegebene Bedeutung haben

mit den Maßgaben, daß

Z nicht für Amino steht und

$R^2$ nicht für Wasserstoff steht, wenn

$R^3$ für Alkyl oder Aryl steht,

in an sich bekannter Weise reduktiv aminiert,

oder indem man

[D] Verbindungen der allgemeinen Formel (XVIII)

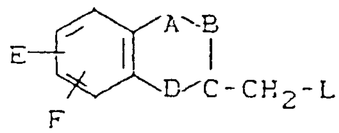

(XVIII),

in welcher
A, B, C, D, E und F und X die oben angegebene Bedeutung haben und
L eine leaving group für Alkylierungsmittel bedeutet,
mit Aminen der Formeln

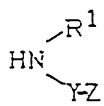

(XV)

$H_2N-R^1$ (XVI)

$H_2N-Y-Z$ (XVII),

worin
$R^1$, Y und Z die oben genannte Bedeutung haben,
mit den Maßgaben, daß
Z nicht für Amino steht und
$R^2$ nicht für Wasserstoff steht, wenn
$R^3$ für Alkyl oder Aryl steht,
umsetzt,
oder mit einem Alkaliazid umsetzt und anschließend die Azidofunktion zu einer Aminofunktion reduziert,
diese in an sich bekannter Weise durch Alkylierung, reduktive Alkylierung, Acylierung, Reaktion mit Isocyanaten oder Sulfonierungen umsetzt,
oder indem man
[E] reaktive Carbonsäurederivate der Formel (XIX)

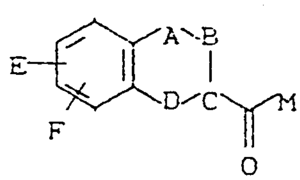

(XIX),

worin
A, B, C, D, E und F die oben angegebene Bedeutung haben, und
M eine leaving group für Acylierungsmittel bedeutet,
mit Aminen der Formeln

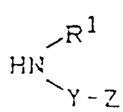

(XV)

$H_2N-R^1$ (XVI)

$H_2N-Y-Z$ (XVII),

worin
$R^1$, Y und Z die oben genannte Bedeutung haben,
mit den Maßgaben, daß
Z nicht für Amino steht, und
$R^2$ nicht für Wasserstoff steht, wenn
$R^3$ für Alkyl oder Aryl steht,
umsetzt,
und die so erhaltenen Amide der Formeln (XX) und (XXI)

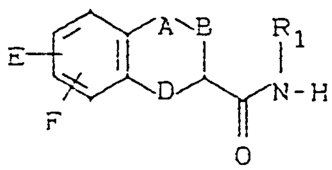

(XX)

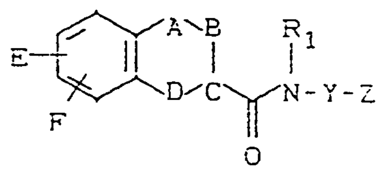

(XXI),

in welcher
A, B, C, D, E, F, $R^1$, Y und Z die oben angegebene Bedeutung haben,
katalytisch mit Wasserstoff mit komplexen Metallhydriden oder mit Boranen reduziert,
oder indem man
[F] Verbindungen der allgemeinen Formel (XXII)

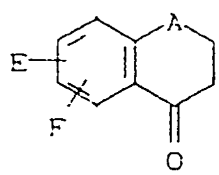

(XXII),

in der A, E und F die oben angegebene Bedeutung haben mit Formaldehyd und Aminen der Formel (XV)

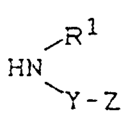

(XV),

worin
$R^1$, Y und Z die oben angegebene Bedeutung haben.
mit den Maßgaben, daß
Z nicht für Amino steht und
$R^2$ nicht für Wasserstoff steht, wenn
$R^3$ für Alkyl oder Aryl steht,
umsetzt und die erhaltenen Zwischenprodukte der allgemeinen Formel (XXIII)

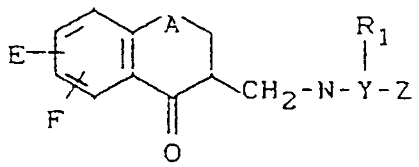

(XXIII),

in welcher
A, E, F, $R^1$, Y und Z die oben angegebene Bedeutung haben,
durch Reduktion der Carbonylfunktion oder durch partielle Reduktion der Carbonylfunktion zur Alkoholfunktion, anschließender Eliminierung von Wasser und gegebenenfalls eine Hydrierung der C=C-Doppelbindung mit Wasserstoff umsetzt.

**9.** Arzneimittel, enthaltend substituierte Aminomethyltetraline und/oder ihre heterocyclischen Analoga nach den Ansprüchen 1 bis 6.

**10.** Arzneimittel nach Anspruch 9 zur Behandlung von Erkrankungen des zentralen Nervensystems.

**11.** Arzneimittel nach Anspruch 9 und 10 zur Behandlung von Depressionen und cerebralen Infarktgeschehen.

**12.** Verwendung von substituierten Aminomethyltetralinen und/oder ihren heterocyclischen Analoga nach den Ansprüchen 1 bis 6 zur Herstellung von Arzneimitteln.

**13.** Verwendung nach Anspruch 12 zur Herstellung von Arzneimitteln zur Behandlung von Erkrankungen des zentralen Nervensystems.

**Patentansprüche für folgenden Vertragsstaat : ES**

**1.** Verfahren zur Herstellung von substituierten Aminomethyltetralinen sowie ihren heterocyclischen Analoga der allgemeinen Formel

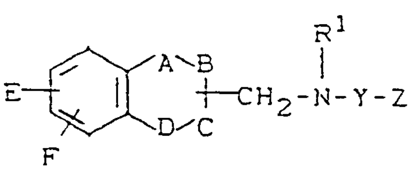

(I),

in welcher
Y
- eine geradkettige Alkylenkette mit 2- 5 Kohlenstoffatomen bedeutet,

Z
- eine Gruppe der Formel

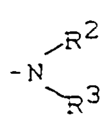

bedeutet,
wobei
$R^2$ und $R^3$ gleich oder verschieden sind und
- für Wasserstoff, Methyl, Ethyl, Propyl, oder
- für Phenyl, Benzyl oder Pyridyl stehen, das gegebenenfalls substituiert ist durch Fluor, Chlor, Methyl oder Methoxy

- für eine Gruppe der Formel

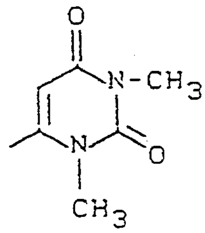

stehen oder

- für eine Gruppe der Formel $-COR^9$ oder $-SO_2R^{10}$ stehen,

worin

$R^9$

- für Methyl, Ethyl oder Ethoxy steht oder
- für Phenyl oder Benzyl steht, gegebenenfalls substituiert durch Methyl, Methoxy, Fluor oder Chlor

$R^{10}$

- für Methyl, Ethyl oder Propyl steht,
- für Phenyl, Naphthyl oder Benzyl steht, das gegebenenfalls durch Fluor oder Chlor substitutiert ist oder
- für eine Gruppe der Formel

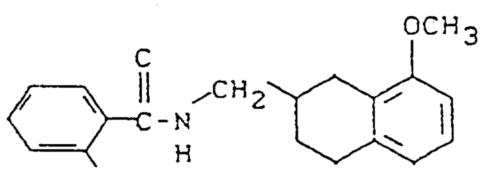

steht

$R^2$ und $R^3$ gemeinsam mit dem Stickstoffatom einen Heterocyclus aus der Reihe

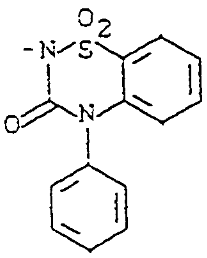 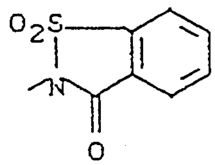 , 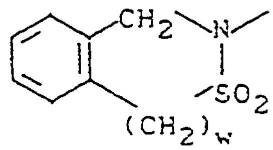

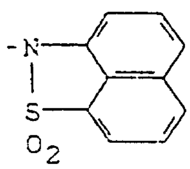 , 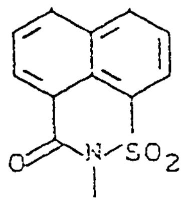 , 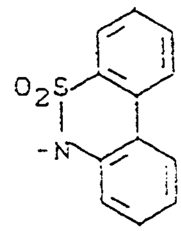

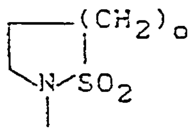 oder 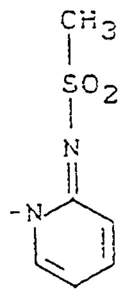

bilden,
wobei

w
- eine Zahl 0, 1 oder 2 bedeutet und

o
- eine Zahl 1 oder 2 bedeutet

$R^1$
- Wasserstoff, Ethyl, Methyl, Propyl oder Benzyl bedeutet, oder
- die Gruppe $-(Y^1-Z^1)$ bedeutet, wobei $Y^1$ und $Z^1$ gleich oder verschieden zu Y und Z sein können und die oben angegebene Bedeutung von Y und Z haben,

A und D für eine Gruppe der Formel $-CH_2-$, für Sauerstoff oder für den CH- oder N-Teil einer C=C-C=N-Doppelbindung stehen, mit der Maßgabe, daß entweder nur A oder nur D für Sauerstoff stehen darf,

B
- für eine Gruppe der Formel $>CH_2$ oder $\geqslant CH$ oder für den CH-Teil einer C=C- oder C=N-Doppelbindung steht,

C
- für eine Gruppe der Formel $\geqslant CH$ oder den C-Teil einer C=C oder C=N Doppelbindung steht,

E und F gleich oder verschieden sind und für Wasserstoff, Ethoxy, Methoxy, Fluor, Cyano oder eine Gruppe der Formel $-CONR^2R^3$ stehen, worin $R^2$ und $R^3$ Wasserstoff bedeuten,

E und F gemeinsam einen Phenyl oder Cyclohexanring bilden,
und deren Salze,
dadurch gekennzeichnet, daß man

[A] Verbindungen der allgemeinen Formel (II)

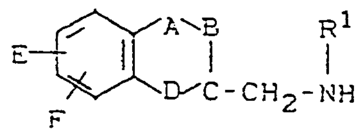

(II),

in welcher
A, B, C, D, E, F und $R^1$ die angegebene Bedeutung haben,
(A1) mit Alkylierungsmitteln der Formel (III)

L-Y-Z (III),

in der
Y und Z die oben angegebenen Bedeutungen haben,
mit den Maßgaben, daß
Z nicht für Amino steht und
$R^2$ nicht für Wasserstoff steht, wenn
$R^3$ für Alkyl oder Aryl steht, und
L eine leaving group für Alkylierungsmittel bedeutet
umsetzt, oder
(A2) mit Aldehyden der Formel (IV)

OCH-$Y^2$-Z (IV),

in welcher
Z die oben angegebene Bedeutung hat und $Y^2$ eine um eine Methylengruppe verkürzte Alkylenkette Y ist,
reduktiv alkyliert,
oder
(A3) mit reaktiven Säurederivaten der allgemeinen Formel (V)

M-CO-$Y^2$-Z (V),

in welcher
$Y^2$ und Z die unter den Verfahrensvarianten [A1] angegebenen Bedeutungen haben und M eine leaving group für Alkylierungsmittel bedeutet,
umsetzt,
und die entsprechenden Säureamide mit Wasserstoff in Gegenwart eines Katalysators, mit Boranen oder mit komplexen Metallhydriden reduziert,
oder
(A4) mit Nitrilen der Formel

G-CN

in welcher
G für monochloriertes Niederalkyl($C_1$ bis $C_3$), Vinyl oder Niederalkyl($C_1$ bis $C_3$)-substituiertes Vinyl steht,
zu Verbindungen der Formel (VI) und (VII)

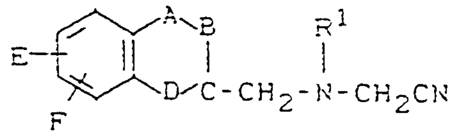

(VI)

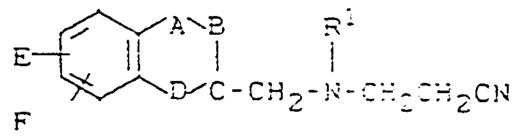

(VII),

worin
A, B, C, D, E, F und $R^1$ die oben angegebene Bedeutung haben,
alkyliert,
die erhaltenen Nitrile zu den Aminen (VIII) und (IX)

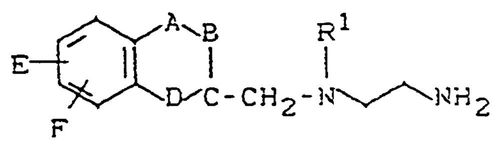

(VIII)

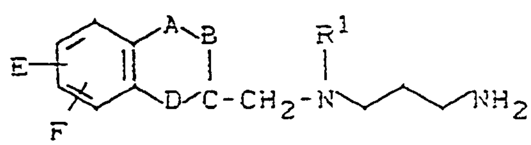

(IX),

worin
A, B, C, D, E, F und $R^1$ die oben genannte Bedeutung haben,
hydriert,
und diese in an sich bekannter Weise durch Alkylierung, reduktive Alkylierung, Acylierung, Reaktion mit Isocyanaten oder Sulfonylierungen umsetzt,
oder indem man
[B] Verbindungen der allgemeinen Formel (X)

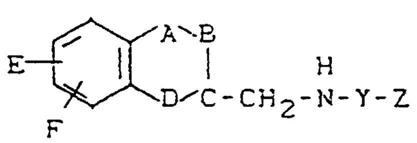

(X),

in welcher
A, B, C, D, E, F, Y und Z die oben angegebenen Bedeutungen haben,
mit den Maßgaben, daß
Z nicht für Amino steht und
$R^2$ nicht für Wasserstoff steht, wenn
$R^3$ für Alkyl oder Aryl steht,
(B1) mit Alkylierungsmitteln der Formel (XI)

$R^1$-L (XI),

worin
- $R^1$ die oben angegebene Bedeutung hat und
- L eine leaving group für Alkylierungsmittel bedeutet,
  alkyliert,

oder

(B2) mit Aldehyden der Formel (XII)

$R^{14}$-CHO (XII),

in der $R^{14}$ eine um eine Methylengruppe verkürzter Rest $R^1$ bedeutet, reduktiv alkyliert oder
(B3) mit reaktiven Säurederivaten der allgemeinen Formel (XIII)

M-CO-$R^{14}$ (XIII)

in welcher
$R^{14}$ die oben angegebene Bedeutung hat und
M eine leaving group für Acylierungsmittel bedeutet,
umsetzt
und die entsprechenden Säureamide mit Wasserstoff in Gegenwart eines Katalysators oder mit komplexen Metallhydriden reduziert,
oder indem man
[C] Aldehyde der Formel (XIV)

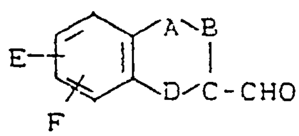

(XIV),

in der
A, B, C, D, E und F die oben angegebene Bedeutung haben,
mit Aminen der Formel (XV), (XVI) oder (XVII)

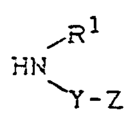

(XV)

$H_2N$-$R^1$ (XVI)

$H_2N$-Y-Z (XVII),

worin
$R^1$, Y und Z die oben angegebene Bedeutung haben
mit den Maßgaben, daß
Z nicht für Amino steht und
$R^2$ nicht für Wasserstoff steht, wenn
$R^3$ für Alkyl oder Aryl steht,
in an sich bekannter Weise reduktiv aminiert,
oder indem man
[D] Verbindungen der allgemeinen Formel (XVIII)

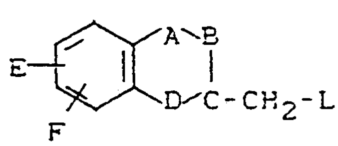

(XVIII),

in welcher
A, B, C, D, E und F und X die oben angegebene Bedeutung haben und
L eine leaving group für Alkylierungsmittel bedeutet,
mit Aminen der Formeln

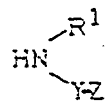

(XV)

$H_2N-R^1$ (XVI)

$H_2N-Y-Z$ (XVII),

worin
$R^1$, Y und Z die oben genannte Bedeutung haben,
mit den Maßgaben, daß
Z nicht für Amino steht und
$R^2$ nicht für Wasserstoff steht, wenn
$R^3$ für Alkyl oder Aryl steht,
umsetzt,
oder mit einem Alkaliazid umsetzt und anschließend die Azidofunktion zu einer Aminofunktion reduziert,
diese in an sich bekannter Weise durch Alkylierung, reduktive Alkylierung, Acylierung, Reaktion mit Isocyanaten oder Sulfonierungen umsetzt,
oder indem man
[E] reaktive Carbonsäurederivate der Formel (XIX)

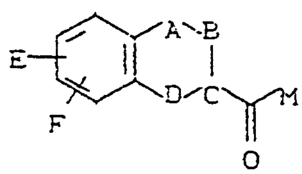

(XIX),

worin
A, B, C, D, E und F die oben angegebene Bedeutung haben, und
M eine leaving group für Acylierungsmittel bedeutet,
mit Aminen der Formeln

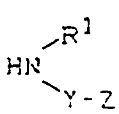

(XV)

$H_2N-R^1$ (XVI)

$H_2N-Y-Z$ (XVII),

worin

$R^1$, Y und Z die oben genannte Bedeutung haben,
mit den Maßgaben, daß
Z nicht für Amino steht, und
$R^2$ nicht für Wasserstoff steht, wenn
$R^3$ für Alkyl oder Aryl steht,
umsetzt,
und die so erhaltenen Amide der Formeln (XX) und (XXI)

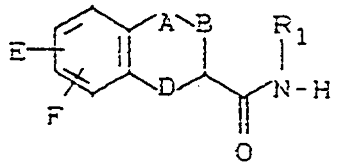

(XX)

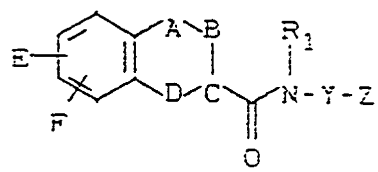

(XXI),

in welcher
A, B, C, D, E, F, $R^1$, Y und Z die oben angegebene Bedeutung haben,
Katalytisch mit Wasserstoff mit komplexen Metallhydriden oder mit Boranen reduziert,
oder indem man
[F] Verbindungen der allgemeinen Formel (XXII)

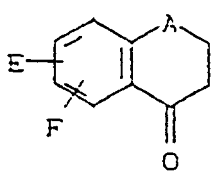

(XXII),

in der A, E und F die oben angegebene Bedeutung haben mit Formaldehyd und Aminen der Formel (XV)

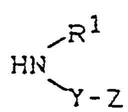

(XV),

worin
$R^1$, Y und Z die oben angegebene Bedeutung haben.
mit den Maßgaben, daß
Z nicht für Amino steht und
$R^2$ nicht für Wasserstoff steht, wenn
$R^3$ für Alkyl oder Aryl steht,
umsetzt und die erhaltenen Zwischenprodukte der allgemeinen Formel (XXIII)

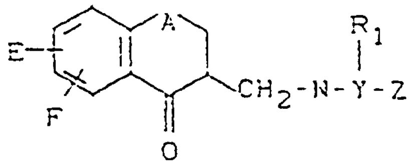

(XXIII),

in welcher
A, E, F, $R^1$, Y und Z die oben angegebene Bedeutung haben,

durch Reduktion der Carbonylfunktion oder durch partielle Reduktion der Carbonylfunktion zur Alkoholfunktion, anschließender Eliminierung von Wasser und gegebenenfalls eine Hydrierung der C = C-Doppelbindung mit Wasserstoff umsetzt.

**2.** Verfahren nach Anspruch 1 zur Herstellung von (-)-2-{N-[4-(1,1-Dioxido-3-oxo-2,3-dihydro-benzisothiazol-2-yl)butyl)aminomethyl}chroman und dessen Salze.

**3.** Verfahren nach Anspruch 1 zur Herstellung von ( + )-2-{N-[4-(1,1-Dioxido-3-oxo-2,3-dihydro-benzisothiazol-2-yl)butyl]aminomethyl}chroman und dessen Salze.

**4.** Verfahren nach Anspruch 1 zur Herstellung von ( + )-2-{N-[4-(1,1-Dioxido-3-oxo-2,3-dihydro-benzisothiazol-2-yl)butyl]aminomethyl}-8-methoxy-chroman und dessen Salze.

**5.** Verfahren nach Anspruch 1 zur Herstellung von (-)-2-{N-[4-(1,1-Dioxido-3-oxo-2,3-dihydro-benzisothiazol-2-yl)-butyl]aminomethyl}-8-methoxy-chroman und dessen Salze.

**6.** Verwendung von substituierten Aminomethyltetralinen und/oder ihren heterocyclischen Analoga nach den Ansprüchen 1 bis 5 zur Herstellung von Arzneimitteln.

**7.** Verwendung nach Anspruch 6 zur Herstellung von Arzneimitteln zur Behandlung von Erkrankungen des zentralen Nervensystems.

**Claims**
**Claims for the following Contracting States : AT, BE, CH, DE, FR, GB, GR, IT, LI, LU, NL, SE**

**1.** Substituted aminomethyltetralins and their heterocyclic analogues of the general formula

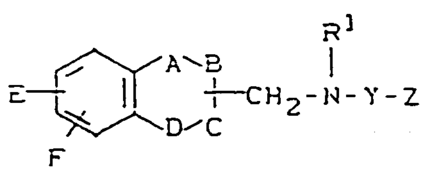

(I),

in which

Y - denotes a straight-chain alkylene chain having 2-5 carbon atoms,

Z - denotes a group of the formula

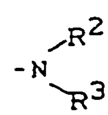

where

$R^2$ and $R^3$ are identical or different and
- represent hydrogen, methyl, ethyl, propyl or
- represent phenyl, benzyl or pyridyl, which is optionally substituted by fluorine, chlorine, methyl or methoxy

- represent a group of the formula

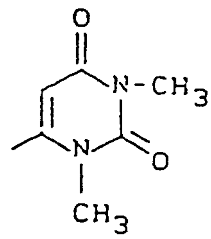

or

- represent a group of the formula $-COR^9$ or $-SO_2R^{10}$,

in which

$R^9$

- represents methyl, ethyl or ethoxy or
- represents phenyl or benzyl, optionally substituted by methyl, methoxy, fluorine or chlorine,

$R^{10}$

- represents methyl, ethyl or propyl,
- represents phenyl, naphthyl or benzyl, which is optionally substituted by fluorine or chlorine or
- represents a group of the formula

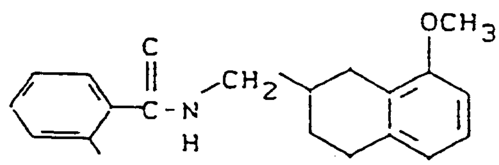

or

$R^2$ and $R^3$ together with the nitrogen atom form a heterocycle from the series comprising

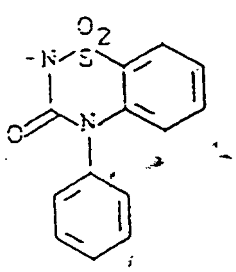

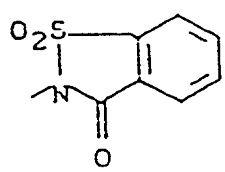 ,

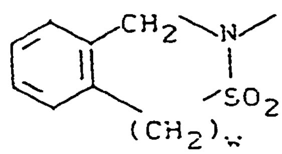

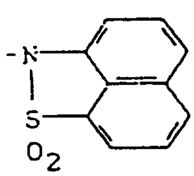 ,

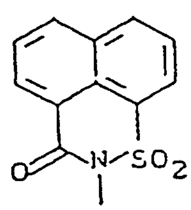 ,

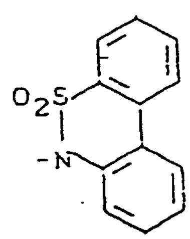

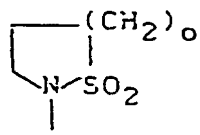

or

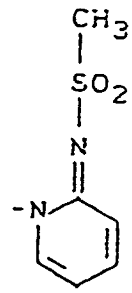

where

w denotes a number 0, 1 or 2 and

o denotes a number 1 or 2

$R^1$

- denotes hydrogen, ethyl, methyl, propyl or benzyl or
- denotes the group -($Y^1$-$Z^1$), where $Y^1$ and $Z^1$ may be identical or different to Y and Z and have the abovementioned meaning of Y and Z,

A and D represent a group of the formula $-CH_2-$, oxygen or the CH or N part of a C=C or C=N double bond, with the proviso that either only A or only D may represent oxygen,

B represents a group of the formula $>CH_2$ or $>CH$ or the CH part of a C=C or C=N double bond,

C

- represents a group of the formula $>CH$ or the C part of a C=C or C=N double bond,

E and F are identical or different and represent hydrogen, ethoxy, methoxy, fluorine, cyano or a group of the formula $-CONR^2R^3$ in which $R^2$ and $R^3$ denote hydrogen,

or

E and F together form a phenyl or cyclohexane ring,

and their salts.

**2.** Substituted aminomethyltetralins and their hterocyclic analogues according to Claim 1 in which

Y

- denotes a straight-chain or alkylene chain having 2-5 carbon atoms,

Z

- denotes a group of the formula

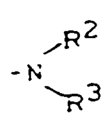

in which

$R^2$ and $R^3$ are identical or different and

- represent hydrogen, methyl, benzyl or pyridyl

- represent a group of the formula

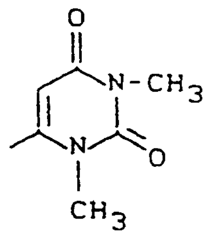

or

- represent a group of the formula $-COR^9$ or $SO_2R^{10}$,

in which

$R^9$

- represents phenyl or ethoxy

$R^{10}$

- represents phenyl or naphthyl which may be substituted by fluorine or
- represents methyl or
- represents a group of the formula

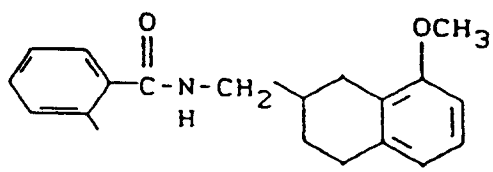

or $R^2$ and $R^3$ together with the nitrogen atom form a heterocycle of the formula

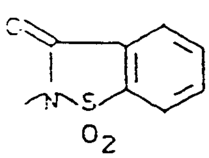
,
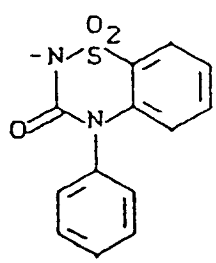
,
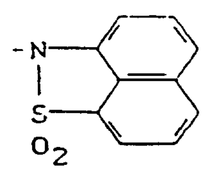

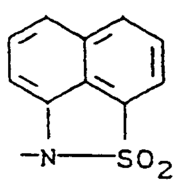
,
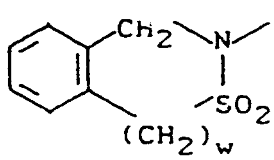
,
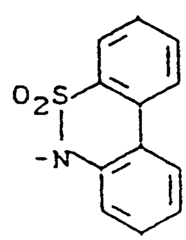

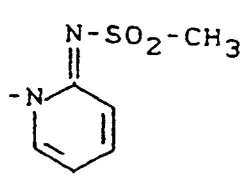
or
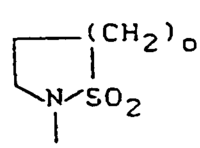

in which

w
- denotes a number 0, 1 or 2 and

o
- denotes a number 1 or 2 and

$R^1$
- denotes hydrogen, methyl, propyl or benzyl or
- denotes a group $-(Y^1-Z^1)$, where $Y^1$ and $Z^1$ may be identical or different to Y and Z and have the abovementioned meaning of Y and Z

A and D represent a group of the formula $-CH_2-$, oxygen or the CH or N part of a C=C or C=N double bond, with the proviso that either only A or only D may represent oxygen,

B represents a group of the formula $>CH_2$ or $>CH$ or the CH part of a C=C or C=N double bond,

C
- represents a group of the formula $>CH$ or the C part of a C=C or C=N double bond,

E and F are identical or different and represent hydrogen, ethoxy, methoxy, fluorine, cyano or a group of the formula $-CONR^2R^3$, in which $R^2$ and $R^3$ denote hydrogen

or

E and F together form a cyclohexane or phenyl ring, and their salts.

**3.** (-)-2-{N-[4-(1,1-Dioxido-3-oxo-2,3-dihydro-benzisothiazol-2-yl)butyl]aminomethyl)chroman and its salts according to Claim 1.

**4.** (+)-2-{N-[4-(1,1-Dioxido-3-oxo-2,3-dihydro-benzisothiazol-2-yl)butyl]aminomethyl}chroman and its salts according to Claim 1.

**5.** (+)-2-{N-[4-(1,1-Dioxido-3-oxo-2,3-dihydro-benzisothiazol-2-yl)butyl]aminomethyl}-8-methoxy-chroman and its salts according to Claim 1.

**6.** (-)-2-{N-[4-(1,1-Dioxido-3-oxo-2,3-dihydro-benzisothiazol-2-yl)-butyl]aminomethyl}-8-methoxy-chroman and its salts according to Claim 1.

**7.** Substituted aminomethyltetralins and their heterocyclic analogues according to Claims 1 to 6 for therapeutic treatment.

**8.** Process for the preparation of substituted aminomethyltetralins and their heterocyclic analogues according to Claims 1 to 6 characterized in that

[A] compounds of the general formula (II)

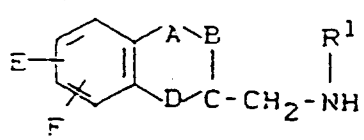

(II)

in which

A, B, C, D, E, F and $R^1$ have the meaning indicated in Claim 1,

(A1) are reacted with alkylating agents of the formula (III)

L-Y-Z (III),

in which

Y and Z have the meanings indicated in Claim 1,

with the provisos that
Z does not represent amino and
$R^2$ does not represent hydrogen if
$R^3$ represents alkyl or aryl, and
L denotes a leaving group for alkylating agents
or
(A2) are reductively alkylated using aldehydes of the formula (IV)

$OCH-Y^2-Z$ (IV),

in which
Z has the abovementioned meaning and $Y^2$ is an alkylene chain Y shortened by one methylene group,
or
(A3) are reacted with reactive acid derivatives of the general formula (V)

$M-CO-Y^2-Z$ (V),

in which
$Y^2$ and Z have the meanings indicated under process variant [A1] and M denotes a leaving group for alkylating agents,
and the corresponding acid amides are reduced using hydrogen in the presence of a catalyst, using boranes or using complex metal hydrides,
or
(A4) are alkylated using nitriles of the formula

G - CN

in which
G represents monochlorinated lower alkyl ($C_1$ to $C_3$), vinyl or lower alkyl ($C_1$ to $C_3$)-substituted vinyl, to give compounds of the formulae (VI) and (VII)

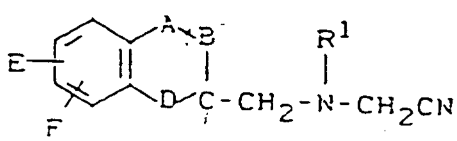

(VI)

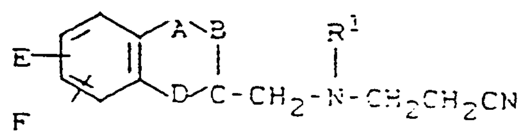

(VII)

in which
A, B, C, D, E, F and $R^1$ have the abovementioned meaning,
the nitriles obtained are hydrogenated to give the amines (VIII) and (IX)

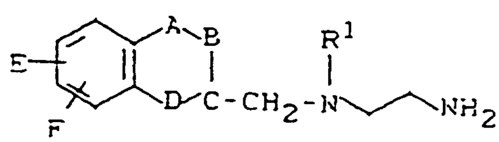

(VIII)

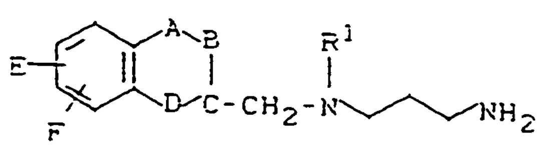

(IX)

in which
A, B, C, D, E, F and $R^1$ have the abovementioned meaning,
and these are reacted in a manner known per se by alkylation, reductive alkylation, acylation, reaction with isocyanates or sulphonylations,
or in that
[B] compounds of the general formula (X)

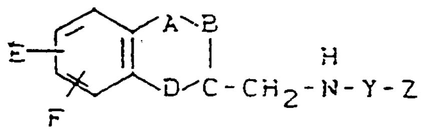

in which
A, B, C, D, E, F, Y and Z have the abovementioned meanings,
with the provisos that
Z does not represent amino and
$R^2$ does not represent hydrogen if
$R^3$ represents alkyl or aryl,
(B1) are alkylated using alkylating agents of the formula (XI)

$R^1$-L (XI)

in which
$R^1$ has the abovementioned meaning and
L denotes a leaving group for alkylating agents,
or
(B2) are reductively alkylated using aldehydes of the formula (XII)

$R^{14}$-CHO (XII)

in which $R^{14}$ denotes a radical $R^1$ shortened by one methylene group,
or
(B3) are reacted with reactive acid derivatives of the general formula (XIII)

M-CO-$R^{14}$ (XIII)

in which
$R^{14}$ has the abovementioned meaning and
M denotes a leaving group for acylating agents,
and the corresponding acid amides are reduced using hydrogen in the presence of a catalyst or using complex metal hydrides,
or in that
[C] aldehydes of the formula (XIV)

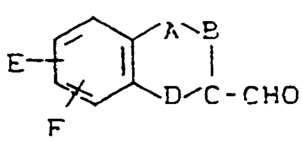

**(XIV)**

in which
A, B, C, D, E and F have the abovementioned meaning,
are reductively aminated using amines of the formula (XV), (XVI) or (XVII)

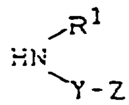

(XV)

$H_2N-R^1$ (XVI)

$H_2N-Y-Z$ (XVII)

in which
$R^1$, Y and Z have the abovementioned meaning
with the provisos that
Z does not represent amino and
$R^2$ does not represent hydrogen if
$R^3$ represents alkyl or aryl,
in a manner known per se,
or in that
[D] compounds of the general formula (XVIII)

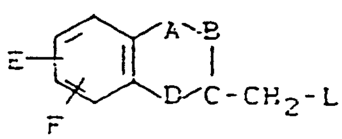

(XVIII),

in which
A, B, C, D, E and F and X have the abovementioned meaning and
L denotes a leaving group for alkylating agents,
are reacted with amines of the formulae

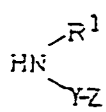

(XV)

$H_2N-R^1$ (XVI)

$H_2N-Y-Z$ (XVII),

in which
$R^1$, Y and Z have the abovementioned meaning,
with the provisos that
Z does not represent amino and
$R^2$ does not represent hydrogen if
$R^3$ represents alkyl or aryl,
or reacted with an alkali metal azide and the azido function is subsequently reduced to an amino function,
and this is reacted in a manner known per se by alkylation, reductive alkylation, acylation, reaction with isocyanates or sulphonylations,
or in that

[E] reactive carboylic acid derivatives of the formula (XIX)

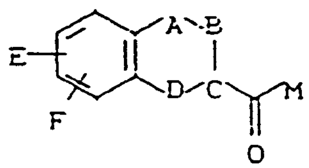

(XIX)

in which
A, B, C, D, E and F have the abovementioned meaning, and
M denotes a leaving group for acylating agents,
are reacted with amines of the formulae

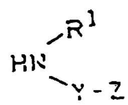

(XV)

$H_2N\text{-}R^1$ (XVI

$H_2N\text{-}Y\text{-}Z$ (XVII)

in which
$R^1$, Y and Z have the abovementioned meaning,
with the provisos that
Z does not represent amino and
$R^2$ does not represent hydrogen if
$R^3$ represents alkyl or aryl,
and the amides thus obtained of the formulae (XX) and (XXI)

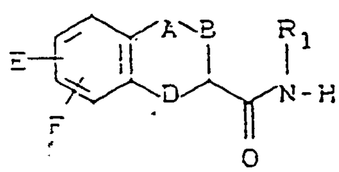

(XX)

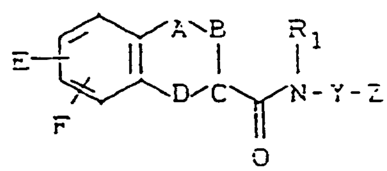

(XXI)

in which
A, B, C, D, E, F, $R^1$, Y and Z have the abovementioned meaning,
are catalytically reduced using hydrogen, using complex metal hydrides or using boranes,
or in that

[F] compounds of the general formula (XXII)

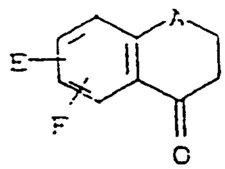

(XXII)

in which A, E and F have the abovementioned meaning are reacted with formaldehyde and amines of the formula (XV)

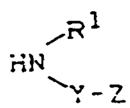

(XV),

in which
$R^1$, Y and Z have the abovementioned meaning,
with the provisos that
Z does not represent amino and
$R^2$ does not represent hydrogen if
$R^3$ represents alkyl or aryl,
and the intermediates obtained of the general formula (XXIII)

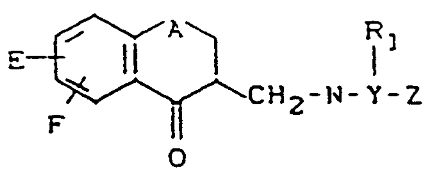

(XXIII)

in which
A, E, F, $R^1$, Y and Z have the abovementioned meaning,
are reacted by reduction of the carbonyl function or by partial reduction of the carbonyl function to the alcohol function, subsequent elimination of water and, if desired, hydrogenation of the C = C double bond using hydrogen.

**9.** Medicaments, containing substituted aminomethyltetralins and/or their heterocyclic analogues according to Claims 1 to 6.

**10.** Medicaments according to Claim 9 for the treatment of disorders of the central nervous system.

**11.** Medicaments according to Claims 9 and 10 for the treatment of depression and cerebral infarct.

**12.** Use of substituted aminomethyltetralins and/or their heterocyclic analogues according to Claims 1 to 6 for the production of medicaments.

**13.** Use according to Claim 12 for the production of medicaments for the treatment of disorders of the central nervous system.

**Claims for the following Contracting State : ES**

**1.** Process for the preparation of substituted aminomethyltetralins and their heterocyclic analogues of the general formula

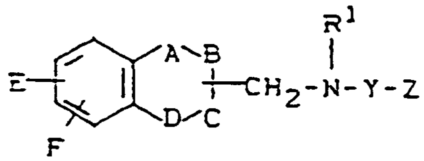

(I),

in which

Y - denotes a straight-chain alkylene chain having 2-5 carbon atoms,

Z - denotes a group of the formula

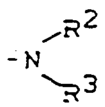

where

$R^2$ and $R^3$ are identical or different and

- represent hydrogen, methyl, ethyl, propyl or
- represent phenyl, benzyl or pyridyl, which is optionally substituted by fluorine, chlorine, methyl or methoxy
- represent a group of the formula

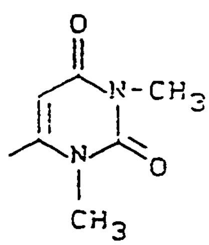

or

- represent a group of the formula $-COR^9$ or $-SO_2R^{10}$,

in which

$R^9$
- represents methyl, ethyl or ethoxy or
- represents phenyl or benzyl, optionally substituted by methyl, methoxy, fluorine or chlorine,

$R^{10}$
- represents methyl, ethyl or propyl,
- represents phenyl, naphthyl or benzyl, which is optionally substituted by fluorine or chlorine or
- represents a group of the formula

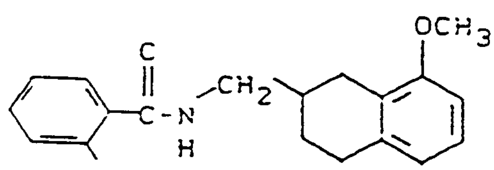

.

or

$R^2$ and $R^3$ together with the nitrogen atom form a heterocycle from the series comprising

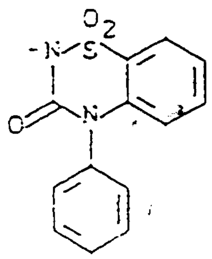

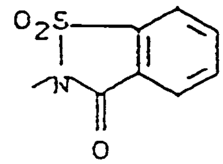

,

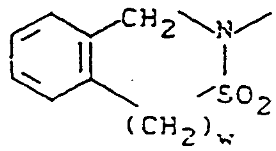

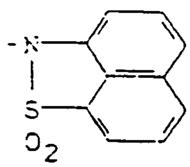

,

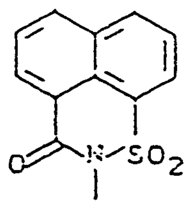

,

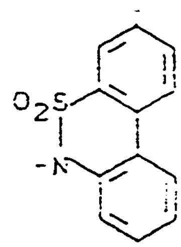

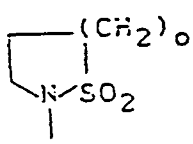

or

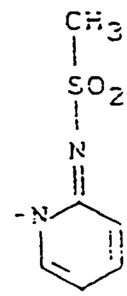

where

w
- denotes a number 0, 1 or 2 and

o
- denotes a number 1 or 2

$R^1$
- denotes hydrogen, ethyl, methyl, propyl or benzyl or
- denotes the group $-(Y^1-Z^1)$, where $Y^1$ and $Z^1$ may be identical or different to Y and Z and have the abovementioned meaning of Y and Z,

A and D represent a group of the formula $-CH_2-$, oxygen or the CH or N part of a C = C or C = N double bond with the proviso that either only A or only D may represent oxygen,

B
- represents a group of the formula $>CH_2$ or $\geqslant CH$ or the CH part of a C = C or C = N double bond,

C
- represents agroup of the formula $\geqslant CH$ or the C part of a C = C or C = N double bond,

E and F are identical or different and represent hydrogen, ethoxy, methoxy, fluorine, cyano or a group of the formula $-CONR^2R^3$ in which $R^2$ and $R^3$ denote hydrogen
or
E and F together form a phenyl or cyclohexane ring,
and their salts,
characterized in that

[A] compounds of the general formula (II)

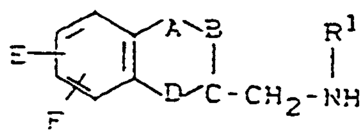

(II)

in which
A, B, C, D, E, F and $R^1$ have the meaning mentioned,
(A1) are reacted with alkylating agents of the formula (III)

L-Y-Z (III),

in which
Y and Z have the abovementioned meanings,
with the provisos that
Z does not represent amino and
$R^2$ does not represent hydrogen if
$R^3$ represents alkyl or aryl, and
L denotes a leaving group for alkylating agents
or
(A2) are reductively alkylated using aldehydes of the formula (IV)

OCH-$Y^2$-Z (IV),

in which
Z has the abovementioned meaning and $Y^2$ is an alkylene chain Y shortened by one methylene group,
or
(A3) are reacted with reactive acid derivatives of the general formula (V)

M-CO-$Y^2$-Z (V),

in which
$Y^2$ and Z have the meanings indicated under process variant [A1] and M denotes a leaving group for alkylating agents,
and the corresponding acid amides are reduced using hydrogen in the presence of a catalyst, using boranes or using complex metal hydrides,
or
(A4) are alkylated using nitriles of the formula

G - CN

in which
G represents monochlorinated lower alkyl ($C_1$ to $C_3$), vinyl or lower alkyl ($C_1$ to $C_3$)-substituted vinyl, to give compounds of the formulae (VI) and (VII)

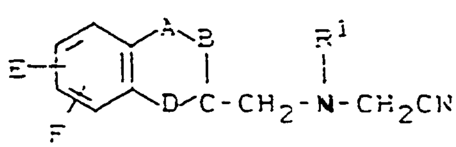

(VI)

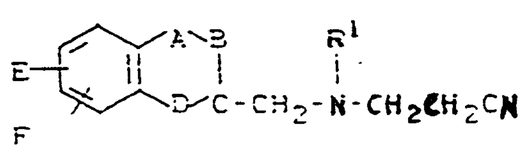

(VII)

in which

A, B, C, D, E, F and $R^1$ have the abovementioned meaning,
the nitriles obtained are hydrogenated to give the amines (VIII) and (IX)

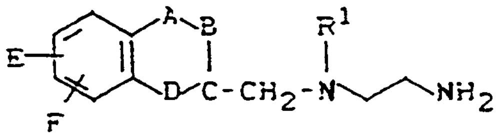

(VIII)

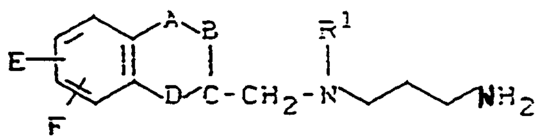

(IX)

in which
A, B, C, D, E, F and $R^1$ have the abovementioned meaning,
and these are reacted in a manner known per se by alkylation, reductive alkylation, acylation, reaction with isocyanates or sulphonylations,
or in that
[B] compounds of the general formula (X)

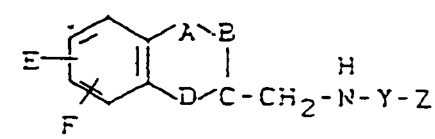

(X),

in which
A, B, C, D, E, F, Y and Z have the abovementioned meanings,
with the provisos that
Z does not represent amino and
$R^2$ does not represent hydrogen if
$R^3$ represents alkyl or aryl,
(B1) are alkylated using alkylating agents of the formula (XI)

$R^1$-L (XI)

in which
$R^1$ has the abovementioned meaning and
L denotes a leaving group for alkylating agents,
or
(B2) are reductively alkylated using aldehydes of the formula (XII)

$R^{14}$-CHO (XII)

in which $R^{14}$ denotes a radical $R^1$ shortened by one methylene group,
or
(B3) are reacted with reactive acid derivatives of the general formula (XIII)

M-CO-$R^{14}$ (XIII)

in which
$R^{14}$ has the abovementioned meaning and
M denotes a leaving group for acylating agents,
and the corresponding acid amides are reduced using hydrogen in the presence of a catalyst or using complex metal hydrides,

or in that
[C] aldehydes of the formula (XIV)

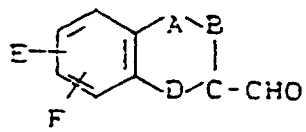

(XIV)

in which
A, B, C, D, E and F have the abovementioned meaning,
are reductively aminated using amines of the formula (XV), (XVI) or (XVII)

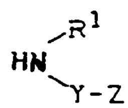

(XV)

$H_2N-R^1$ (XVI)

$H_2N-Y-Z$ (XVII)

in which
$R^1$, Y and Z have the abovementioned meaning
with the provisos that
Z does not represent amino and
$R^2$ does not represent hydrogen if
$R^3$ represents alkyl or aryl,
in a manner known per se,
or in that
[D] compounds of the general formula (XVIII)

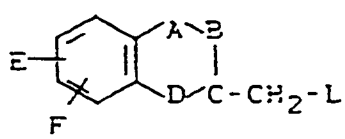

(XVIII),

in which
A, B, C, D, E and F and X have the abovementioned meaning and
L denotes a leaving group for alkylating agents,
are reacted with amines of the formulae

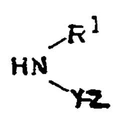

(XV)

$H_2N-R^1$ (XVI)

$H_2N-Y-Z$ (XVII),

in which
$R^1$, Y and Z have the abovementioned meaning,
with the provisos that
Z does not represent amino and
$R^2$ does not represent hydrogen if
$R^3$ represents alkyl or aryl,
or reacted with an alkali metal azide and the azido function is subsequently reduced to an amino function,
and this is reacted in a manner known per se by alkylation, reductive alkylation, acylation, reaction with isocyanates or sulphonylations,
or in that
[E] reactive carboxylic acid derivatives of the formula (XIX)

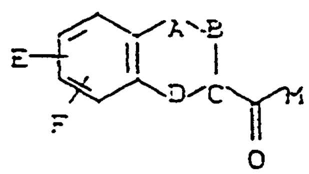

(XIX)

in which
A, B, C, D, E and F have the abovementioned meaning, and
M denotes a leaving group for acylating agents,
are reacted with amines of the formulae

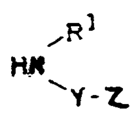

(XV)

$H_2N-R^1$ (XVI

$H_2N-Y-Z$ (XVII)

in which
$R^1$, Y and Z have the abovementioned meaning,
with the provisos that
Z does not represent amino and
$R^2$ does not represent hydrogen if
$R^3$ represents alkyl or aryl,
and the amides thus obtained of the formulae (XX) and (XXI)

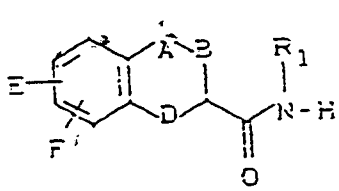

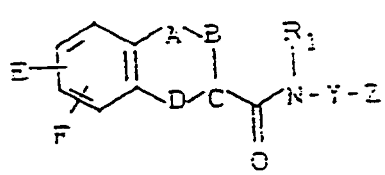

(XX) (XXI)

in which
A, B, C, D, E, F, $R^1$, Y and Z have the abovementioned meaning,
are catalytically reduced using hydrogen, using complex metal hydrides or using boranes,
or in that
[F] compounds of the general formula (XXII)

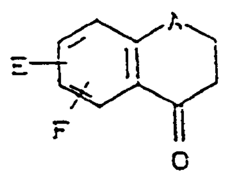

(XXII)

in which A, E and F have the abovementioned meaning are reacted with formaldehyde and amines of the formula (XV)

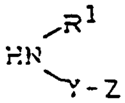

(XV),

in which
$R^1$, Y and Z have the abovementioned meaning,
with the provisos that
Z does not represent amino and
$R^2$ does not represent hydrogen if
$R^3$ represents alkyl or aryl,
and the intermediates obtained of the general formula (XXIII)

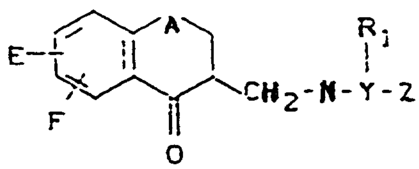

(XXIII)

in which
A, E, F, $R^1$, Y and Z have the abovementioned meaning,
are reacted by reduction of the carbonyl function or by partial reduction of the carbonyl function to the alcohol function, subsequent elimination of water and, if desired, hydrogenation of the C = C double bond using hydrogen.

**2.** Process according to Claim 1 for the preparation of (-)-2-{N-[4-(1,1-dioxido-3-oxo-2,3-dihydro-benzisothiazol-2-yl)butyl]aminomethyl}chroman and its salts.

**3.** Process according to Claim 1 for the preparation of ( + )-2-{N-[4-(1,1-dioxido-3-oxo-2,3-dihydro-benzisothiazol-2-yl)butyl]aminomethyl}chroman and its salts.

**4.** Process according to Claim 1 for the preparation of ( + )-2-{N-[4-(1,1-dioxido-3-oxo-2,3-dihydro-benzisothiazol-2-yl)butyl]aminomethyl}-8-methoxy-chroman and its salts.

**5.** Process according to Claim 1 for the preparation of (-)-2-{N-[4-(1,1-dioxido-3-oxo-2,3-dihydro-benzisothiazol-2-yl)-butyl]aminomethyl}-8-methoxy-chroman and its salts.

**6.** Use of substituted aminomethyltetralins and/or their heterocyclic analogues according to Claims 1 to 5 for the production of medicaments.

7. Use according to Claim 6 for the production of medicaments for the treatment of disorders of the central nervous system.

**Revendications**

**Revendications pour les Etats contractants suivants : AT, BE, CH, DE, FR, GB, GR, IT, LI, LU, NL, SE**

1. Aminométhyltétralines substituées, ainsi que leurs analogues hétérocycliques répondant à la formule générale :

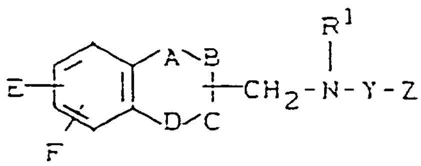

(I),

dans laquelle

Y représente une chaîne alkylène droite contenant 2 à 5 atomes de carbone,

Z représente un groupe de formule

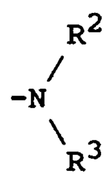

où

$R^2$ et $R^3$ sont identiques ou différents et représentent

- un atome d'hydrogène, un groupe méthyle, un groupe éthyle, un groupe propyle, ou
- un groupe phényle, un groupe benzyle ou un groupe pyridyle, éventuellement substitués par un atome de fluor, par un atome de chlore, par un groupe méthyle ou par un groupe méthoxy,
- un groupe de formule

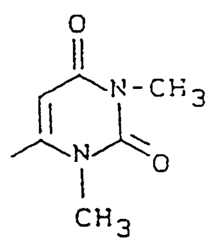

ou

- un groupe de formule $-COR^9$ ou $-SO_2R^{10}$

où

$R^9$

- représente un groupe méthyle, un groupe éthyle ou un groupe éthoxy, ou
- un groupe phényle ou un groupe benzyle, éventuellement substitués par un groupe méthyle, par un groupe méthoxy, par un atome de fluor ou par un atome de chlore,

$R^{10}$

- représente un groupe méthyle, un groupe éthyle ou un groupe propyle,

- un groupe phényle, un groupe naphtyle ou un groupe benzyle, éventuellement substitués par un atome de fluor ou par un atome de chlore, ou
- un groupe de formule

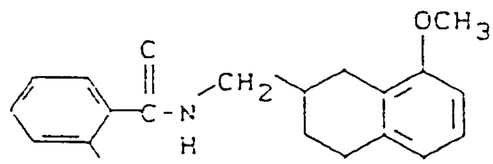

$R^2$ et $R^3$ forment, ensemble avec l'atome d'azote, un composé hétérocyclique de la série

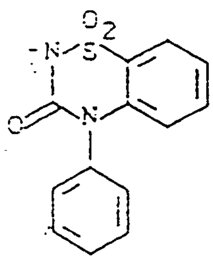

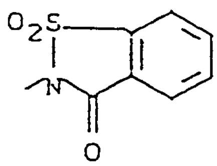

,

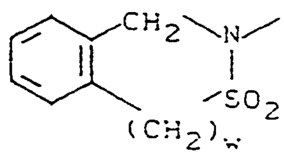

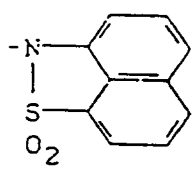

,

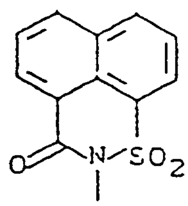

,

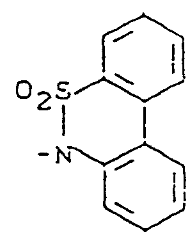

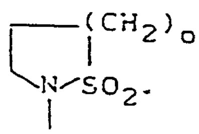

ou

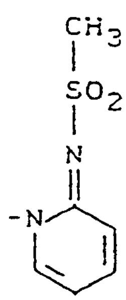

où

w
- représente le nombre 0, 1 ou 2 et

o
- représente le nombre 1 ou 2,

$R^1$
- représente un atome d'hydrogène, un groupe éthyle, un groupe méthyle, un groupe propyle ou un groupe benzyle, ou
- le groupe $-(Y^1-Z^1)$ où $Y^1$ et $Z^1$ peuvent être identiques ou différents par rapport à Y et Z, et ont la signification indiquée ci-dessus pour Y et pour Z,

A et D représentent un groupe de formule $-CH_2-$; un atome d'oxygène; ou la fraction CH ou N d'une double liaison C=C-C=N, avec cette mesure que, soit seulement A, soit seulement D, peut représenter un atome d'oxygène,

B
- représente un groupe de formule $\rangle CH_2$ ou $\rangle CH$ ou encore la fraction CH d'une double liaison C=C ou C=N,

C
- représente un groupe de formule >CH ou la fraction C d'une double liaison C = C ou C = N,

E et F sont identiques ou différents et représentent un atome d'hydrogène, un groupe éthoxy, un groupe méthoxy, un atome de fluor, un groupe cyano ou un groupe de formule $-CONR^2R^3$ où $R^2$ et $R^3$ représentent un atome d'hydrogène,

E et F forment, ensemble, un groupe phényle ou un noyau cyclohexane,

et leurs sels.

**2.** Aminométhyltétralines substituées, ainsi que leurs analogues hétérocycliques selon la revendication 1, dans lesquelles

Y représente une chaîne alkylène droite contenant 2 à 5 atomes de carbone,

Z représente un groupe de formule

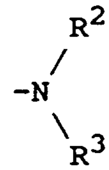

où

$R^2$ et $R^3$ sont identiques ou différents et représentent
- un atome d'hydrogène, un groupe méthyle, un groupe benzyle ou un groupe pyridyle, ou
- un groupe de formule

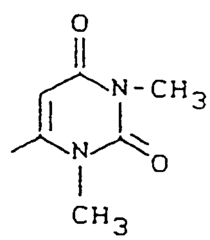

ou
- un groupe de formule $-COR^9$ ou $-SO_2R^{10}$

où

$R^9$
- représente un groupe phényle ou un groupe éthoxy,

$R^{10}$
- représente un groupe phényle ou un groupe naphtyle, pouvant être substitués par un atome de fluor, ou
- un groupe méthyle,
- un groupe de formule

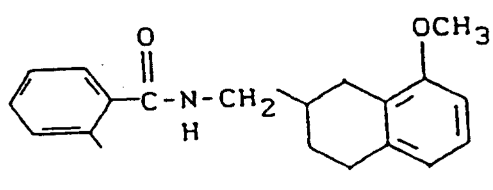

ou bien

$R^2$ et $R^3$ forment, ensemble avec l'atome d'azote, un composé hétérocyclique de la série

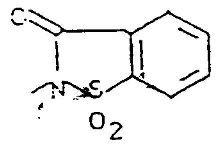 , 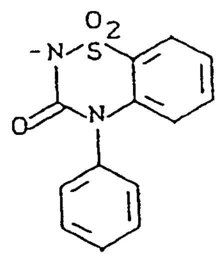 , 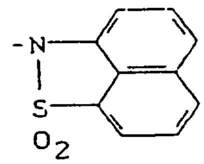

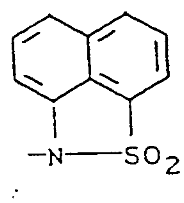 , 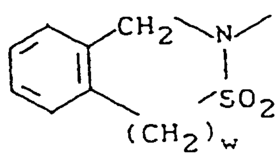 , 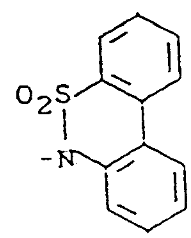

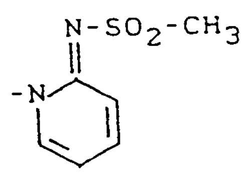 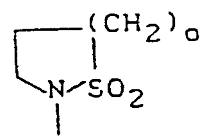

où

w
- représente le nombre 0, 1 ou 2 et

o
- représente le nombre 1 ou 2,

$R^1$
- représente un atome d'hydrogène, un groupe méthyle, un groupe propyle ou un groupe benzyle, ou
- le groupe $-(Y^1-Z^1)$ où $Y^1$ et $Z^1$ peuvent être identiques ou différents par rapport à Y et Z, et ont la signification indiquée ci-dessus pour Y et pour Z,

A et D représentent un groupe de formule $-CH_2-$; un atome d'oxygène; ou la fraction CH ou N d'une double liaison C=C-C=N, avec cette mesure que, soit seulement A, soit seulement D, peut représenter un atome d'oxygène,

B
- représente un groupe de formule $>CH_2$ ou $>CH$ ou encore la fraction CH d'une double liaison C=C ou C=N,

C
- représente un groupe de formule $>CH$ ou la fraction C d'une double liaison C=C ou C=N,

E et F sont identiques ou différents et représentent un atome d'hydrogène, un groupe éthoxy, un groupe méthoxy, un atome de fluor, un groupe cyano ou un groupe de formule $-CONR^2R^3$ où $R^2$ et $R^3$ représentent un atome d'hydrogène,

E et F forment, ensemble, un noyau cyclohexane ou un noyau phényle,

et leurs sels.

**3.** (-)-2-{N-[4-(1,1-dioxydo-3-oxo-2,3-dihydro-benzisothiazol-2-yl)butyl]aminométhyl}chromane et ses sels selon la revendication 1.

**4.** (+)-2-{N-[4-(1,1-dioxydo-3-oxo-2,3-dihydro-benzisothiazol-2-yl)butyl]aminométhyl)chromane et ses sels selon la revendication 1.

**5.** (+)-2-{N-[4-(1,1-dioxydo-3-oxo-2,3-dihydro-benzisothiazol-2-yl)butyl]aminométhyl}-8-méthoxy-chromane et ses sels selon la revendication 1.

**6.** (-)-2-{N-[4-(1,1-dioxydo-3-oxo-2,3-dihydro-benzisothiazol-2-yl)butyl]aminométhyl}-8-méthoxy-chromane et ses sels selon la revendication 1.

**7.** Aminométhyltétralines substituées, ainsi que leurs analogues hétérocycliques selon les revendications 1 à 6, pour un traitement thérapeutique.

**8.** Procédé pour la préparation d'aminométhyltétralines substituées et de leurs analogues hétérocycliques selon les revendications 1 à 6, caractérisé en ce que

[A] on fait réagir des composés répondant à la formule générale (II)

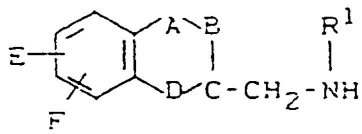

(II),

dans laquelle

A, B, C, D, E, F et $R^1$ ont la signification indiquée à la revendication 1,

(A1) avec des agents d'alkylation de formule (III)

L-Y-Z (III)

dans laquelle

Y et Z ont les significations indiquées à la revendication 1,

avec les mesures que

- Z ne représente pas un groupe amino et
- $R^2$ ne représente pas un atome d'hydrogène lorsque
- $R^3$ représente un groupe alkyle ou un groupe aryle, et
- L représente un groupe qui se sépare pour des agents d'alkylation,

ou bien

(A2) on les soumet à une alkylation par voie de réduction avec des aldéhydes de formule (IV)

OCH-$Y^2$-Z (IV)

dans laquelle

Z a la signification indiquée ci-dessus et $Y^2$ représente une chaîne alkylène Y raccourcie d'un groupe méthylène,

ou bien

(A3) on les fait réagir avec des dérivés réactifs d'acides répondant à la formule générale (V)

M-CO-$Y^2$-Z (V)

dans laquelle

$Y^2$ et Z ont les significations indiquées dans les variantes opératoires (A1) et M représente un groupe qui se sépare pour des agents d'alkylation,

et on soumet à une réduction les amides d'acides correspondants avec de l'hydrogène, en présence d'un catalyseur, avec des boranes ou avec des hydrures de métaux complexes,

ou bien

(A4) on les soumet à une alkylation avec des nitriles de formule

G-CN

dans laquelle

G représente un groupe alkyle inférieur en $C_1$-$C_3$ monochloré, un groupe vinyle ou un groupe vinyle substitués par un groupe alkyle inférieur en $C_1$-$C_3$,

pour obtenir les composés de formules (VI) et (VII)

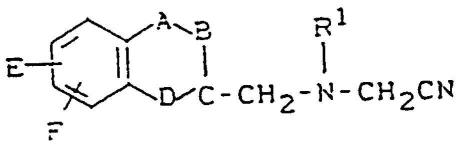

(VI)

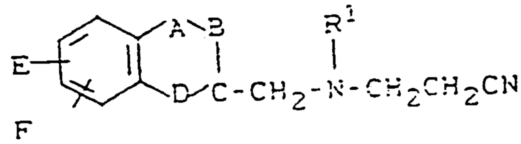

(VII),

dans lesquelles

A, B, C, D, E, F et $R^1$ ont la signification indiquée ci-dessus,

on soumet à une hydrogénation les nitriles obtenus pour obtenir les amines (VIII) et (IX)

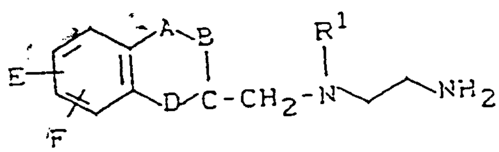

(VIII)

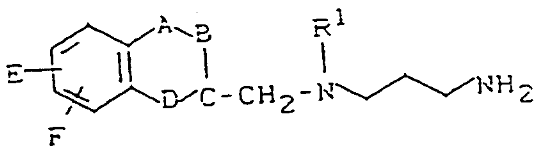

(IX),

dans lesquelles

A, B, C, D, E, F et $R^1$ ont la signification indiquée ci-dessus,

et on fait réagir ces dernières d'une manière connue en soi par alkylation, par alkylation par voie de réduction, par acylation, par réaction avec des isocyanates ou par des sulfonylations,

ou bien en ce que

[B] on soumet des composés de formule générale (X)

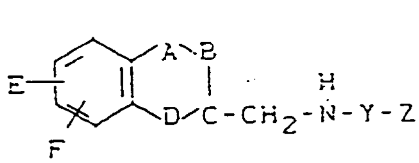

(X),

dans laquelle

A, B, C, D, E, F, Y et Z ont la signification indiquée ci-dessus,

avec les mesures que

Z ne représente pas un groupe amino, et

$R^2$ ne représente pas un atome d'hydrogène, lorsque

$R^3$ représente un groupe alkyle ou un groupe aryle,

(B1) à une alkylation avec des agents d'alkylation de formule (XI)

$R^1$-L (XI)

dans laquelle
$R^1$ a la signification indiquée ci-dessus, et
L représente un groupe qui se sépare pour des agents d'alkylation,
ou bien
(B2) à une alkylation par voie de réduction avec des aldéhydes de formule (XII)

$R^{14}$-CHO (XII)

dans laquelle $R^{14}$ représente un radical $R^1$ raccourci d'un groupe méthylène,
ou bien
(B3) on les fait réagir avec des dérivés d'acides réactifs de formule générale (XIII)

M-CO-$R^{14}$ (XIII)

dans laquelle
$R^{14}$ a la signification indiquée ci-dessus, et
M représente un groupe qui se sépare pour des agents d'acylation,
et on soumet à une réduction les amides d'acides correspondants avec de l'hydrogène en présence d'un catalyseur ou avec des hydrures de métaux complexes, ou bien en ce que
[C] on soumet des aldéhydes de formule (XIV)

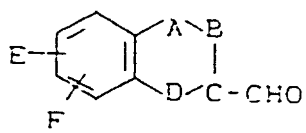
(XIV),

dans laquelle
A, B, C, D, E et F ont la signification indiquée ci-dessus,
à une amination par voie de réduction, d'une manière connue en soi avec des amines de formule (XV), (XVI) ou (XVII)

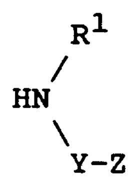
(XV)

$H_2N$-$R^1$ (XVI)

$H_2N$-Y-Z (XVII)

dans lesquelles
$R^1$, Y et Z ont la signification indiquée ci-dessus,
avec les mesures que
Z ne représente pas un groupe amino et
$R^2$ ne représente pas un atome d'hydrogène lorsque
$R^3$ représente un groupe alkyle ou un groupe aryle,
ou bien en ce que

[D] on fait réagir des composés de formule générale (XVIII)

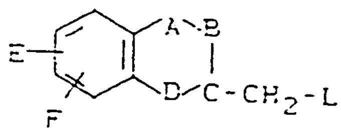

(XVIII),

dans laquelle
A, B, C, D, E et F et X ont la signification indiquée ci-dessus,
et L représente un groupe qui se sépare pour des agents d'alkylation,
avec des amines de formules

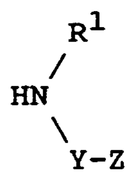

(XV)

$H_2N-R^1$ (XVI)

$H_2N-Y-Z$ (XVII)

dans lesquelles
$R^1$, Y et Z ont la signification indiquée ci-dessus,
avec les mesures que

- Z ne représente pas un groupe amino et
- $R^2$ ne représente pas un atome d'hydrogène lorsque
- $R^3$ représente un groupe alkyle ou un groupe aryle,

ou bien on les fait réagir avec un azide de métal alcalin et ensuite, on réduit la fonction azido à une fonction amino, on fait réagir ces derniers d'une manière connue en soi par alkylation, par alkylation par voie de réduction, par acylation, par mise en réaction avec des isocyanates ou par sulfonations, ou bien en ce que
[E] on fait réagir des dérivés réactifs d'acides carboxyliques de formule (XIX)

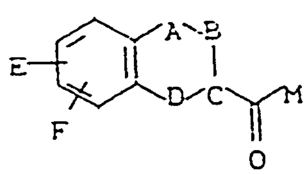

(XIX),

dans laquelle
A, B, C, D, E et F ont la signification indiquée ci-dessus,
et M représente un groupe qui se sépare pour des agents d'acylation,
avec des amines de formules

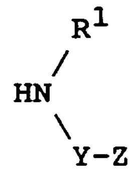

(XV)

$H_2N\text{-}R^1$ (XVI)

$H_2N\text{-}Y\text{-}Z$ (XVII)

dans lesquelles
$R^1$, Y et Z ont la signification indiquée ci-dessus,
avec les mesures que

Z ne représente pas un groupe amino et
$R^2$ ne représente pas un atome d'hydrogène lorsque
$R^3$ représente un groupe alkyle ou un groupe aryle,

et on soumet à une réduction les amides ainsi obtenus répondant aux formules (XX) et (XXI)

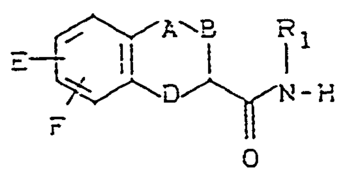

(XX)

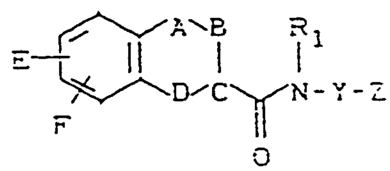

(XXI),

dans lesquelles
A, B, C, D, E, F, $R^1$, Y et Z ont la signification indiquée ci-dessus,
par voie catalytique avec de l'hydrogène, avec des hydrures de métaux complexes ou avec des boranes,
ou bien en ce que
[F] on fait réagir des composés de formule générale (XXII)

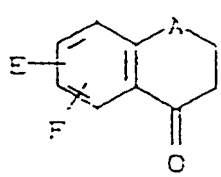

(XXII),

dans laquelle
A, E et F ont la signification indiquée ci-dessus
avec du formaldéhyde et des amines de formule (XV)

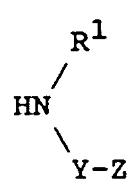

(XV)

dans laquelle
$R^1$, Y et Z ont la signification indiquée ci-dessus
avec les mesures que
Z ne représente pas un groupe amino et
$R^2$ ne représente pas un atome d'hydrogène lorsque
$R^3$ représente un groupe alkyle ou un groupe aryle, et
on fait réagir les produits intermédiaires obtenus répondant à la formule générale (XXIII)

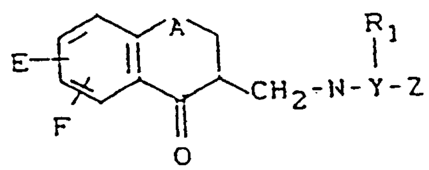
(XXIII),

dans laquelle
A, E, F, $R^1$, Y et Z ont la signification indiquée ci-dessus
par réduction de la fonction carbonyle ou par réduction partielle de la fonction carbonyle en fonction alcool, par élimination ultérieure de l'eau et éventuellement par hydrogénation de la double liaison C = C avec de l'hydrogène.

9. Médicament contenant des aminométhyltétralines substituées et/ou leurs analogues hétérocycliques selon les revendications 1 à 6.

10. Médicament selon la revendication 9 pour le traitement de maladies du système nerveux central.

11. Médicament selon les revendications 9 et 10 pour le traitement des dépressions et des infarctus cérébraux.

12. Utilisation d'aminométhyltétralines substituées et/ou de leurs analogues hétérocycliques selon les revendications 1 à 6 pour la préparation de médicaments.

13. Utilisation selon la revendication 12 pour la préparation de médicaments à des fins de traitement de maladies du système nerveux central.

**Revendications pour l'Etat contractant suivant : ES**

1. Procédé pour la préparation d'aminométhyltétralines substituées et de leurs analogues hétérocycliques répondant à la formule générale :

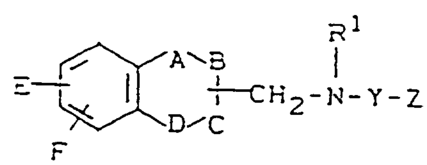
(I),

dans laquelle
Y représente une chaîne alkylène droite contenant 2 à 5 atomes de carbone,
Z représente un groupe de formule

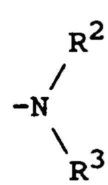

où

$R^2$ et $R^3$ sont identiques ou différents et représentent

- un atome d'hydrogène, un groupe méthyle, un groupe éthyle, un groupe propyle, ou
- un groupe phényle, un groupe benzyle ou un groupe pyridyle, éventuellement substitués par un atome de fluor, par un atome de chlore, par un groupe méthyle ou par un groupe méthoxy,
- un groupe de formule

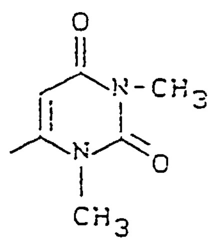

ou

- un groupe de formule $-COR^9$ ou $-SO_2R^{10}$

où

$R^9$

- représente un groupe méthyle, un groupe éthyle ou un groupe éthoxy, ou
- un groupe phényle ou un groupe benzyle, éventuellement substitués par un groupe méthyle, par un groupe méthoxy, par un atome de fluor ou par un atome de chlore,

$R^{10}$

- représente un groupe méthyle, un groupe éthyle ou un groupe propyle,
- un groupe phényle, un groupe naphtyle ou un groupe benzyle, éventuellement substitués par un atome de fluor ou par un atome de chlore, ou
- un groupe de formule

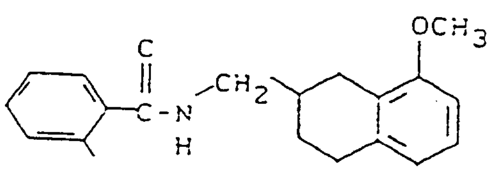

$R^2$ et $R^3$ forment, ensemble avec l'atome d'azote, un composé hétérocyclique de la série

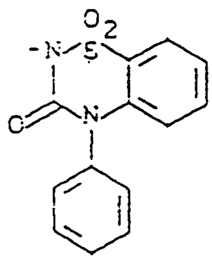
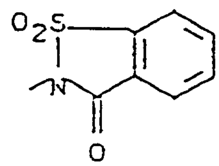,
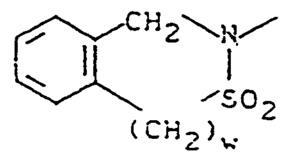

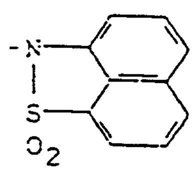,
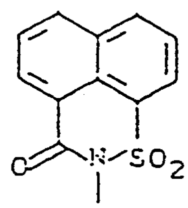,
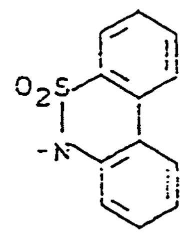

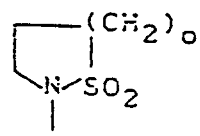
ou
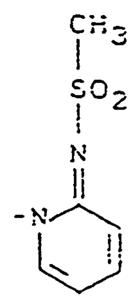

où

w
- représente le nombre 0, 1 ou 2 et

o
- représente le nombre 1 ou 2,

$R^1$
- représente un atome d'hydrogène, un groupe éthyle, un groupe méthyle, un groupe propyle ou un groupe benzyle, ou
- le groupe $-(Y^1-Z^1)$ où $Y^1$ et $Z^1$ peuvent être identiques ou différents par rapport à Y et Z, et ont la signification indiquée ci-dessus pour Y et pour Z,

A et D représentent un groupe de formule $-CH_2-$; un atome d'oxygène; ou la fraction CH ou N d'une double liaison C=C-C=N, avec cette mesure que, soit seulement A, soit seulement D, peut représenter un atome d'oxygène,

B
- représente un groupe de formule $>CH_2$ ou $\geq CH$ ou encore la fraction CH d'une double liaison C=C ou C=N,

C
- représente un groupe de formule $\geq CH$ ou la fraction C d'une double liaison C=C ou C=N,

E et F sont identiques ou différents et représentent un atome d'hydrogène, un groupe éthoxy, un groupe méthoxy, un atome de fluor, un groupe cyano ou un groupe de formule $-CONR^2R^3$ où $R^2$ et $R^3$ représentent un atome d'hydrogène,

E et F forment, ensemble, un groupe phényle ou un noyau cyclohexane,

et leurs sels.

caractérisé en ce que

[A] on fait réagir des composés répondant à la formule générale (II)

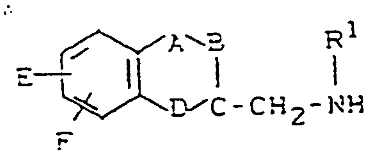

(II),

dans laquelle
A, B, C, D, E, F et $R^1$ ont la signification indiquée,
(A1) avec des agents d'alkylation de formule (III)

L-Y-Z (III)

dans laquelle
Y et Z ont les significations indiquées ci-dessus,
avec les mesures que

- Z ne représente pas un groupe amino et
- $R^2$ ne représente pas un atome d'hydrogène lorsque
- $R^3$ représente un groupe alkyle ou un groupe aryle, et
- L représente un groupe qui se sépare pour des agents d'alkylation,

ou bien
(A2) on les soumet à une alkylation par voie de réduction avec des aldéhydes de formule (IV)

OCH-$Y^2$-Z (IV)

dans laquelle

- Z a la signification indiquée ci-dessus et $Y^2$ représente une chaîne alkylène Y raccourcie d'un groupe méthylène,

ou bien
(A3) on les fait réagir avec des dérivés réactifs d'acides répondant à la formule générale (V)

M-CO-$Y^2$-Z (V)

dans laquelle
$Y^2$ et Z ont les significations indiquées dans les variantes opératoires (A1) et M représente un groupe qui se sépare pour des agents d'alkylation,
et on soumet à une réduction les amides d'acides correspondants avec de l'hydrogène, en présence d'un catalyseur, avec des boranes ou avec des hydrures de métaux complexes,
ou bien
(A4) on les soumet à une alkylation avec des nitriles de formule

G-CN

dans laquelle

- G représente un groupe alkyle inférieur en $C_1$-$C_3$ monochloré, un groupe vinyle ou un groupe vinyle substitués par un groupe alkyle inférieur en $C_1$-$C_3$,

pour obtenir les composés de formules (VI) et (VII)

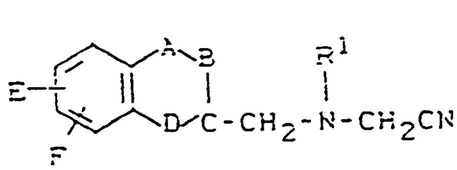

(VI)

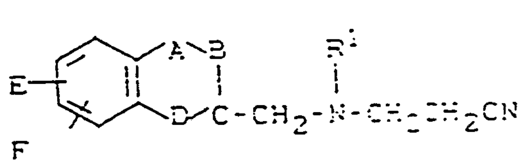

(VII),

dans lesquelles
A, B, C, D, E, F et $R^1$ ont la signification indiquée ci-dessus,
on soumet à une hydrogénation les nitriles obtenus pour obtenir les amines (VIII) et (IX)

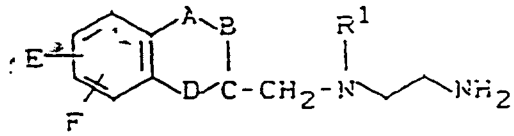

(VIII)

;

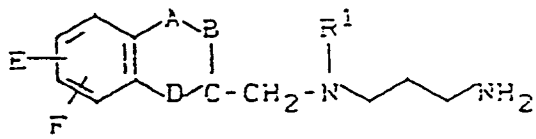

(IX),

dans lesquelles
A, B, C, D, E, F et $R^1$ ont la signification indiquée ci-dessus,
et on fait réagir ces dernières d'une manière connue en soi par alkylation, par alkylation par voie de réduction, par acylation, par réaction avec des isocyanates ou par des sulfonylations,
ou bien en ce que
[B] on soumet des composés de formule générale (X)

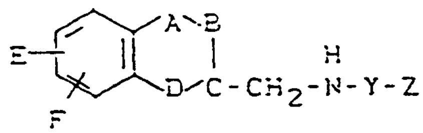

(X),

dans laquelle
A, B, C, D, E, F, Y et Z ont la signification indiquée ci-dessus,
avec les mesures que

- Z ne représente pas un groupe amino, et
- $R^2$ ne représente pas un atome d'hydrogène, lorsque
- $R^3$ représente un groupe alkyle ou un groupe aryle,

(B1) à une alkylation avec des agents d'alkylation de formule (XI)

$R^1$-L (XI)

dans laquelle

- $R^1$ a la signification indiquée ci-dessus, et
- L représente un groupe qui se sépare pour des agents d'alkylation,

ou bien
(B2) à une alkylation par voie de réduction avec des aldéhydes de formule (XII)

$R^{14}$-CHO (XII)

dans laquelle $R^{14}$ représente un radical $R^1$ raccourci d'un groupe méthylène,
ou bien
(B3) on les fait réagir avec des dérivés d'acides réactifs de formule générale (XIII)

M-CO-$R^{14}$ (XIII)

dans laquelle

- $R^{14}$ a la signification indiquée ci-dessus, et
- M représente un groupe qui se sépare pour des agents d'acylation,

et on soumet à une réduction les amides d'acides correspondants avec de l'hydrogène en présence d'un catalyseur ou avec des hydrures de métaux complexes,
ou bien en ce que
[C] on soumet des aldéhydes de formule (XIV)

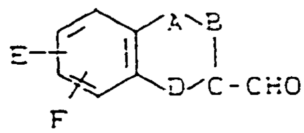

(XIV),

dans laquelle
A, B, C, D, E et F ont la signification indiquée ci-dessus,
à une amination par voie de réduction, d'une manière connue en soi
avec des amines de formule (XV), (XVI) ou (XVII)

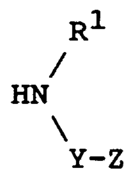

(XV)

$H_2N$-$R^1$ (XVI)

$H_2N$-Y-Z (XVII)

dans lesquelles
$R^1$, Y et Z ont la signification indiquée ci-dessus,
avec les mesures que

Z ne représente pas un groupe amino et
$R^2$ ne représente pas un atome d'hydrogène lorsque
$R^3$ représente un groupe alkyle ou un groupe aryle,

ou bien en ce que
[D] on fait réagir des composés de formule générale (XVIII)

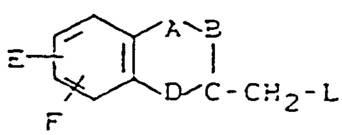

(XVIII),

dans laquelle
A, B, C, D, E et F et X ont la signification indiquée ci-dessus,
et L représente un groupe qui se sépare pour des agents d'alkylation,
avec des amines de formules

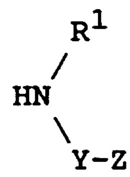

(XV)

$H_2N-R^1$ (XVI)

$H_2N-Y-Z$ (XVII)

dans lesquelles
$R^1$, Y et Z ont la signification indiquée ci-dessus,
avec les mesures que

Z ne représente pas un groupe amino et
$R^2$ ne représente pas un atome d'hydrogène lorsque
$R^3$ représente un groupe alkyle ou un groupe aryle,

ou bien on les fait réagir avec un azide de métal alcalin et ensuite, on réduit la fonction azido à une fonction amino,
on fait réagir ces derniers d'une manière connue en soi par alkylation, par alkylation par voie de réduction, par acylation, par mise en réaction avec des isocyanates ou par sulfonations,
ou bien en ce que
[E] on fait réagir des dérivés réactifs d'acides carboxyliques de formule (XIX)

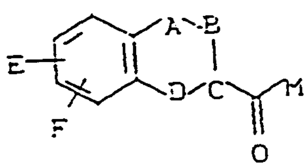

(XIX),

dans laquelle
A, B, C, D, E et F ont la signification indiquée ci-dessus,
et M représente un groupe qui se sépare pour des agents d'acylation,
avec des amines de formules

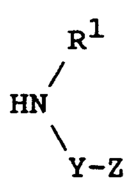

(XV)

$H_2N-R^1$ (XVI)

$H_2N-Y-Z$ (XVII)

dans lesquelles
$R^1$, Y et Z ont la signification indiquée ci-dessus,
avec les mesures que

Z ne représente pas un groupe amino et

$R^2$ ne représente pas un atome d'hydrogène lorsque
$R^3$ représente un groupe alkyle ou un groupe aryle,

et on soumet à une réduction les amides ainsi obtenus répondant aux formules (XX) et (XXI)

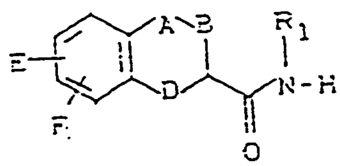

(XX)

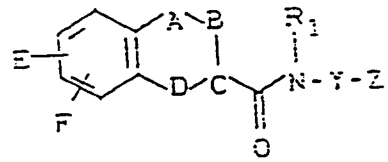

(XXI),

dans lesquelles
A, B, C, D, E, F, $R^1$, Y et Z ont la signification indiquée ci-dessus,
par voie catalytique avec de l'hydrogène, avec des hydrures de métaux complexes ou avec des boranes,
ou bien en ce que
[F] on fait reagir des composés de formule générale (XXII)

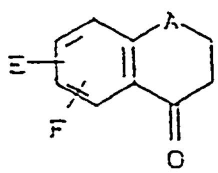

(XXII),

dans laquelle
A, E et F ont la signification indiquée ci-dessus
avec du formaldéhyde et des amines de formule (XV)

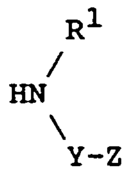

(XV)

dans laquelle
$R^1$, Y et Z ont la signification indiquée ci-dessus
avec les mesures que

Z ne représente pas un groupe amino et
$R^2$ ne représente pas un atome d'hydrogène lorsque
$R^3$ représente un groupe alkyle ou un groupe aryle, et

on fait réagir les produits intermédiaires obtenus répondant à la formule générale (XXIII)

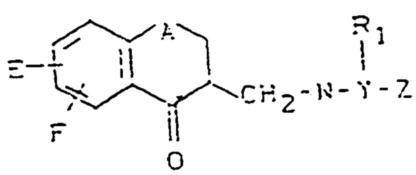

(XXIII),

dans laquelle
A, E, F, $R^1$, Y et Z ont la signification indiquée ci-dessus
par réduction de la fonction carbonyle ou par réduction partielle de la fonction carbonyle en fonction alcool, par élimination ultérieure de l'eau et éventuellement par hydrogénation de la double liaison C = C avec de l'hydrogène.

**2.** Procédé selon la revendication 1 pour la préparation du (-)-2-{N-[4-(1,1-dioxydo-3-oxo-2,3-dihydro-benziso-thiazol-2-yl)butyl]aminométhyl}chromane et de ses sels.

**3.** Procédé selon la revendication 1 pour la préparation du (+)-2-{N-[4-(1,1-dioxydo-3-oxo-2,3-dihydro-benziso-thiazol-2-yl)butyl]aminométhyl}chromane et de ses sels.

**4.** Procédé selon la revendication 1 pour la préparation du (+)-2-{N-[4-(1,1-dioxydo-3-oxo-2,3-dihydro-benziso-thiazol-2-yl)butyl]aminométhyl}-8-méthoxy-chromane et de ses sels.

**5.** Procédé selon la revendication 1 pour la préparation du (-)-2-{N-[4-(1,1-dioxydo-3-oxo-2,3-dihydro-benziso-thiazol-2-yl)butyl]aminométhyl}-8-méthoxy-chromane et de ses sels.

**6.** Utilisation d'aminométhyltétralines substituées et/ou de leurs analogues hétérocycliques selon les revendications 1 à 5 pour la préparation de médicaments.

**7.** Utilisation selon la revendication 6 pour la préparation de médicaments à des fins de traitement de maladies du système nerveux central.